# EUROPEAN PATENT APPLICATION

(11) **EP 4 324 925 A1**
(43) Date of publication of application: **21.02.2024**
(21) Application number: 22788198.4
(22) Date of filing: 14.04.2022
(51) Int. Cl.: C12N 15/13, C07K 16/18

(54) **ANTI-C1S ANTIBODY**

(30) Priority: 15.04.2021 JP 2021068964
(71) Applicant: CHUGAI SEIYAKU KABUSHIKI KAISHA, Tokyo 115-8543 (JP)
(72) Inventor: KOGA, Hikaru, Gotemba-shi, Shizuoka 412-8513 (JP)
(74) Representative: Vossius & Partner Patentanwälte Rechtsanwälte mbB
(86) International application number: PCT/JP2022/017780
(87) International publication number: WO 2022/220275

(57) **Abstract**

The present invention provides antibodies that comprises an antigen-binding region and an antibody constant region and binds to C1s in a pH-dependent manner. The present invention provides pharmaceutical compositions comprising any one of the antibodies, and methods of treating an individual having a complement-mediated disease or disorder, or preventing an individual potentially having a complement-mediated disease or disorder, the method comprising administering any one of the antibodies to the individual.

## Description

### [Technical Field]

The present invention relates to antibodies such as anti-C1s antibodies, and methods of using the same.

### [Background Art]

The C1 complex is a large protein complex which functions as the key initiator of the classical pathway cascade. The C1 complex consists of three components, C1q, C1r and C1s, which are in molar ratio of 1:2:2 respectively (NPL 1). The classical pathway is initiated when the C1 complex binds to a target that is bound by antibodies. C1q, which has 6 globular heads, mediates the binding of C1 complex to the antibodies by avidity interaction with the Fc regions. Once tightly bound to the target, C1r within the C1 complex autoactivates and become enzymatically active. The activated C1r then cleaves and activates proenzyme C1s within the C1 complex (NPL 2). Subsequently, active C1s cleaves its substrates, complement components C2 and C4 into C2a/C2b, and C4a/C4b fragments respectively. This leads to the assembly of C4b2a, a C3 convertase, on the target surface which cleaves C3 to form C3b. C3b in turn cleaves C5 to initiate the formation of the terminal membrane attack complex, C5b, C6, C7, C8 and C9, which lyses the target via pore formation.

Both C1s and C1r proteins have an identical domain organization, which is CUB1-EGF-CUB2-CCP1-CCP2-Serine Protease (NPL 3). The CUB1-EGF-CUB2 domain mediates the interaction between C1r and C1s to form the C1r2s2 tetramer (NPL 4), and also between C1r2s2 and C1q (NPL 5). In contrast, the CCP1-CCP2-Serine Protease domains of C1r and C1s are responsible for proteolytic cleavage of their respective substrates (NPL 6, NPL 7).The C1r2s2 tetramer interacts with the six stems in C1q through six binding sites within the CUB1-EGF-CUB2 domains of the tetramer (NPL 5).

While a properly functioning complement system defends the host against pathogens, dysregulation or inappropriate activation of the classical pathway results in a variety of complement-mediated disorders such as, and not limited to, autoimmune hemolytic anemias (AIHA), Behcet's disease, Bullous Pemphigus (BP), immune thrombocytopenia purpura (ITP) etc. Therefore, inhibition of excessive or uncontrolled activation of the classical pathway can provide clinical benefit to patients with such disorders.

HI532, an antibody which binds to the beta domain of C1s, was reported to be able to inhibit the interaction of C1r2s2 with C1q (NPL 8). However, this antibody was not able to completely neutralize hemolytic activity of human serum and 30% of activity remained even after 24hrs incubation of serum with the antibody.

Antibodies are highly attractive pharmaceuticals as they are stable in plasma, highly specific for their target, and generally exhibit superior pharmacokinetic profiles. However, due to their large molecular size, the dosage of therapeutic antibodies is usually high. In the case of targets that exist in high abundance, the required therapeutic dose of antibodies is even higher. As a result, methods that improve antibody pharmacokinetics, pharmacodynamics, and antigen binding properties are attractive ways to reduce the dosage and high production costs associated with therapeutic antibodies.

It has been reported that antibodies that bind to an antigen in a pH-dependent manner (herein below also referred to as "pH-dependent antibody" or "pH-dependent-binding antibody") enable a single antibody molecule to neutralize multiple antigen molecules (NPL 9, PTL 1). The pH-dependent antibody binds strongly to its antigen at neutral pH conditions in the plasma, but dissociates from the antigen under the acidic pH condition within the endosome of a cell. Once dissociated from the antigen, the antibody is recycled back to the plasma by FcRn receptors whereas the dissociated antigens are degraded within the lysosome of the cell. The recycled antibody is then free to bind to and neutralize antigen molecules again and this process continues to be repeated as long as the antibody remains in circulation.

### [Citation List]

### [Patent Literature]

[PTL 1] WO2009/125825

### [Non Patent Literature]

[NPL 1] Wang et. al. Mol Cell. 2016 Jul 7;63(1): 135-45
[NPL 2] Mortensen et. al. Proc Natl Acad Sci USA. 2017 Jan 31;114(5):986-991
[NPL 3] Gal et. al. Mol Immunol. 2009 Sep;46(14):2745-52
[NPL 4] Almitairi et. al. Proc Natl Acad Sci USA. 2018 Jan 23;115(4):768-773
[NPL 5] Bally et. al. J Biol Chem. 2009 Jul 17;284(29): 19340-8
[NPL 6] Rossi et. al. 1998 J Biol Chem. 1998 Jan 9;273(2): 1232-9
[NPL 7] Lacroix et. al. J Biol Chem. 2001 Sep 28;276(39):36233-40
[NPL 8] Tseng et. al. Mol Immunol. 1997 Jun;34(8-9):671-9
[NPL 9] Igawa et. al. Nat Biotechnol. 2010 Nov;28(11):1203-7

### [Summary of Invention]

### [Technical Problem]

The invention provides anti-complement component antibodies such as anti-C1s antibodies, pharmaceutical compositions comprising the same, and methods of using the same.

### [Solution to Problem]

In one embodiment, the present invention provides antibodies that comprise an antigen-binding region and an antibody constant region, have a dissociation promoting function (displacement function) such that the antibody binds to C1qrs complex and promotes dissociation of C1q from C1qrs complex and/or a blocking function such that the antibody binds to C1r2s2 and inhibits the binding of C1q to C1r2s2, and bind to C1s in a pH-dependent manner. In a specific embodiment, the antibodies of the present invention have a mutant constant region comprising at least one amino acid alteration that decreases FcyR-binding activity and/or at least one amino acid alteration that decreases isoelectric point (pI) of an Fc region.

Specifically, the present invention relates to the following:
[1] An isolated antibody that comprises an antigen-binding region and an antibody constant region,
   wherein the antibody promotes dissociation of C1q from C1qrs complex and/or inhibits binding of C1q to C1r2s2, and
   wherein the antigen-binding region comprises a combination of HVR-H1, HVR-H2, HVR-H3, HVR-L1, HVR-L2, and HVR-L3 selected from the group consisting of 1) to 6) below:
      1) HVR-H1, HVR-H2, HVR-H3, HVR-L1, HVR-L2, and HVR-L3 that comprise the amino acid sequences consisting of SEQ ID NOs: 25, 26, 27, 60, 61, and 62, respectively;
      2) HVR-H1, HVR-H2, HVR-H3, HVR-L1, HVR-L2, and HVR-L3 that comprise the amino acid sequences consisting of SEQ ID NOs: 37, 38, 39, 56, 57, and 58, respectively;
      3) HVR-H1, HVR-H2, HVR-H3, HVR-L1, HVR-L2, and HVR-L3 that comprise the amino acid sequences consisting of SEQ ID NOs: 25, 26, 27, 56, 57, and 58, respectively;
      4) HVR-H1, HVR-H2, HVR-H3, HVR-L1, HVR-L2, and HVR-L3 that comprise the amino acid sequences consisting of SEQ ID NOs: 25, 26, 27, 48, 49, and 50, respectively;
      5) HVR-H1, HVR-H2, HVR-H3, HVR-L1, HVR-L2, and HVR-L3 that comprise the amino acid sequences consisting of SEQ ID NOs: 29, 30, 31, 52, 53, and 54, respectively; and
      6) HVR-H1, HVR-H2, HVR-H3, HVR-L1, HVR-L2, and HVR-L3 that comprise the amino acid sequences consisting of SEQ ID NOs: 33, 34, 35, 56, 57, and 58, respectively.
[2] The antibody of [1], wherein the antibody comprises a heavy chain variable region (VH) and a light chain variable region (VL) selected from the group consisting of 1) to 6) below:
   1) a VH and VL that comprise the amino acid sequences consisting of SEQ ID NOs: 24 and 59, respectively;
   2) a VH and VL that comprise the amino acid sequences consisting of SEQ ID NOs: 36 and 55, respectively;
   3) a VH and VL that comprise the amino acid sequences consisting of SEQ ID NOs: 24 and 55, respectively;
   4) a VH and VL that comprise the amino acid sequences consisting of SEQ ID NOs: 24 and 47, respectively;
   5) a VH and VL that comprise the amino acid sequences consisting of SEQ ID NOs: 28 and 51, respectively; and
   6) a VH and VL that comprise the amino acid sequences consisting of SEQ ID NOs: 32 and 55, respectively.
[3] The antibody of [1] or [2], wherein a ratio of a KD value in acidic pH range to a KD value in neutral pH range, an acidic KD/ neutral KD ratio, is 107 or more.
[4] The antibody of any one of [1] to [3], wherein the antigen-binding region can specifically bind to a CUB1-EGF-CUB2 domain of human C1s.
[5] The antibody of any one of [1] to [4], wherein the antibody has a mutant constant region comprising at least one amino acid alteration that decreases Fcy receptor-binding activity.
[6] The antibody of [5], wherein the mutant constant region comprises an amino acid alteration at at least one of positions 235 and 236 according to EU numbering.
[7] The antibody of any one of [1] to [6], wherein the antibody has a mutant constant region comprising at least one amino acid alteration, and wherein the amino acid alteration decreases isoelectric point (pI) of the mutant constant region as compared to that of a parent constant region.
[8] The antibody of [7], wherein the mutant constant region comprises an amino acid alteration at at least one of positions 137, 268, 274, 355, and 419 according to EU numbering.
[9] The antibody of any one of [1] to [8], wherein pI is 7.8 or less.
[10] The antibody of any one of [1] to [8], wherein the constant region comprises a heavy chain constant region comprising the amino acid sequence consisting of SEQ ID NO: 45 and a light chain constant region comprising the amino acid sequence consisting of SEQ ID NO: 23.
[11] An antibody having binding activity to C1s, wherein the antibody comprises a combination of HVR-H1, HVR-H2, HVR-H3, HVR-L1, HVR-L2, and HVR-L3 selected from the group consisting of 1) to 6) below:
   1) HVR-H1, HVR-H2, HVR-H3, HVR-L1, HVR-L2, and HVR-L3 that comprise the amino acid sequences consisting of SEQ ID NOs: 25, 26, 27, 60, 61, and 62, respectively;
   2) HVR-H1, HVR-H2, HVR-H3, HVR-L1, HVR-L2, and HVR-L3 that comprise the amino acid sequences consisting of SEQ ID NOs: 37, 38, 39, 56, 57, and 58, respectively;
   3) HVR-H1, HVR-H2, HVR-H3, HVR-L1, HVR-L2, and HVR-L3 that comprise the amino acid sequences consisting of SEQ ID NOs: 25, 26, 27, 56, 57, and 58, respectively;
   4) HVR-H1, HVR-H2, HVR-H3, HVR-L1, HVR-L2, and HVR-L3 that comprise the amino acid sequences consisting of SEQ ID NOs: 25, 26, 27, 48, 49, and 50, respectively;
   5) HVR-H1, HVR-H2, HVR-H3, HVR-L1, HVR-L2, and HVR-L3 that comprise the amino acid sequences consisting of SEQ ID NOs: 29, 30, 31, 52, 53, and 54, respectively; and
   6) HVR-H1, HVR-H2, HVR-H3, HVR-L1, HVR-L2, and HVR-L3 that comprise the amino acid sequences consisting of SEQ ID NOs: 33, 34, 35, 56, 57, and 58, respectively.
[12] The antibody of [11], wherein the antibody comprises a heavy chain variable region (VH) and a light chain variable region (VL) selected from the group consisting of 1) to 6) below:
   1) a VH and VL that comprise the amino acid sequences consisting of SEQ ID NOs: 24 and 59, respectively;
   2) a VH and VL that comprise the amino acid sequences consisting of SEQ ID NOs: 36 and 55, respectively;
   3) a VH and VL that comprise the amino acid sequences consisting of SEQ ID NOs: 24 and 55, respectively;
   4) a VH and VL that comprise the amino acid sequences consisting of SEQ ID NOs: 24 and 47, respectively;
   5) a VH and VL that comprise the amino acid sequences consisting of SEQ ID NOs: 28 and 51, respectively; and
   6) a VH and VL that comprise the amino acid sequences consisting of SEQ ID NOs: 32 and 55, respectively.
[13] The antibody of [11] or [12], wherein the antibody constant region comprises an H chain constant region comprising the amino acid sequence consisting of SEQ ID NO: 45 and an L chain constant region comprising the amino acid sequence consisting of SEQ ID NO: 23.
[14] An antibody comprising a heavy chain (H chain) and a light chain (L chain) selected from the group consisting of 1) to 6) below:
   1) an H chain and L chain that comprise the amino acid sequences consisting of SEQ ID NOs: 66 and 67, respectively;
   2) an H chain and L chain that comprise the amino acid sequences consisting of SEQ ID NOs: 68 and 69, respectively;
   3) an H chain and L chain that comprise the amino acid sequences consisting of SEQ ID NOs: 70 and 71, respectively;
   4) an H chain and L chain that comprise the amino acid sequences consisting of SEQ ID NOs: 72 and 73, respectively;
   5) an H chain and L chain that comprise the amino acid sequences consisting of SEQ ID NOs: 74 and 75, respectively; and
   6) an H chain and L chain that comprise the amino acid sequences consisting of SEQ ID NOs: 76 and 77, respectively.
[15] A pharmaceutical composition comprising the antibody of any one of [1] to [14] and at least one pharmaceutically acceptable carrier.
[16] The pharmaceutical composition of [15], which is for use in treating an individual having a complement-mediated disease or disorder, or preventing an individual potentially having a complement-mediated disease or disorder.
[17] A method of treating an individual having a complement-mediated disease or disorder, or preventing an individual potentially having a complement-mediated disease or disorder, the method comprising administering to the individual an effective amount of the antibody of any one of [1] to [14].
[18] The antibody of any one of [1] to [14], which is for use in treating or preventing a complement-mediated disease or disorder.
[19] A therapeutic or prophylactic agent for a complement-mediated disease or disorder, the agent comprising the antibody of any one of [1] to [14].
[20] Use of the antibody of any one of [1] to [14] in the manufacture of a therapeutic or prophylactic agent for a complement-mediated disease or disorder.
[21] An isolated antibody comprising an antigen-binding region and an antibody constant region,
   wherein a ratio of a KD value in acidic pH range to a KD value in neutral pH range, an acidic KD/ neutral KD ratio, is 107 or more, and
   wherein the antibody constant region comprises at least one amino acid alteration that decreases Fcγ receptor-binding activity and at least one amino acid alteration that decreases isoelectric point (pI).

### [Brief Description of Drawings]

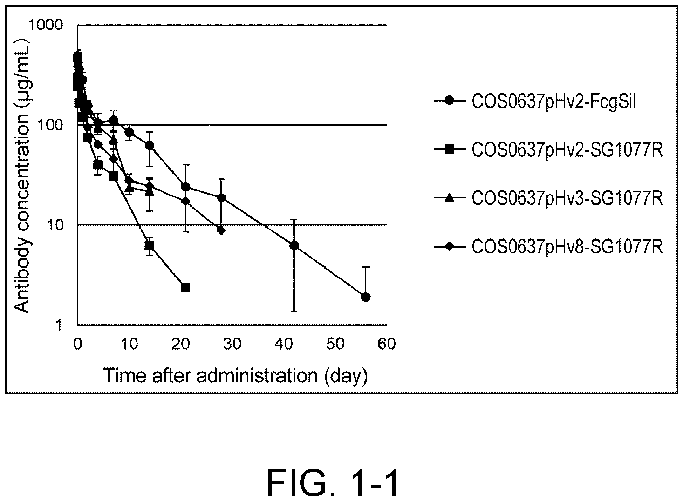

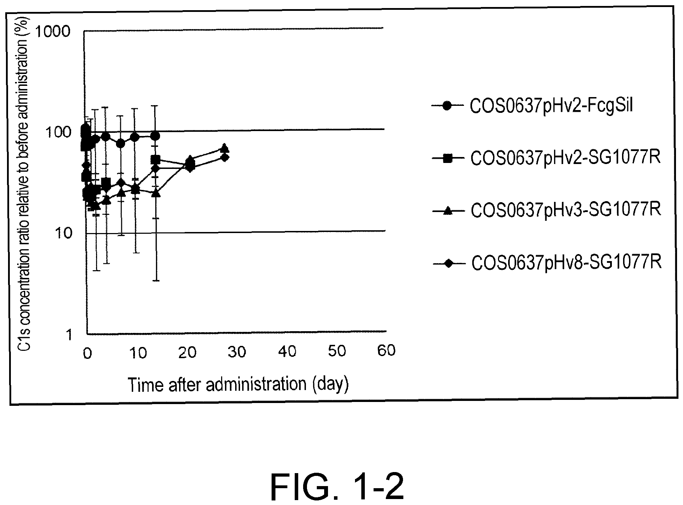

Fig. 1-1 shows the changes in antibody concentrations in plasma of male cynomolgus monkeys after receiving a single intravenous administration of COS0637pHv2-FcgSil, COS0637pHv2-SG1077R, COS0637pHv3-SG1077R, and COS0637pHv8-SG1077R antibodies (log-linear). Data are shown by mean ± standard deviation (N = 3).
Fig. 1-2 shows the changes in the ratio of C1s concentration in plasma of male cynomolgus monkeys after receiving a single intravenous administration of COS0637pHv2-FcgSil, COS0637pHv2-SG1077R, COS0637pHv3-SG1077R, and COS0637pHv8-SG1077R antibodies to the C1s concentration in plasma before the administration (log-linear). Data are shown by mean ± standard deviation (N = 3).

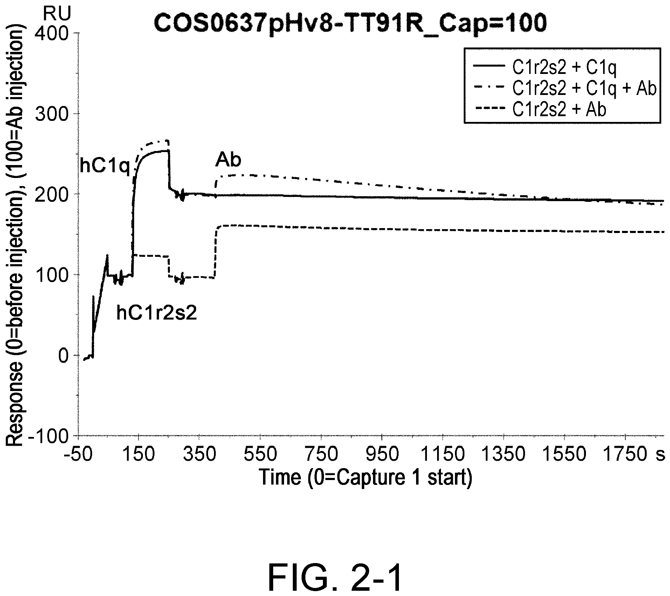

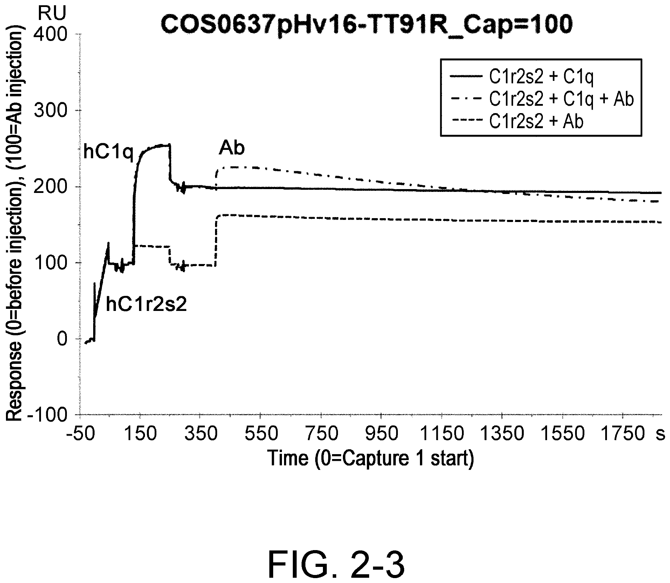

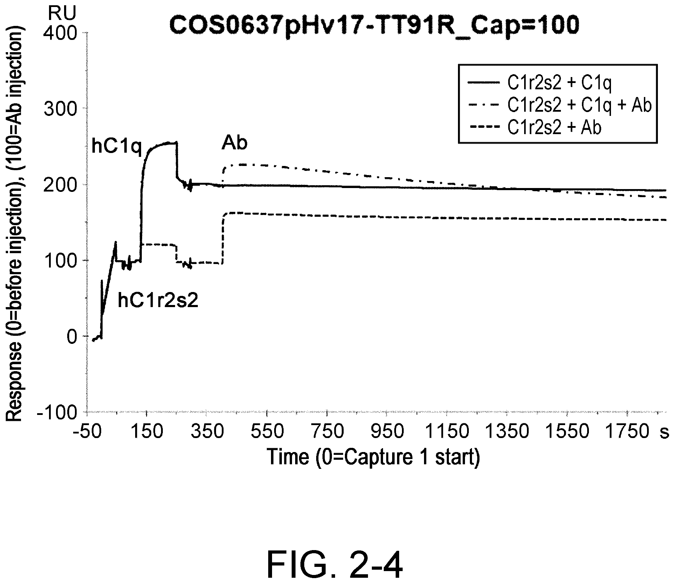

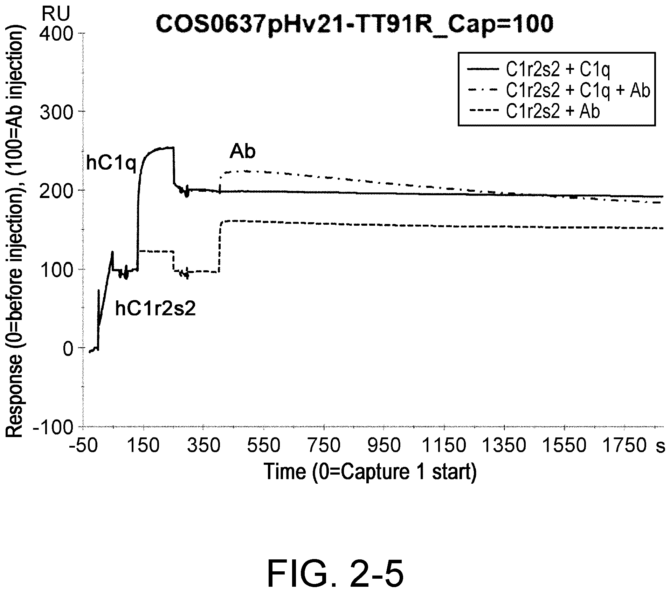

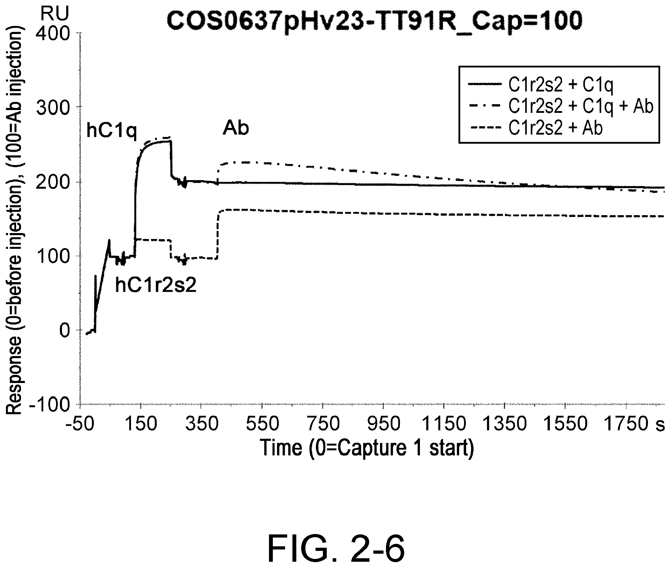

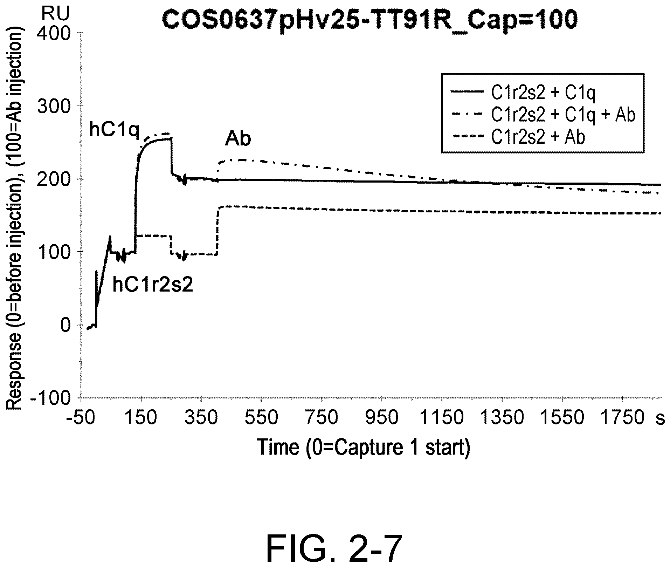

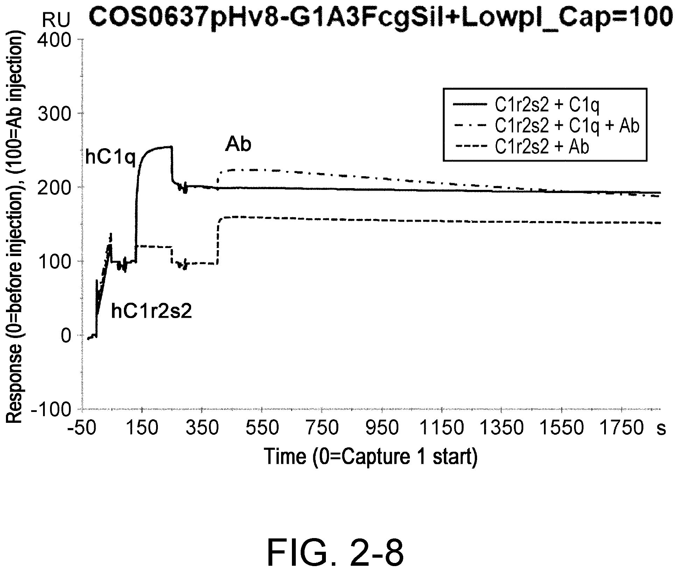

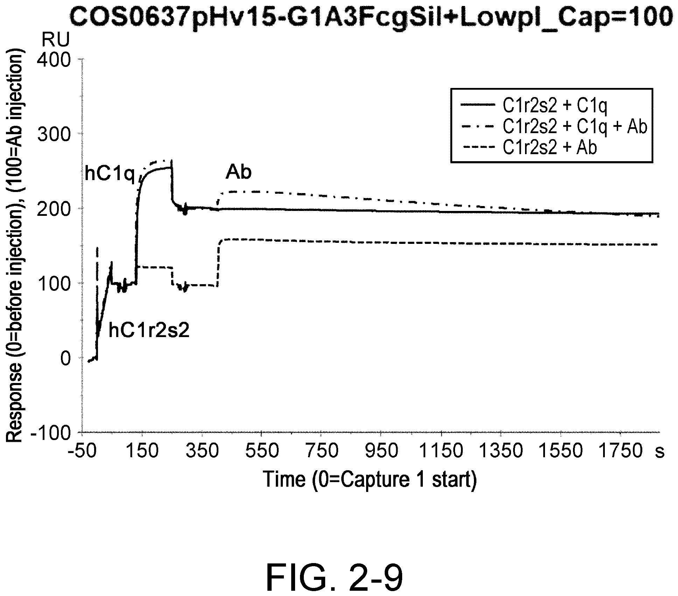

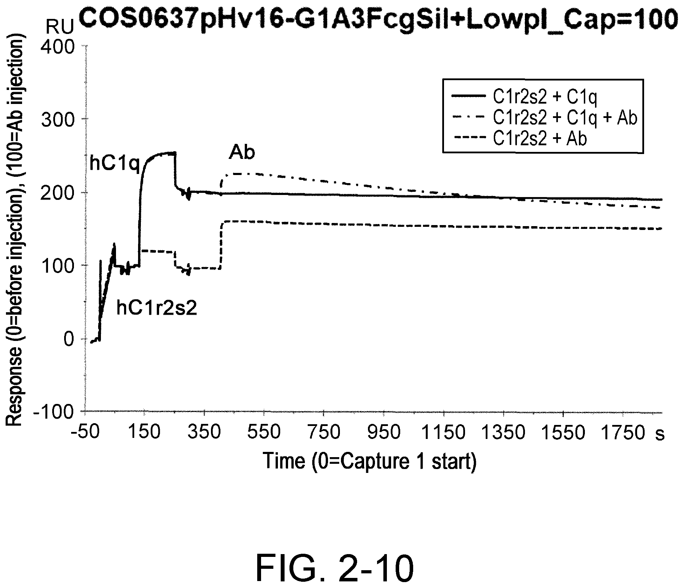

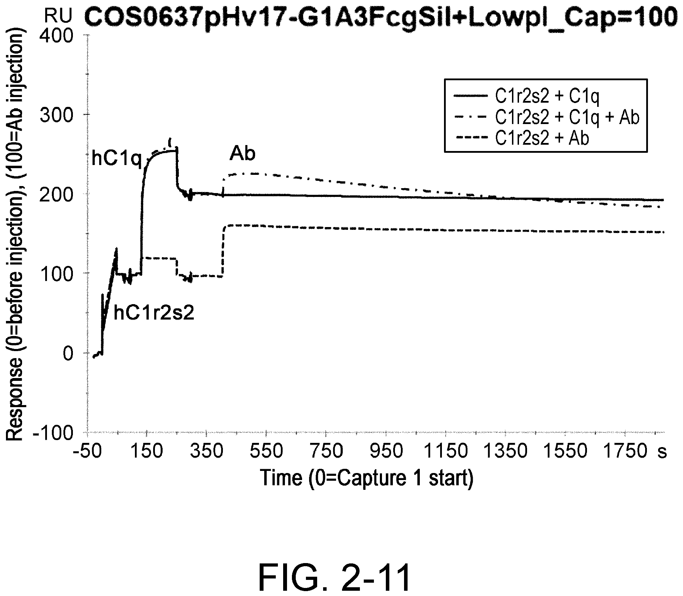

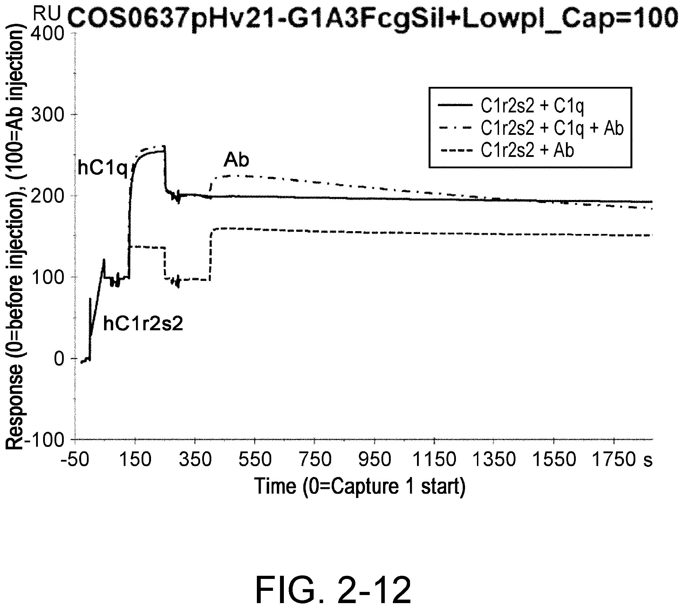

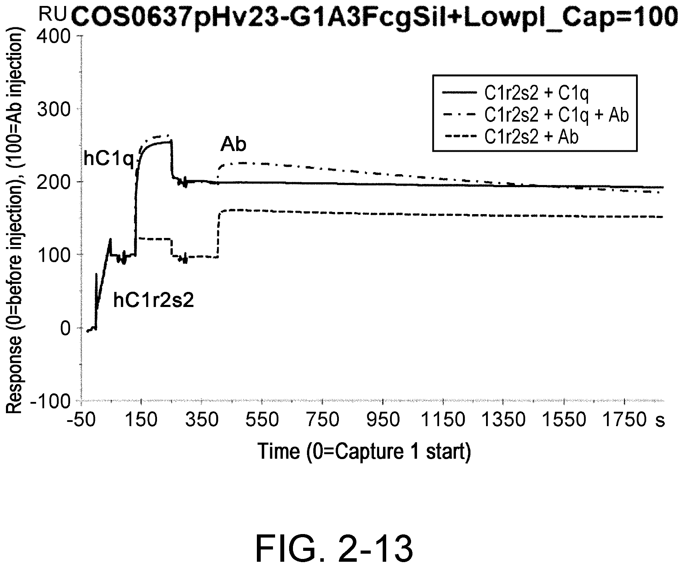

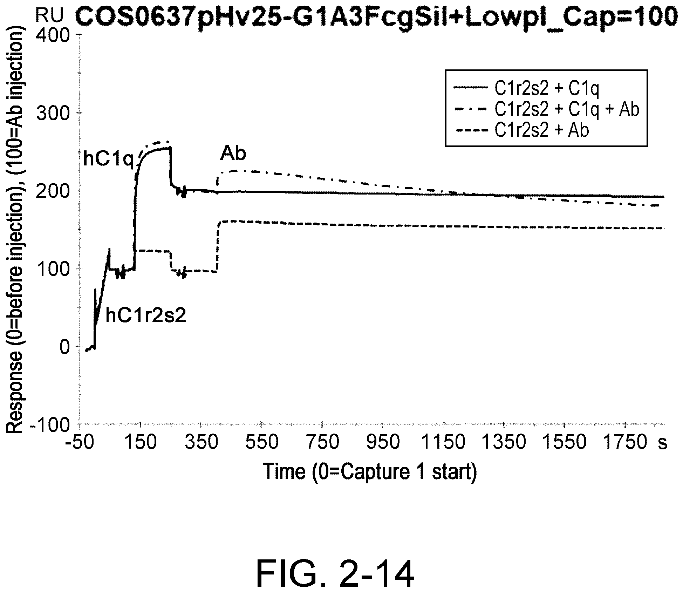

Fig. 2-1 shows the evaluation of the anti-C1s antibody COS0637pHv8-TT91R for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv8-TT91R was injected as the Ab.

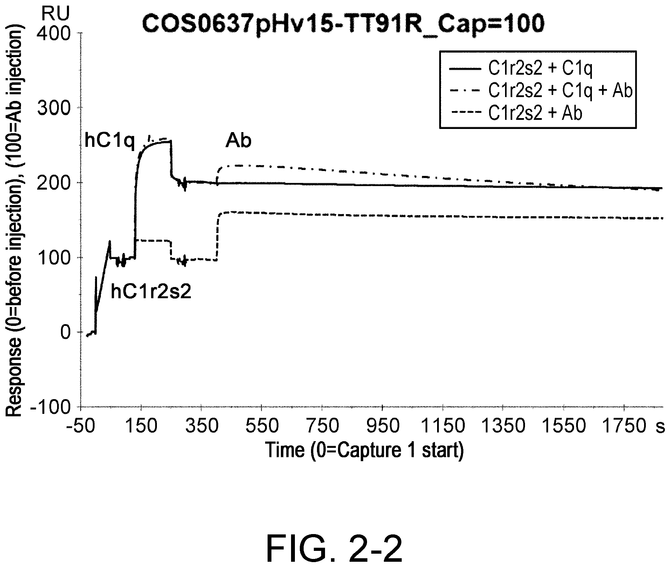

Fig. 2-2 shows the evaluation of the anti-C1s antibody COS0637pHv15-TT91R for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv15-TT91R was injected as the Ab.
Fig. 2-3 shows the evaluation of the anti-C1s antibody COS0637pHv16-TT91R for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv16-TT91R was injected as the Ab.
Fig. 2-4 shows the evaluation of the anti-C1s antibody COS0637pHv17-TT91R for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv17-TT91R was injected as the Ab.
Fig. 2-5 shows the evaluation of the anti-C1s antibody COS0637pHv21-TT91R for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv21-TT91R was injected as the Ab.
Fig. 2-6 shows the evaluation of the anti-C1s antibody COS0637pHv23-TT91R for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv23-TT91R was injected as the Ab.
Fig. 2-7 shows the evaluation of the anti-C1s antibody COS0637pHv25-TT91R for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv25-TT91R was injected as the Ab.
Fig. 2-8 shows the evaluation of the anti-C1s antibody COS0637pHv8-G IA3FcgSil+LowpI for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv8-G1A3FcgSil+LowpI was injected as the Ab.
Fig. 2-9 shows the evaluation of the anti-C1s antibody COS0637pHv15-G IA3FcgSil+LowpI for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv15-G1A3FcgSil+LowpI was injected as the Ab.
Fig. 2-10 shows the evaluation of the anti-C1s antibody COS0637pHv16-G IA3FcgSil+LowpI for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv16-G1A3FcgSil+LowpI was injected as the Ab.
Fig. 2-11 shows the evaluation of the anti-C1s antibody COS0637pHv17-G IA3FcgSil+LowpI for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv17-G1A3FcgSil+LowpI was injected as the Ab.
Fig. 2-12 shows the evaluation of the anti-C1s antibody COS0637pHv21-G IA3FcgSil+LowpI for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv21-G1A3FcgSil+LowpI was injected as the Ab.
Fig. 2-13 shows the evaluation of the anti-C1s antibody COS0637pHv23-G IA3FcgSil+LowpI for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv23-G1A3FcgSil+LowpI was injected as the Ab.
Fig. 2-14 shows the evaluation of the anti-C1s antibody COS0637pHv25-G1A3FcgSil+LowpI for the C1q displacement function. The solid line indicates the sensorgram obtained when hC1q was injected to hC1r2s2 and then buffer was injected (C1r2s2+C1q): sensorgram 1; the dashed-dotted line indicates the sensorgram obtained when hC1q was injected to hC1r2s2 and then antibody was injected (C1r2s2+C1q+Ab): sensorgram 2; and the broken line indicates the sensorgram obtained when hC1q was not injected to hC1r2s2 and only the antibody was injected (C1r2s2+Ab): sensorgram 3. COS0637pHv25-G1A3FcgSil+LowpI was injected as the Ab.

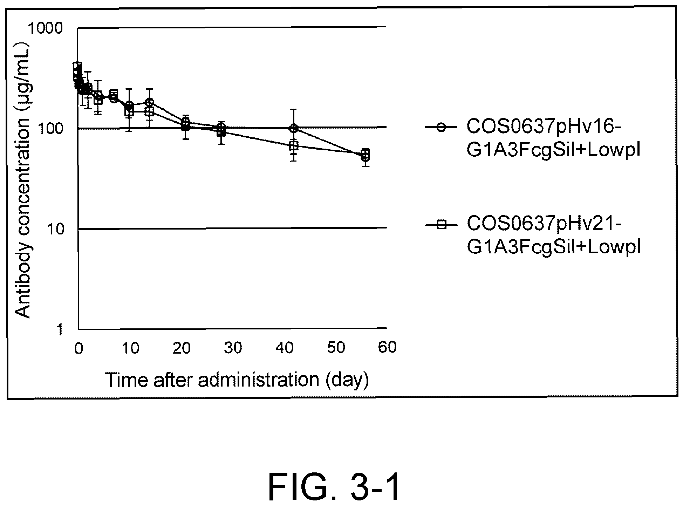

Fig. 3-1 shows the changes in antibody concentrations in plasma of male cynomolgus monkeys after receiving a single intravenous administration of COS063 7pHv 16-G1A3FcgSil+LowpI and COS0637pHv21-G1A3FcgSil+LowpI antibodies (log-linear). Data are shown by mean ± standard deviation (COS0637pHv16-G1A3FcgSil+LowpI antibody; N = 3, COS0637pHv21-G1A3FcgSil+LowpI antibody; N = 2).

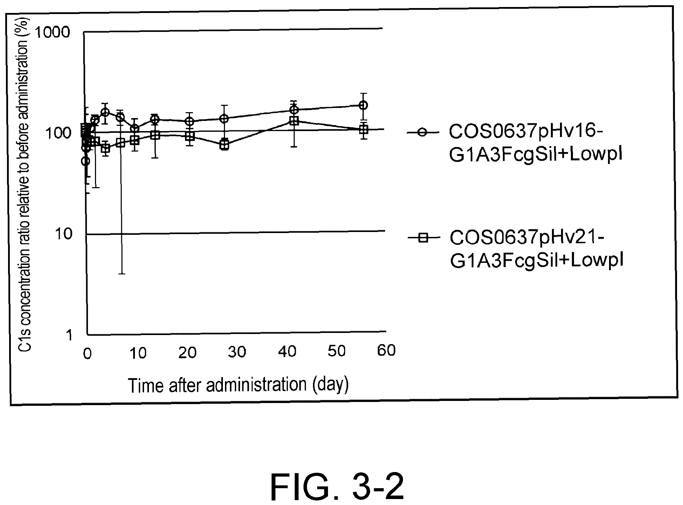

Fig. 3-2 shows the changes in the ratio of C1s concentration in plasma of male cynomolgus monkeys after receiving a single intravenous administration of COS063 7pHv 16-G1A3FcgSil+LowpI and COS0637pHv21-G1A3FcgSil+LowpI antibodies to the C1s concentration in plasma before the administration (log-linear). Data are shown by mean ± standard deviation (COS0637pHv16-G1A3FcgSil+LowpI antibody; N = 3, COS0637pHv21-G1A3FcgSil+LowpI antibody; N = 2).

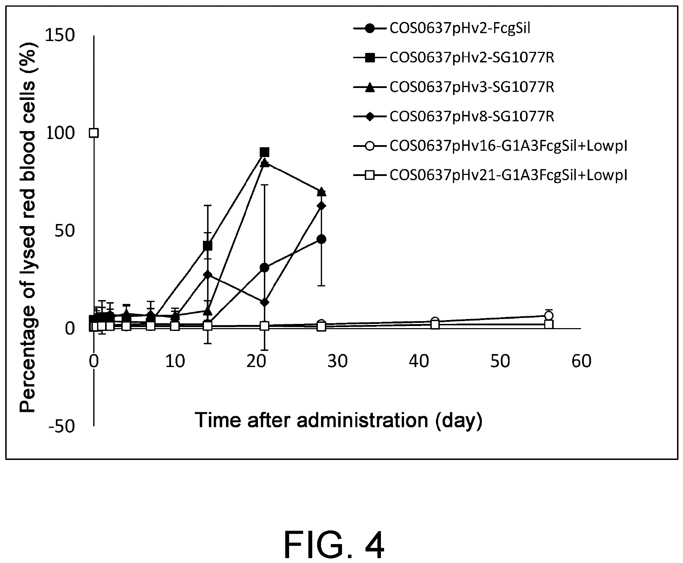

Fig. 4 shows the evaluation of anti-C1s antibodies for the function of complement neutralization in monkeys. Complement neutralization activities in monkeys of anti-C1s antibodies (COS0637pHv2-FcgSil, COS0637pHv2-SG1077R, COS0637pHv3-SG1077R, COS0637pHv8-SG1077R, COS0637pHv16-G1A3FcgSil+LowpI, COS0637pHv21-G IA3FcgSil+LowpI) are shown. All specimen suppressed lysis of red blood cells immediately after administration. Suppression of red blood cell lysis was observed up to 56 days after administration for COS0637pHv16-G1A3FcgSil+Low pI and COS0637pHv21-G1A3FcgSil+Low pI.

### [Description of Embodiments]

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J. P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

### I. DEFINITIONS

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y 1992), provide one skilled in the art with a general guide to many of the terms used in the present application. All references cited herein, including patent applications and publications, are incorporated by reference in their entirety.

For purposes of interpreting this specification, the following definitions will apply and whenever appropriate, terms used in the singular will also include the plural and vice versa. It is to be understood that the terminology used herein is for the purpose of describing particular embodiments only, and is not intended to be limiting. In the event that any definition set forth below conflicts with any document incorporated herein by reference, the definition set forth below shall control.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd or KD). Affinity can be measured by common methods known in the art, including those described herein. Specific illustrative and exemplary embodiments for measuring binding affinity are described in the following. "Affinity", "binding affinity", "binding ability", and "binding activity" may be used interchangeably. The term "binding activity" refers to the strength of the sum total of noncovalent interactions between a single or more binding sites of a molecule (e.g., an antibody) and its binding partner (e.g., an antigen). Herein, binding activity is not strictly limited to an activity which reflects a 1:1 interaction between members of a binding pair (e.g., antibody and antigen). When members of a binding pair can bind to each other in the manner of both monovalent and multivalent binding, binding activity is the strength of the sum total of these bindings. The binding activity of a molecule X for its partner Y can generally be represented by the dissociation constant (KD). Alternatively, the association and dissociation rates (Kon and Koff) may be used for the assessment of binding. Binding activity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-C1s antibody", "an antibody that binds to C1s", and "antibody having binding activity to C1s" refer to an antibody that is capable of binding C1s with sufficient affinity such that the antibody is useful as a diagnostic and/or therapeutic agent in targeting C1s. In one embodiment, the extent of binding of an anti-C1s antibody to an unrelated, non-C1s protein is less than about 10% of the binding of the antibody to C1s as measured, e.g., by a radioimmunoassay (RIA). In certain embodiments, an antibody that binds to C1s has a dissociation constant (Kd) of 1 micromolar (micro M) or less, 100 nM or less, 10 nM or less, 1 nM or less, 0.1 nM or less, 0.01 nM or less, or 0.001 nM or less (e.g. 10⁻⁸ M or less, e.g. from 10⁻⁸M to 10⁻¹³ M, e.g., from 10⁻⁹ M to 10⁻¹³ M). In certain embodiments, an anti-C1s antibody binds to an epitope of C1s that is conserved among C1s from different species.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called alpha, delta, epsilon, gamma, and mu, respectively.

The term "cytotoxic agent" as used herein refers to a substance that inhibits or prevents a cellular function and/or causes cell death or destruction. Cytotoxic agents include, but are not limited to, radioactive isotopes (e.g., ²¹¹At, ¹³¹I, ¹²⁵I, ⁹⁰Y, ¹⁸⁶Re, ¹⁸⁸Re, ¹⁵³Sm, ²¹²Bi, ³²P, ²¹²Pb and radioactive isotopes of Lu); chemotherapeutic agents or drugs (e.g., methotrexate, adriamycin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "epitope" includes any determinant capable of being bound by an antibody. An epitope is a region of an antigen that is bound by an antibody that targets that antigen, and includes specific amino acids that directly contact the antibody. Epitope determinants can include chemically active surface groupings of molecules such as amino acids, sugar side chains, phosphoryl or sulfonyl groups, and can have specific three dimensional structural characteristics, and/or specific charge characteristics. Generally, antibodies specific for a particular target antigen will preferentially recognize an epitope on the target antigen in a complex mixture of proteins and/or macromolecules.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (residues 446-447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the FR and HVR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., supra. In one embodiment, for the VH, the subgroup is subgroup III as in Kabat et al., supra.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs, that is, comprise total six HVRs, three in the VH (H1, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., supra.

An "immunoconjugate" is an antibody conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-C1s antibody" or "isolated nucleic acid encoding an anti-C1r antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies composing the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa and lambda, based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR) software, or GENETYX (registered trademark) (Genetyx Co., Ltd.). Those skilled in the art can determine appropriate parameters for aligning sequences, including any algorithms needed to achieve maximal alignment over the full length of the sequences being compared.

The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

The phrase "specifically bind to", as used herein, refers to an activity or a characteristic of an antibody to bind to an antigen of no interest at a level of binding that includes background (i.e., non-specific) binding but does not include significant (i.e., specific) binding. In other words, "specifically bind to" refers to an activity or a characteristic of an antibody to bind to an antigen of interest at a level of binding that includes significant (i.e., specific) binding in addition to or in place of background (i.e., non-specific) binding. The specificity can be measured by any methods mentioned in this specification or known in the art. The above-mentioned level of non-specific or background binding may be zero, or may not be zero but near zero, or may be very low enough to be technically neglected by those skilled in the art. For example, when a skilled person cannot detect or observe any significant (or relatively strong) signal for binding between the antibody and the antigen of no interest in a suitable binding assay, it can be said that the antibody "does not specifically bind to" the antigen of no interest. In contrast, when a skilled person can detect or observe any significant (or relatively strong) signal for binding between the antibody and the antigen of interest in a suitable binding assay, it can be said that the antibody "specifically binds to" the antigen of interest.

The term "C1s," as used herein, refers to any native C1s from any vertebrate source, including mammals such as primates (e.g. humans) and rodents (e.g., mice and rats), unless otherwise indicated. The term encompasses "full-length" unprocessed C1s as well as any form of C1s that results from processing in the cell. The term also encompasses naturally occurring variants of C1s, e.g., splice variants or allelic variants. The amino acid sequence of an exemplary human C1s is shown in SEQ ID NO: 1. The amino acid sequence of an exemplary cynomolgus monkeyC1s is shown in SEQ ID NO: 3.

The term "C1r," as used herein, refers to any native C1r from any vertebrate source, including mammals such as primates (e.g. humans) and rodents (e.g., mice and rats), unless otherwise indicated. The term encompasses "full-length" unprocessed C1r as well as any form of C1r that results from processing in the cell. The term also encompasses naturally occurring variants of C1r, e.g., splice variants or allelic variants. The amino acid sequence of an exemplary human C1r is shown in SEQ ID NO: 2. The amino acid sequence of an exemplary cynomolgus monkey C1r is shown in SEQ ID NO: 4.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

### II. ANTIBODY

In one aspect, the invention is based, in part, on an antibody that comprises an antigen-binding region and an antibody constant region. In certain embodiments, antibodies that bind to C1s are provided. In certain embodiments, antibodies that specifically bind to C1s are provided. Antibodies of the invention are useful, e.g., for the diagnosis or treatment of complement-mediated disease or disorder.

In one embodiments, species of the C1s may be selected from one species or more. In particular embodiments, the species are human and non-human animal. In particular embodiments, the species are human, rat, and monkey (e.g. cynomolgus, rhesus macaque, marmoset, chimpanzee, and baboon). In particular embodiments, the species are human and monkey (e.g. cynomolgus, rhesus macaque, marmoset, chimpanzee, and baboon). In particular embodiments, the species are human and cynomolgus.

In the embodiments, the antibodies encompass various types of antibody including antibody fragments, chimeric and humanized antibodies, human antibodies, library-derived antibodies, and multispecific antibodies. In the embodiment, the antibody may be a full length antibody, e.g., an intact IgG1, IgG2, IgG3 or IgG4 antibody or other antibody class or isotype as defined herein.

### (Antibody Fragments)

In certain embodiments, an antibody provided herein is an antibody fragment. Antibody fragments include, but are not limited to, Fab, Fab', Fab'-SH, F(ab')₂, Fv, and scFv fragments, and other fragments described below. For a review of certain antibody fragments, see Hudson et al. Nat. Med. 9: 129-134 (2003). For a review of scFv fragments, see, e.g., Pluckthun, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., U.S. Patent No. 6,248,516 B1).

Antibody fragments can be made by various techniques, including but not limited to proteolytic digestion of an intact antibody as well as production by recombinant host cells (e.g. E. coli or phage), as described herein.

### (Chimeric and Humanized Antibodies)

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### (Human Antibodies)

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23: 1117-1125 (2005). See also, e.g., U.S. Patent Nos. 6,075,181 and 6,150,584 describing XENOMOUSE (registered trademark) technology; U.S. Patent No. 5,770,429 describing HUMAB (registered trademark) technology; U.S. Patent No. 7,041,870 describing K-M MOUSE (registered trademark) technology, and U.S. Patent Application Publication No. US 2007/0061900, describing VELOCIMOUSE (registered trademark) technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3): 185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### (Library-Derived Antibodies)

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### (Multispecific Antibodies)

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for C1s and the other is for any other antigen. In certain embodiments, bispecific antibodies may bind to two different epitopes of C1s. Bispecific antibodies may also be used to localize cytotoxic agents to cells which express C1s. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bispecific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (scFv) dimers (see, e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting Fab" or "DAF" comprising an antigen binding site that binds to C1s as well as another, different antigen (see, US 2008/0069820, for example).

### A. Isolated antibody

In certain embodiments, the antibody is an isolated antibody. In the embodiment, the isolated antibody comprises an antigen-binding region and an antibody constant region.

In the embodiment, the isolated antibody may have a displacement function such that the antibody specifically binds to C1s and promotes dissociation of C1q from C1qrs complex. In the embodiment, the isolated antibody may have a blocking function such that the antibody specifically binds to C1s and inhibits binding of C1q to C1r2s2. The isolated antibody may have either or both of the displacement function and the blocking function. The antibody preferably has both of the functions.

In one embodiment, the isolated antibody specifically binds to C1s in a pH-dependent manner. As a particular example of the embodiment, in a case where a binding activity of the antibody to human and/or cynomolgus C1s is measured by surface plasmon resonance,
i) a dissociation constant (KD) value in neutral pH range can be reliably calculated, and a KD value in acidic pH range cannot be reliably calculated due to no binding activity or considerably low binding activity, or
ii) a ratio of a KD value in acidic pH range to a KD value in neutral pH range, an acidic KD/ neutral KD ratio, is more than 10 provided that both of the KD values in neutral pH range and in acidic pH range can be reliably calculated.

Such antibodies are expected to be especially superior as pharmaceuticals, because the dose and frequency of administration in patients can be reduced and as a result the total dosage can be reduced. Anti-C1s antibodies are expected to have superior safety profile compared to antibodies that bind to and remove C1qrs complexes from plasma, as they will only remove C1r2s2 (through the binding to C1s) from plasma not but C1q from plasma. As a result, side effects associated with C1q depletion can be avoided. In addition, antibodies with rapid displacement of C1q are expected to have faster neutralization of complement activity, which can translate to faster onset of treatment efficacy.

### (a1) BIACORE (registered trademark) /Displacement concept

In one embodiment, an isolated antibody that inhibits the interaction between C1q and C1r2s2 complex is an antibody binding to C1qrs complex on a chip for surface plasmon resonance assay, e.g., a BIACORE (registered trademark) chip and promotes dissociation of C1q from C1qrs complex. In some embodiment, a function of binding to C1qrs complex and promoting dissociation of C1q from C1qrs complex mentioned above is herein called "displacement function/activity" or "C1q displacement function/activity". The function/activity can be suitably assessed qualitatively or quantitatively using a surface plasmon resonance assay, for example, a BIACORE (registered trademark) assay as described herein. In further embodiments, the antibody can be determined as an antibody having a displacement function when a value of response unit (RU) in the presence of the antibody is lower than a value of response unit (RU) in the absence of the antibody as determined by surface plasmon resonance assay, for example a BIACORE (registered trademark) assay, when a sufficient time passed. In a sensorgram obtained from such an assay, one can identify that the curve in the presence of C1q and the antibody approaches the curve in the presence of C1q with the absence of the antibody. In further embodiments, one can identify a "time point of crossover" where the curve in the presence of C1q with the absence of the antibody crosses the curve in the presence of C1q and the antibody (see the Examples for detail). To be strict, multiple time points of crossover may be observed even in a single sensorgram due to noise or oscillation of the latter curve when crossing the former curve. In such a case, any of the multiple time points of crossover may be selected as the "time point of crossover". "Passing a sufficient time" means that the time point of the measurement of the value of response unit (RU) is sufficiently after the "time point of crossover" for the purpose of the measurement. In some embodiments, the time point of the measurement of the value of response unit (RU) is at least 60s, 100s, 150s, 200s, 500s, 700s, 1000s, 1500s or 2000s after the time point of the start of antibody injection. Alternatively, the time point of the measurement may be at least 100s, 200s, 300s, 400s, 500s, 600s, 700s, 800s, 900s, 1000s, 3000s, 5000s, 7000s, or 10000s after the time point of crossover.

In one embodiment, an isolated antibody that inhibits the interaction between C1q and C1r2s2 complex can be determined as an antibody having a displacement function when the time point of crossover (e.g., in a BIACORE (registered trademark) assay) is within 60s, 100s, 150s, 200s, 500s, 700s, 1000s, 1500s, or 2000s after the time point of the start of antibody injection, as determined by, for example, a BIACORE (registered trademark) assay using the following conditions: The capture levels of C1r2s2 complex and C1q are at 200 resonance unit (RU) and 200 resonance unit (RU), respectively, and the antibody as an analyte is injected at 500 nM at 10 microliter (micro L)/min.

In one embodiment, an isolated antibody that inhibits the interaction between C1q and C1r2s2 complex can be determined as an antibody having a displacement function when almost all (or all) of C1q are dissociated from C1qrs complex within 100s, 300s, 500s, 700s, 1000s, 1500s, 2000s, 3000s, 5000s, 7000s, or 10000s after the time point of the start of antibody injection, as determined by, for example a BIACORE (registered trademark) assay using the following conditions: The capture levels of C1r2s2 complex and C1q are at 200 resonance unit (RU) and 200 resonance unit (RU), respectively, and the antibody as an analyte is injected at 500 nM at 10 micro L/min. For example, in a sensorgram obtained from such an assay, it can be determined that "almost all (or all) of C1q are dissociated from C1qrs complex" when the value (RU) in the presence of C1q and the antibody comes close to or reaches the value (RU) in the presence of the antibody with the absence of C1q. Herein, "almost all (of C1q)" refers to a percentage of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more; and "all (of C1q)" refers to a percentage of 100%. The percentage of dissociated C1q may be quantitatively determined by any assay described herein. In some embodiments, the present invention provides a method of screening for an antibody that displaces C1q from C1r2s2 complex, using the above-mentioned method of measuring the "displacement function/activity" of such an antibody. In one embodiment, the screening method comprises selecting an antibody that inhibits the interaction between C1q and C1r2s2 complex; i.e., selecting an antibody that binds to C1qrs complex and promotes dissociation of C1q from C1qrs complex. The antibody having the displacement function/activity can be suitably selected using a surface plasmon resonance assay, for example, a BIACORE (registered trademark) assay as described herein. In some embodiments, the screening method comprises determining (i) a value of response unit (RU) in presence of the antibody and (ii) a value of response unit (RU) in the absence of the antibody, by surface plasmon resonance assay, for example a BIACORE (registered trademark) assay, when a sufficient time passed. The screening method may comprise comparing the value of (i) above and the value of (ii) above. The screening method may comprise selecting the antibody when the value of (i) above is lower than the value of (ii) above. The screening method may comprise identifying a "time point of crossover" where the curve in the presence of C1q with the absence of the antibody crosses the curve in the presence of C1q and the antibody. As mentioned above, multiple time points of crossover may be observed even in a single sensorgram, and any of the multiple time points of crossover may be selected as the "time point of crossover". In some embodiments, the screening method may comprise measuring the value of response unit (RU) at at least 60s, 100s, 150s, 200s, 500s, 700s, 1000s, 1500s or 2000s after the time point of the start of antibody injection. Alternatively, the screening method may comprise measuring the value of response unit (RU) at at least 100s, 200s, 300s, 400s, 500s, 600s, 700s, 800s, 900s, 1000s, 3000s, 5000s, 7000s, or 10000s after the time point of crossover. In some embodiments, the screening method may comprise selecting an antibody that inhibits the interaction between C1q and C1r2s2 complex or an antibody having a displacement function, when the time point of crossover of the antibody is within 60s, 100s, 150s, 200s, 500s, 700s, 1000s, 1500s, or 2000s after the time point of the start of antibody injection, as determined by, for example, a BIACORE (registered trademark) assay using the following conditions: The capture levels of C1r2s2 complex and C1q are at 200 resonance unit (RU) and 200 resonance unit (RU), respectively, and the antibody as an analyte is injected at 500 nM at 10 microliter (micro L)/min. In some embodiments, the screening method may comprise selecting an antibody that inhibits the interaction between C1q and C1r2s2 complex or an antibody having a displacement function, when almost all (or all) of C1q are dissociated from C1qrs complex within 100s, 300s, 500s, 700s, 1000s, 1500s, 2000s, 3000s, 5000s, 7000s, or 10000s after the time point of the start of antibody injection, as determined by, for example a BIACORE (registered trademark) assay using the following conditions: The capture levels of C1r2s2 complex and C1q are at 200 resonance unit (RU) and 200 resonance unit (RU), respectively, and the antibody as an analyte is injected at 500 nM at 10 micro L/min. As mentioned above, "almost all (of C1q)" refers to a percentage of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, and "all (of C1q)" refers to 100%, and the percentage of dissociated C1q may be quantitatively determined by any assay described herein including a BIACORE (registered trademark) assay.

### (a2) BIACORE (registered trademark) Blocking concept

In one embodiment, the invention provides isolated antibodies that inhibit the interaction between C1q and C1r2s2 complex, wherein the antibody has a blocking function such that the antibody binds to C1r2s2 and inhibits the binding of C1q to C1r2s2. In further embodiment, the antibody has the blocking ratio at least 60%, 65%, 70%, 75%, 80%, 85%, 90%, 95% or more. The blocking function/activity or blocking ratio can be determined by using a BIACORE (registered trademark) assay. The following conditions can be used for evaluating the level of C1q blocking: The capture levels of C1r2s2 are aimed at 50, 100, 200, 400 resonance unit (RU). Antibody variants are injected at 250, 500, 1000, 2000 nM to saturate antibody binding, followed by human C1q injection at 50, 100, 200 nM with or without antibody variants at 250, 500, 1000, 2000 nM. The blocking ratio is calculated by the following formula: [1- (human C1q binding response in the presence of antibody variant / human C1q binding response in the absence of antibody variant)] x 100%.

### (a3) pH dependency

In one embodiment, the isolated antibody specifically binds to C1s in a pH-dependent manner. In a preferred embodiment, the binding activity of the antibody to C1s is lower in acidic pH range (e.g., at pH 6.0) than in neutral pH range (e.g., at pH 7.4).

In the embodiment, the antibody may have considerably low binding to C1s in acidic pH range such that, when binding activity is measured by surface plasmon resonance and a dissociation constant (KD) value is calculated by the data, the KD value in acidic pH range possesses lower reliability or the binding activity to C1s in acidic pH range is undetectable; that is, in a case where a binding activity of the antibody to human and/or cynomolgus C1s is measured by surface plasmon resonance,
i) a KD value in neutral pH range can be reliably calculated, and a KD value in acidic pH range cannot be reliably calculated due to no binding activity or considerably low binding activity, or
ii) a ratio of a KD value in acidic pH range to a KD value in neutral pH range, an acidic KD/ neutral KD ratio, is more than 10 provided that both of the KD values in neutral pH range and in acidic pH range can be reliably calculated.

In the embodiment, the acidic KD/ neutral KD ratio of ii) is preferably 14 or more, 44 or more, 45 or more, 72 or more, 99 or more, 100 or more, 107 or more, 110 or more, 117 or more, 120 or more, 138 or more, 181 or more, 209 or more, 225 or more, 278 or more. In the embodiment, the acidic KD/ neutral KD ratio of ii) is more preferably 44 or more, 45 or more, 72 or more, 99 or more, 100 or more, 107 or more, 110 or more, 117 or more, 120 or more, 138 or more, 181 or more, 209 or more, 225 or more, 278 or more.

What the word 'reliably' in the case means is described as below. The KD value of each samples at pH 7.4 and pH 6.0 are determined at 37 degrees C using BIACORE (registered trademark) T200 instrument (Cytiva). Purified Mouse Anti-Human Ig kappa Light Chain (GE Healthcare) can be immobilized onto all flow cells of a CM5 sensor chip using an amine coupling kit (GE Healthcare). A buffer including 20 mM ACES, 150 mM NaCl, 1.2 mM CaCl₂, 1 mg/mL bovine serum albumin (BSA) (IgG-free), 1 mg/mL CMD (CM-Dextran sodium salt), 0.05% Tween (registered trademark) 20, and 0.005% NaN₃ (pH 7.4 or pH 6.0) is used as a running buffer. Each antibody can be captured onto the sensor surface via Anti-Human Ig kappa Light chain. Antibody capture levels are adjusted to 50 resonance unit (RU). For the KD value at pH7.4, human C1r2s2 complex is prepared such that the protein complex can be injected at 0, 25, 40, 100, 200, 400 nM, 0, 12.5, 25, 40, 100, 200 nM, or 0, 6.3, 12.5, 25, 50, 100 nM, at 30 micro L/min. For the KD value at pH6.0, human or cyno C1r2s2 complex are prepared such that the protein complex can be injected at 0, 200, 400, 800, 1600, 3200 nM, or 0, 50, 100, 200, 400, 800 nM, at 30 micro L/min with, e.g., Glycine pH 2.0 (GE Healthcare). Sensor surface is regenerated each cycle with, e.g., Glycine pH 2.0 (GE Healthcare). The KD values are obtained using BIACORE (registered trademark) T200 Evaluation software, version 2.0 (Cytiva). The KD values at pH6.0 are compared with the KD values at pH7.4 (an acidic KD/ neutral KD ratio). If Quality control result of BIACORE (registered trademark) software mentioned "kinetics constants cannot be uniquely determined" for an antibody, we regarded that KD value of the antibody cannot be reliably calculated.

In one embodiment of the case, the binding activity by surface plasmon resonance is measured at 37 degrees C using a sensor chip onto which each antibody was captured by human Ig kappa light chain at 50 resonance unit and a running buffer that comprises 20 mM ACES (N-(2-Acetamido)-2-aminoethanesulfonic acid), 150 mM NaCl, 1.2 mM CaCl₂, 1 mg/mL bovine serum albumin (BSA), 1 mg/mL CM-Dextran sodium salt (CMD), 0.05% polysorbate 20, 0.005% NaN₃.

In the embodiment, the antibodies do not encompass antibodies whose KD value in neutral pH range cannot be reliably calculated due to no binding activity or considerably low binding activity.

In addition to binding to C1s in a pH-dependent manner, the effect of calcium on a pH-dependent antibody's affinity to C1s may be another important property. C1s forms dimers at high calcium concentrations but dissociates into monomers at low calcium concentrations. When C1s is in a dimeric state, a bivalent antibody is able to form immune complexes by crosslinking multiple C1s molecules. This allows the antibody to bind to C1s molecules within the complex by both affinity and avidity interactions, thus increasing the apparent affinity of the antibody. In contrast, when C1s is in a monomeric state, the antibody only binds by affinity interaction to C1s.This means that the pH-dependent C1s antibody can form immune complex with dimeric C1s in the plasma, but once within the acidic endosome, C1s will dissociate into monomers. This leads to the disassembly of the immune complex which then enhances the pH-dependent dissociation of the antibody from the antigen.

In one aspect, in an isolated anti-C1s antibody, the ratio of the KD value for its C1s-binding activity at acidic pH to the KD value for the C1s-binding activity at neutral pH (KD(acidic pH)/KD(neutral pH)) is more than 10 when measured at a high calcium concentration at both neutral and acidic pH. In one aspect, in an isolated anti-C1s antibody, the ratio of the KD value for its C1s-binding activity at acidic pH to the KD value for the C1s-binding activity at neutral pH (KD(acidic pH)/KD(neutral pH)) is more than 10 when measured at a high calcium concentration at neutral pH and at a low calcium concentration at acidic pH. In some embodiments, in an isolated anti-C1s antibody, the ratio of the KD value for its C1s-binding activity at acidic pH to the KD value for the C1s-binding activity at neutral pH (KD(acidic pH)/KD(neutral pH)) is more than 10 when measured at a low calcium concentration at both neutral and acidic pH, wherein the anti-C1s antibody binds to the dimeric state of C1s.

Without being bound by a particular theory, in case that 1) an epitope structure of C1s bound by the antibody can be conformationally changed by the non-existence of calcium thereby altering the affinity of the antibody or 2) the interaction (affinity or avidity) of the antibody can vary depending on the condition of C1s (a monomeric state or a dimeric state), the measurement by using specific conditions (at a high calcium concentration at neutral pH and at a low calcium concentration at acidic pH) may be used to evaluate the ratio of the KD value (KD(acidic pH)/KD(neutral pH)).

In other words, the antibody binds to C1s with a higher affinity at neutral pH than at acidic pH as described in (i) or (ii) below:
(i) when measured at a high calcium concentration at both neutral and acidic pH, the ratio of the KD value for C1s-binding activity at acidic pH to the KD value for C1s-binding activity at neutral pH (KD(acidic pH)/KD(neutral pH)) is more than 10,
(ii) when measured at a high calcium concentration at neutral pH and at a low calcium concentration at acidic pH, the ratio of the KD value for C1s-binding activity at acidic pH to the KD value for C1s-binding activity at neutral pH (KD(acidic pH)/KD(neutral pH)) is more than 10.

More generally, without being bound by a particular theory, in case that 1) an epitope structure of a certain antigen bound by an antibody can be conformationally changed by the non-existence of calcium thereby altering the affinity of the antibody or 2) the interaction (affinity or avidity) of the antibody can vary depending on the condition of the antigen (a monomeric state or a dimeric state), the measurement by using specific conditions (at a high calcium concentration at neutral pH and at a low calcium concentration at acidic pH) may be used to evaluate the ratio of the KD value (KD(acidic pH)/KD(neutral pH)).

Therefore, the antibody binds to an antigen with a higher affinity at neutral pH than at acidic pH as follows: when measured at a high calcium concentration at neutral pH and at a low calcium concentration at acidic pH, the ratio of the KD value for antigen binding activity at acidic pH to the KD value for antigen binding activity at neutral pH (KD(acidic pH)/KD(neutral pH)) is more than 10.

The above-mentioned KD ratio, i.e., KD(acidic pH)/KD(neutral pH) may be compared between the parent antibody (i.e., the original antibody before modification of this invention) and an antibody into which one or more amino acid mutations (e.g., additions, insertions, deletions, or substitution) have been introduced with respect to the original (parent) antibody. The original (parent) antibody may be any known or newly isolated antibody as long as it specifically binds to C1s. Thus, in one aspect, in an isolated anti-C1s antibody, the ratio of the KD value for the C1s-binding activity at acidic pH to the KD value for the C1s-binding activity at neutral pH (KD(acidic pH)/KD(neutral pH)) is at least 1.2 times, 1.4 times, 1.6 times, 1.8 times, 2 times, 2.5 times, 3 times, 3.5 times, 4 times, 5 times, 8 times, 10 times higher than the ratio of the KD value for the C1s-binding activity at acidic pH to the KD value for the C1s-binding activity at neutral pH (KD(acidic pH)/KD(neutral pH)) of the original (parent) antibody. In other words, the present invention provides an isolated anti-C1s antibody wherein the isolated anti-C1s antibody has been introduced with one or more amino acid mutations (e.g., additions, insertions, deletions, or substitution) from a parent (original) antibody, and the ratio of (i) to (ii) below is at least 1.2, 1.4, 1.6, 1.8, 2, 2.5, 3, 3.5, 4, 5, 8, or 10: (i) the ratio of the KD value for the C1s-binding activity at acidic pH to the KD value for the C1s-binding activity at neutral pH (KD(acidic pH)/KD(neutral pH)) of the isolated antiC1s antibody; (ii) the ratio of the KD value for the C1s-binding activity at acidic pH to the KD value for the C1s-binding activity at neutral pH (KD(acidic pH)/KD(neutral pH)) of the parent (original) antibody. These KD ratios may be measured at any (high or low) calcium concentration, e.g., measured at a high calcium concentration at both neutral and acidic pH, or measured at a high calcium concentration at neutral pH and at a low calcium concentration at acidic pH.

In one aspect, the antibodies have antigen-binding activity which is different between intracellular condition and extracellular condition. Intracellular and extracellular conditions refer to conditions that are different between inside and outside of the cell. Categories of conditions include, for example, ion concentration, more specifically, metal ion concentration, hydrogen ion concentration (pH) and calcium ion concentration. "Intracellular condition" preferably refers to an environment characteristic to the environment inside the endosome, while "extracellular condition" preferably refers to an environment characteristic to the environment in plasma. Antibodies with the property of having an antigen-binding activity that changes according to the ion concentration can be obtained by screening a large number of antibodies for domains having such property. For example, antibodies with the above-described property can be obtained by producing a large number of antibodies whose sequences are different from each another by a hybridoma method or an antibody library method, and measuring their antigen binding activities at different ion concentrations. The B cell cloning method is one of examples of methods of screening for such antibodies. Furthermore, as described below, at least one distinctive amino acid residue that can confer an antibody with the property of having an antigen-binding activity that changes according to the ion concentration is specified, to prepare as a library of a large number of antibodies that have different sequences while sharing the distinctive amino acid residues as a common structure. Such a library can be screened to efficiently isolate antibodies that have the property described above.

In one aspect, the invention provides an antibody that binds to C1s with a higher affinity at neutral pH than at acidic pH. In another aspect, the invention provides anti-C1s antibodies that exhibit pH-dependent binding to C1s. As used herein, the expression "pH-dependent binding" means "reduced binding at acidic pH as compared to at neutral pH", and both expressions may be interchangeable. For example, anti-C1s antibodies "with pH-dependent binding characteristics" include antibodies that bind to C1s with higher affinity at neutral pH than at acidic pH.

In certain embodiment, the ratio of the KD value for C1s-binding activity at acidic pH to the KD value for C1s-binding activity at neutral pH (KD(acidic pH)/KD(neutral pH)) is more than 10 when measured at a high calcium concentration at both neutral and acidic pH. In particular embodiments, the antibodies bind to C1s with at least 11, 15, 20, 25, 30, 35, 40, 45, 50, 55, 60, 65, 70, 75, 80, 85, 90, 95, 100, 200, 400, 1000, 10000, or more times higher affinity at neutral pH than at acidic pH.

In certain embodiment, the ratio of the KD value for C1s-binding activity at acidic pH to the KD value for C1s-binding activity at neutral pH (KD(acidic pH)/KD(neutral pH)) is more than 10 when measured at a high calcium concentration at neutral pH and at a low calcium concentration at acidic pH. In particular embodiments, the antibodies bind to C1s with at least 11, 15, 20, 25, 30, 35, 40, 45, 50, 55, 60, 65, 70, 75, 80, 85, 90, 95, 100, 200, 400, 1000, 10000, or more times higher affinity at neutral pH than at acidic pH.

In the above-mentioned cases, for example, acidic pH is 6.0 and neutral pH is 7.4, thus KD(acidic pH)/KD(neutral pH) is KD(pH 6.0)/KD(pH 7.4). In this connection, examples of acidic pH and neutral pH are herein described in detail later. In some embodiments, KD(acidic pH)/KD(neutral pH) such as KD(pH 6.0)/KD(pH 7.4) may be 11 to 10,000.

When an antigen is a soluble protein, the binding of an antibody to the antigen can result in an extended half-life of the antigen in plasma (i.e., reduced clearance of the antigen from plasma), since the antibody can have a longer half-life in plasma than the antigen itself and may serve as a carrier for the antigen. This is due to the recycling of the antigen-antibody complex by FcRn through the endosomal pathway in cell (Roopenian and Akilesh (2007) Nat Rev Immunol 7(9): 715-725). However, an antibody with pH-dependent binding characteristics, which binds to its antigen in neutral extracellular environment while releasing the antigen into acidic endosomal compartments following its entry into cells, is expected to have superior properties in terms of antigen neutralization and clearance relative to its counterpart that binds in a pH-independent manner (Igawa et al (2010) Nature Biotechnol 28(11); 1203-1207; Devanaboyina et al (2013) mAbs 5(6): 851-859; International Patent Application Publication No: WO 2009/125825).

In one aspect, the invention provides an antibody that binds to C1s with a higher affinity under a high calcium concentration condition than under a low calcium concentration condition.

In one embodiment, preferred metal ions include, for example, calcium ion. Calcium ion is involved in modulation of many biological phenomena, including contraction of muscles such as skeletal, smooth, and cardiac muscles; activation of movement, phagocytosis, and the like of leukocytes; activation of shape change, secretion, and the like of platelets; activation of lymphocytes; activation of mast cells including secretion of histamine; cell responses mediated by catecholamine alpha receptor or acetylcholine receptor; exocytosis; release of transmitter substances from neuron terminals; and axoplasmic flow in neurons. Known intracellular calcium ion receptors include troponin C, calmodulin, parvalbumin, and myosin light chain, which have several calcium ion-binding sites and are believed to be derived from a common origin in terms of molecular evolution. There are also many known calcium-binding motifs. Such well-known motifs include, for example, cadherin domains, EF-hand of calmodulin, C2 domain of Protein kinase C, Gla domain of blood coagulation protein Factor IX, C-type lectins of acyaroglycoprotein receptor and mannose-binding receptor, A domains of LDL receptors, annexin, thrombospondin type 3 domain, and EGF-like domains.

In one embodiment, when the metal ion is calcium ion, it is desirable that the antigen-binding activity is lower under a low calcium ion concentration condition than under a high calcium ion concentration condition. Meanwhile, the intracellular calcium ion concentration is lower than the extracellular calcium ion concentration. Conversely, the extracellular calcium ion concentration is higher than the intracellular calcium ion concentration. In one embodiment, the low calcium ion concentration is preferably 0.1 micromolar (micro M) to 30 micro M, more preferably 0.5 micro M to 10 micro M, and particularly preferably 1 micro M to 5 micro M which is close to the calcium ion concentration in the early endosome *in vivo.* Meanwhile, in one embodiment, the high calcium ion concentration is preferably 100 micro M to 10 micro M, more preferably 200 micro M to 5 mM, and particularly preferably 0.5 mM to 2.5 mM which is close to the calcium ion concentration in plasma (in blood). In one embodiment, it is preferable that the low calcium ion concentration is the calcium ion concentration in endosomes, and the high calcium ion concentration is the calcium ion concentration in plasma. When the level of antigen-binding activity is compared between low and high calcium ion concentrations, it is preferable that the binding of antibodies is stronger at a high calcium ion concentration than at a low calcium ion concentration. In other words, it is preferable that the antigen-binding activity of an antibody is lower at a low calcium ion concentration ion than at a high calcium ion concentration. When the level of binding activity is expressed with the dissociation constant (KD), the value of KD (low calcium ion concentration) / KD (high calcium ion concentration) is greater than 1, preferably 2 or more, still more preferably 10 or more, and yet more preferably 40, 45, 50, 55, 60, 65, 70, 75, 80, 85, 90, 95, 100, 200, 400, 1000, 10000 or more. The upper limit of the value of KD (low calcium ion concentration) / KD (high calcium ion concentration) is not particularly limited, and may be any value such as 100, 400, 1000, or 10000, as long as it can be produced with the techniques of skilled artisans. It is possible to use the dissociation rate constant (kd) instead of KD. When it is difficult to calculate the KD value, the activity may be assessed based on the level of binding response in BIACORE (registered trademark) when analytes are passed at the same concentration. When antigens are passed over a chip immobilized with antibodies, the binding response at a low calcium concentration is preferably 1/2 or less of the binding response at a high calcium concentration, more preferably 1/3 or less, still more preferably 1/5 or less, and particularly preferably 1/10 or less. It is known that in general the *in vivo* extracellular calcium ion concentration (for example, in plasma) is high, and the intracellular calcium ion concentration (for example, in the endosome) is low. Thus, in one embodiment, it is preferable that the extracellular condition is a high calcium ion concentration, and the intracellular condition is a low calcium ion concentration. When the property that the antigen-binding activity is lower under an intracellular calcium ion concentration condition than under an extracellular calcium ion concentration condition is conferred to an antibody, antigens that have bound to the antibody outside of the cell dissociate from the antibody inside the cell, thereby enhancing antigen incorporation into the cell from the outside of the cell. Such antibodies, when administered to the living body, can reduce antigen concentration in plasma and reduce the physiological activity of antigens *in vivo.* Thus, antibodies are useful. Methods of screening for antigen-binding regions or antibodies having a lower antigen-binding activity under a low calcium ion concentration condition than under a high calcium ion concentration condition include, for example, the method described in WO2012/073992 (for example, paragraphs 0200-0213). Methods for conferring antigen-binding regions with the property of binding more weakly to antigens under a low calcium ion concentration condition than under a high calcium ion concentration condition are not particularly limited, and may be carried out by any methods. Specifically, the methods include, for example, methods for substituting at least one amino acid residue in an antigen-binding region with an amino acid residue having metal chelating activity, and/or inserting into an antigen-binding domain at least one amino acid residue having metal chelating activity. Antibodies in which at least one amino acid residue of the antigen-binding region has been substituted with an amino acid residue having metal chelating activity and/or at least one amino acid residue having metal chelating activity has been inserted into the antigen-binding domain are a preferred embodiment of antibodies.

Amino acid residues having metal chelating activity preferably include, for example, serine, threonine, asparagine, glutamine, aspartic acid, and glutamic acid. Furthermore, amino acid residues that change the antigen-binding activity of antigen-binding regions according to the calcium ion concentration preferably include, for example, amino acid residues that form a calcium-binding motif. Calcium-binding motifs are well known to those skilled in the art, and have been described in detail (for example, Springer et al., (Cell (2000) 102, 275-277); Kawasaki and Kretsinger (Protein Prof. (1995) 2, 305-490); Moncrief et al., (J. Mol. Evol. (1990) 30, 522-562); Chauvaux et al., (Biochem. J. (1990) 265, 261-265); Bairoch and Cox (FEBS Lett. (1990) 269, 454-456); Davis (New Biol. (1990) 2, 410-419); Schaefer et al., (Genomics (1995) 25, 638 to 643); Economou et al., (EMBO J. (1990) 9, 349- 354); Wurzburg et al., (Structure. (2006) 14, 6, 1049-1058)). EF hand in troponin C, calmodulin, parvalbumin, and myosin light chain; C2 domain in protein kinase C; Gla domain in blood coagulation protein factor IX; C-type lectin of acyaroglycoprotein receptor and mannose-binding receptor, ASGPR, CD23, and DC-SIGN; A domain in LDL receptor; annexin domain; cadherin domain; thrombospondin type 3 domain; and EGF-like domain are preferably used as calcium-binding motifs.

Antigen-binding regions can contain amino acid residues that change the antigen-binding activity according to the calcium ion concentration, such as the above-described amino acid residues with metal chelating activity and amino acid residues that form a calcium-binding motif. The location of such amino acid residues in the antigen-binding region is not particularly limited, and they may be located at any position as long as the antigen binding activity changes according to the calcium ion concentration. Meanwhile, such amino acid residues may be contained alone or in combination of two or more, as long as the antigen binding activity changes according to the calcium ion concentration. The amino acid residues preferably include, for example, serine, threonine, asparagine, glutamine, aspartic acid, and glutamic acid. When an antigen-binding region is an antibody variable region, the amino acid residues may be contained in the heavy chain variable region and/or the light chain variable region. In a preferred embodiment, the amino acid residues may be contained in the CDR3 of the heavy chain variable region, more preferably at positions 95, 96, 100a, and/or 101 according to Kabat numbering in the CDR3 of the heavy chain variable region.

In another preferred embodiment, the amino acid residues may be contained in the CDR1 of the light chain variable region, more preferably at positions 30, 31, and/or 32 according to Kabat numbering in the CDR1 of the light chain variable region. In still another preferred embodiment, the amino acid residues may be contained in the CDR2 of the light chain variable region, more preferably at position 50 according to Kabat numbering in the CDR2 of the light chain variable region. In yet another preferred embodiment, the amino acid residues may be contained in the CDR3 of the light chain variable region, more preferably at position 92 according to Kabat numbering in the CDR3 of the light chain variable region.

Furthermore, it is possible to combine the above-described embodiments. For example, the amino acid residues may be contained in two or three CDRs selected from the CDR1, CDR2, and CDR3 of the light chain variable region, more preferably at any one or more of positions 30, 31, 32, 50, and/or 92 according to Kabat numbering in the light chain variable region.

A large number of antigen-binding regions that have different sequences while sharing as a common structure the above-described amino acid residues that change the antigen-biding activity according to the calcium ion concentration, are prepared as a library. The library can be screened to efficiently obtain antigen-binding regions with binding activity to a desired antigen, in which their antigen-binding activity changes according to the calcium ion concentration.

The "affinity" of an antibody for C1s, for purposes of the present disclosure, is expressed in terms of the KD of the antibody. The KD of an antibody refers to the equilibrium dissociation constant of an antibody-antigen interaction. The greater the KD value is for an antibody binding to its antigen, the weaker its binding affinity is for that particular antigen. Accordingly, as used herein, the expression "higher affinity at neutral pH than at acidic pH" (or the equivalent expression "pH-dependent binding") means that the KD of the antibody at acidic pH is greater than the KD of the antibody at neutral pH. For example, in the context of the present invention, an antibody is considered to bind to C1s with higher affinity at neutral pH than at acidic pH if the KD of the antibody binding to C1s at acidic pH is more than 10 times compared with the KD of the antibody binding to C1s at neutral pH. Thus, the present invention includes antibodies that bind to C1s at acidic pH with a KD that is at least 11, 15, 20, 25, 30, 35, 40, 45, 50, 55, 60, 65, 70, 75, 80, 85, 90, 95, 100, 200, 400, 1000, 10000, or more times greater than the KD of the antibody binding to C1s at neutral pH. In another embodiment, the KD value of the antibody at neutral pH can be 10⁻⁷ M, 10⁻⁸ M, 10⁻⁹ M, 10⁻¹⁰ M, 10⁻¹¹ M, 10⁻¹² M, or less. In another embodiment, the KD value of the antibody at acidic pH can be 10⁻⁹ M, 10⁻⁸ M, 10⁻⁷ M, 10⁻⁶ M, or greater.

The binding properties of an antibody for a particular antigen may also be expressed in terms of the kd of the antibody. The kd of an antibody refers to the dissociation rate constant of the antibody with respect to a particular antigen and is expressed in terms of reciprocal seconds (i.e., sec⁻¹). An increase in kd value signifies weaker binding of an antibody to its antigen. The present invention therefore includes antibodies that bind to C1s with a higher kd value at acidic pH than at neutral pH. The present invention includes antibodies that bind to C1s at acidic pH with a kd that is at least 11, 15, 20, 25, 30, 35, 40, 45, 50, 55, 60, 65, 70, 75, 80, 85, 90, 95, 100, 200, 400, 1000, 10000, or more times greater than the kd of the antibody binding to C1s at neutral pH. In another embodiment, the kd value of the antibody at neutral pH can be 10⁻² 1/s, 10⁻³ 1/s, 10⁻⁴ 1/s, 10⁻⁵ 1/s, 10⁻⁶ 1/s, or less. In another embodiment, the kd value of the antibody at acidic pH can be 10⁻³ 1/s, 10⁻² 1/s, 10⁻¹ 1/s, or greater.

In certain instances, a "reduced binding at acidic pH as compared to at neutral pH" is expressed in terms of the ratio of the KD value of the antibody at acidic pH to the KD value of the antibody at neutral pH (or vice versa). For example, an antibody may be regarded as exhibiting "reduced binding to C1s at acidic pH as compared to its binding at neutral pH", for purposes of the present invention, if the antibody exhibits an acidic/neutral KD ratio of 10 or more. In certain exemplary embodiments, the acidic/neutral KD ratio for an antibody can be 11, 15, 20, 25, 30, 35, 40, 45, 50, 55, 60, 65, 70, 75, 80, 85, 90, 95, 100, 200, 400, 1000, 10000, or greater. In another embodiment, the KD value of the antibody at neutral pH can be 10⁻⁷ M, 10⁻⁸ M, 10⁻⁹ M, 10⁻¹⁰ M, 10⁻¹¹ M, 10⁻¹² M, or less. In another embodiment, the KD value of the antibody at acidic pH can be 10⁻⁹ M, 10⁻⁸ M, 10⁻⁷ M, 10⁻⁶ M, or greater.

In certain instances, a "reduced binding at acidic pH as compared to at neutral pH" is expressed in terms of the ratio of the kd value of the antibody at acidic pH to the kd value of the antibody at neutral pH (or vice versa). For example, an antibody may be regarded as exhibiting "reduced binding to C1s at acidic pH as compared to its binding at neutral pH", for purposes, if the antibody exhibits an acidic/neutral kd ratio of 2 or greater. In certain exemplary embodiments, the acidic/neutral kd ratio for an antibody can be 11, 15, 20, 25, 30, 35, 40, 45, 50, 55, 60, 65, 70, 75, 80, 85, 90, 95, 100, 200, 400, 1000, 10000, or greater. In another embodiment, the kd value of the antibody at neutral pH can be 10⁻² 1/s, 10⁻³ 1/s, 10⁻⁴ 1/s, 10⁻⁵ 1/s, 10⁻⁶ 1/s, or less. In another embodiment, the kd value of the antibody at acidic pH can be 10⁻³ 1/s, 10⁻² 1/s, 10⁻¹ 1/s, or greater.

As used herein, the expression "acidic pH" means a pH of 4.0 to 6.5. The expression "acidic pH" includes pH values of 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, and 6.5. In particular aspects, the "acidic pH" is 6.0.

As used herein, the expression "neutral pH" means a pH of 6.7 to about 10.0. The expression "neutral pH" includes pH values of 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, and 10.0. In particular aspects, the "neutral pH" is 7.4.

As used herein, the expression "under high calcium concentration condition" or "at a high calcium concentration" means 100 micro M to 10 mM, more preferably 200 micro M to 5 mM, and particularly preferably 0.5 mM to 2.5 mM which is close to the calcium ion concentration in plasma (in blood). The expression "under high calcium concentration condition" or "at a high calcium concentration" includes calcium concentration values of 100 micro M, 200 micro M, 300 micro M, 400 micro M, 500 micro M, 600 micro M, 700 micro M, 800 micro M, 900 micro M, 0.5 mM, 0.7 mM, 0.9 mM, 1 mM, 1.2 mM, 1.4 mM, 1.6 mM, 1.8 mM, 2.0 mM, 2.2 mM, 2.4 mM, 2.5 mM, 3 mM, 4 mM, 5 mM, 6 mM, 7 mM, 8 mM, 9 mM, and 10 mM Ca²⁺. In particular aspects, "under high calcium concentration condition" or "at a high calcium concentration" refers to 1.2 mM Ca²⁺.

As used herein, the expression "under low calcium concentration condition" or "at a low calcium concentration" means 0.1 micro M to 30 micro M, more preferably 0.5 micro M to 10 micro M, and particularly preferably 1 micro M to 5 micro M which is close to the calcium ion concentration in the early endosome *in vivo.* The expression "under low calcium concentration condition" or "at a low calcium concentration" includes calcium concentration values of 0.1 micro M, 0.5 micro M, 1 micro M, 1.5 micro M, 2.0 micro M, 2.5 micro M, 2.6 micro M, 2.7 micro M, 2.8 micro M, 2.9 micro M, 3.0 micro M, 3.1 micro M, 3.2 micro M, 3.3 micro M, 3.4 micro M, 3.5 micro M, 4.0 micro M, 5.0 micro M, 6.0 micro M, 7.0 micro M, 8.0 micro M, 9.0 micro M, 10 micro M, 15 micro M, 20 micro M, 25 micro M, and 30 micro M Ca²⁺. In particular aspects, "under low calcium concentration condition" or "at a low calcium concentration" refers to 3.0 micro M Ca²⁺.

KD values and kd values, as expressed herein, may be determined using a surface plasmon resonance-based biosensor to characterize antibody-antigen interactions. (See, e.g., Example 5, herein). KD values and kd values can be determined at 25 degrees Celsius (C) or 37 degrees C. This determination can be performed in the presence of 150 mM NaCl. In some embodiments, this determination can be performed by using a surface plasmon resonance technique in which the antibody is immobilized, the antigen serves as analyte, and the following conditions are used: 20 mM ACES and 150mM NaCl at 37 degrees Celsius (C).

In one aspect, the invention provides a method of enhancing the clearance of C1s from plasma in an individual. In some embodiments, the method comprises administering to the individual an effective amount of an anti-C1s antibody to enhance the clearance of C1s from plasma. The invention also provides a method of enhancing the clearance of the complex of C1r and C1s from plasma in an individual. In some embodiments, the method comprises administering to the individual an effective amount of an anti-C1s antibody to enhance the clearance of the complex of C1r and C1s from plasma. In some embodiments, the method comprises administering to the individual an effective amount of an anti-C1s antibody to enhance the clearance of C1r2s2 from plasma. In some embodiments, the method comprises administering to the individual an effective amount of an anti-C1s antibody to enhance the clearance of C1r2s2 from plasma not but C1q from plasma.

In another aspect, the invention provides a method of removing C1s from plasma, the method comprising: (a) identifying an individual in need of having C1s removed from the individual's plasma; (b) providing an antibody that binds to C1s through the antigen-binding (C1s-binding) domain of the antibody and has a KD(pH6.0)/KD(pH7.4) value, defined as the ratio of KD for C1s at pH 6.0 and KD for C1s at pH 7.4, of 11 to 10,000, when KD is determined using a surface plasmon resonance technique, wherein the antibody binds to C1s in plasma in vivo and dissociates from the bound C1s under conditions present in an endosome in vivo, and wherein the antibody is a human IgG or a humanized IgG; and (c) administering the antibody to the individual. In further aspect, such a surface plasmon resonance technique can be used at 37 degrees C and 150 mM NaCl. In further aspect, such a surface plasmon resonance technique can be used in which the antibody is immobilized, the antigen serves as analyte, and the following conditions are used: 20 mM ACES and 150mM NaCl at 37 degrees C.

In another aspect, the invention provides a method of removing C1s from plasma in a subject, the method comprising: (a) identifying a first antibody that binds to C1s through the antigen-binding region of the first antibody; (b) identifying a second antibody that: (1) binds to C1s through the antigen-binding (C1s-binding) domain of the second antibody, (2) is identical in amino acid sequence to the first antibody except having at least one amino acid of a variable region of the first antibody substituted with histidine and/or at least one histidine inserted into a variable region of the first antibody, (3) has a KD(pH6.0)/KD(pH7.4) value that is higher than the first antibody's KD(pH6.0)/KD(pH7.4) value, and is between 11 and 10,000, wherein KD(pH6.0)/KD(pH7.4) is defined as the ratio of KD for C1s at pH 6.0 and KD for C1s at pH 7.4 when KD is determined using a surface plasmon resonance technique, (4) binds to C1s in plasma in vivo, (5) dissociates from the bound C1s under conditions present in an endosome in vivo, and (6) is a human IgG or a humanized IgG; (c) identifying a subject in need of having his or her plasma level of C1s reduced; and (d) administering the second antibody to the subject so that the plasma level of C1s in the subject is reduced. In further aspect, such a surface plasmon resonance technique can be used at 37 degrees C and 150 mM NaCl. In further aspect, such a surface plasmon resonance technique can be used at 37 degrees C and 150 mM NaCl. In further aspect, such a surface plasmon resonance technique can be used in which the antibody is immobilized, the antigen serves as analyte, and the following conditions are used: 20 mM ACES buffer and 150mM NaCl at 37 degrees C.

In another aspect, the invention provides a method of removing C1s from plasma in a subject, the method comprising: (a) identifying a first antibody that: (1) binds to C1s through the antigen-binding region of the first antibody, (2) is identical in amino acid sequence to a second antibody that binds to C1s through the antigen-binding (C1s-binding) domain of the second antibody, except that at least one variable region of the first antibody has at least one more histidine residue than does the corresponding variable region of the second antibody, (3) has a KD(pH6.0)/KD(pH7.4) value that is higher than the second antibody's KD(pH6.0)/KD(pH7.4) value, and is between 11 and 10,000, wherein KD(pH6.0)/KD(pH7.4) is defined as the ratio of KD for C1s at pH 6.0 and KD for C1s at pH 7.4 when KD is determined using a surface plasmon resonance technique, (4) binds to C1s in plasma in vivo, (5) dissociates from the bound C1s under conditions present in an endosome in vivo, and (6) is a human IgG or a humanized IgG; (b) identifying a subject in need of having his or her plasma level of C1s reduced; and (c) administering the first antibody at least once to the subject so that the plasma level of C1s in the subject is reduced. In further aspect, such a surface plasmon resonance technique can be used at 37 degrees C and 150 mM NaCl. In further aspect, such a surface plasmon resonance technique can be used at 37 degrees C and 150 mM NaCl. In further aspect, such a surface plasmon resonance technique can be used in which the antibody is immobilized, the antigen serves as analyte, and the following conditions are used: 20 mM ACES and 150mM NaCl at 37 degrees C. In some cases, the antibody inhibits a component of the classical complement pathway; in some cases, the classical complement pathway component is Cls.

### (a4) pI of isolated antibody

In one embodiment, an isoelectric point (pI) of an isolated antibody is decreased by alteration of a constant region. In this embodiment, the isolated antibody with decreased pI comprises at least one amino acid alteration (e.g., amino acid addition, insertion, deletion, or substitution) in the constant region compared to the parent constant region. In further embodiments, each amino acid alteration decreases the isoelectric point (pI) of the constant region as compared to that of the parent constant region. In further embodiments, the amino acid can be exposed on the surface of the region. pI may be compared between a parent antibody (original antibody before alteration of the present invention) and the antibody after alteration of the present invention in which one or more amino acid mutations (e.g., additions, insertions, deletions, or substitutions) are introduced into an antibody constant region (parent constant region) of the original (parent) antibody. The amino acid alterations decrease the isoelectric point (pI) of the mutant constant region compared to that of the parent constant region. That is, the antibody of the present invention has a mutant constant region comprising at least one amino acid alteration, wherein the amino acid alteration decreases the isoelectric point (pI) of the mutant constant region as compared to that of the parent constant region. The original (parent) antibody may be any known or newly isolated antibody as long as it specifically binds to C1s. In one aspect, pI of the altered anti-C1s antibody is at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, or 2.0 lower than pI of the original (parent) antibody. pI of the altered anti-C1s antibody is preferably 0.6, 0.7, 0.8, 0.9, or 1.0 lower, more preferably 1.1, 1.2, 1.3, 1.4, or1.5 lower, and further preferably 1.6, 1.7, 1.8, 1.9, or 2.0 lower than pI of the original (parent) antibody. In further embodiments, an isolated antibody comprises a constant region and an antigen-binding domain. In further embodiments, antigen-binding activity of the antigen-binding domain varies depending on ion concentration condition.

In further embodiments, the constant region with decreased pI of the present invention comprises at least one amino acid alteration at at least one position selected from the group consisting of: 137, 268, 274, 285, 311, 312, 315, 318, 333, 335, 337, 341, 342, 343, 355, 384, 385, 388, 390, 399, 400, 401, 402, 413, 419, 420, 422, and 431 according to EU numbering. Preferably, a mutant constant region with decreased pI comprises an amino acid alteration at at least one of positions 137, 268, 274, 355, and 419 according to EU numbering. In further embodiments, in the constant region with decreased pI, an amino acid at each of the positions selected is substituted with any one of arginine, glutamic acid, serine, and glutamine.

In specific embodiment, in the constant region with decreased pI, amino acids at positions 137, 268, 274, 355, and 419 (all numbers are according to EU numbering system) are substituted with any one of arginine, glutamic acid, serine, and glutamine.

In one embodiment, an isoelectric point (pI) of the isolated antibody can be also decreased by alteration of the heavy chain variable region and/or the light chain variable region. In the embodiments, the isolated antibody with decreased pI comprises at least one amino acid alteration in the heavy chain variable region and/or the light chain variable region compared to its parent region. Such decreased pI by alteration of the heavy chain variable region and/or the light chain variable region and/or decreased pI by alteration of the constant region can contribute to improvement of PK of the isolated antibody.

In one embodiment of the isolated antibody, pI of the antibody is 7.8 or less, 7.7 or less, 7.6 or less, or 7.5 or less. pI is preferably 7.7 or less, more preferably 7.6 or less, further preferably 7.5 or less. When the pI is less than or equal to either value of them, half-life of the antibodies in blood is prolonged. On the other hand, a possible minimum value of the pI is 4.28 or more by ordinary.

In one embodiment, pI of the antibody can be measured by capillary isoelectric focusing (cIEF). As an example of the embodiment, cIEF is performed on a Protein Simple iCE3 whole-capillary imaging system using fluorocarbon-coated capillary cartridges. The anolyte and catholyte solutions are 0.08 M phosphoric acid in 0.1% m/v methyl cellulose (MC) and 0.1 M sodium hydroxide in 0.1% m/v MC, respectively. All analyzed samples contain a working 0.2 mg/mL antibody, 0.35% m/v MC, 6 mM IDA (iminodiacetic acid), 10 mM arginine, 0.5 w/v % pI marker (5.85) and 0.5 w/v % pI marker (9.99), and 2 vol % pharmalyte 8-10.5, and 2 vol % pharmalyte 5-8. All samples are vortexed and centrifuged briefly prior to loading into the autosampler compartment. Samples are incubated in autosampler for 2 hours before starting the measurement. Focusing at conditions of 1.5 kV for 1 min is followed by 3.0 kV for either 7 min. The autosampler compartment is kept at 10 degrees C. Measurements are repeated two times for each samples, and pI values of each samples were obtained by calculating the average of n=2 measurements.

Alternatively, in one embodiment, pI of the antibody can be measured by capillary isoelectric focusing (cIEF). As an example of the embodiment, cIEF is performed on a Protein Simple iCE3 whole-capillary imaging system using fluorocarbon-coated capillary cartridges. The anolyte and catholyte solutions are 0.08 M phosphoric acid in 0.1% m/v methyl cellulose (MC) and 0.1 M sodium hydroxide in 0.1% m/v MC, respectively. All analyzed samples contain a working 0.35% m/v MC, 4 mM IDA (iminodiacetic acid), 10 mM arginine, pI markers (3.21 or 4.22 or 4.65 or 5.12 or 5.85 or 6.14 or 6.61 or 7.05 or 7.65 or 8.40 or 8.79 or 9.46 or 9.77 or 10.1), and one of the following v/v mixtures of 4% pharmalyte 3-10. All samples are vortexed and centrifuged briefly prior to loading into the autosampler compartment. Focusing at conditions of 1.5 kV for 1 min is followed by 3.0 kV for either 8 min. The autosampler compartment is kept at 10 degrees C. Measurements are repeated two times for each samples, and pI values of each samples were obtained by calculating the average of n=2 measurements.

In one embodiment of the case, the pI is measured by capillary isoelectric focusing, in which a solution containing 0.08 M phosphoric acid in 0.1% m/v methyl cellulose (MC) is used as an anolyte solution, a solution containing 0.1 M sodium hydroxide in 0.1% m/v MC is used as a catholyte solution, and a solution containing 0.5 mg/mL antibody, 0.3% m/v MC, 6.0 mM iminodiacetic acid (IDA), 10 mM arginine, 4 M urea, and pI markers (7.65 and 9.77) is used as a working solution to lyse antibody.

### (a5) Antigen-binding region

In one embodiment, the antibody comprises an antigen-binding region. In a preferred embodiment, the antigen-binding region can specifically bind to an epitope within a CUB1-EGF-CUB2 domain of C1s. In a further preferred embodiment, the antigen-binding region can specifically bind to a CUB1-EGF-CUB2 domain of C1s. In these embodiments, the C1s includes but not limited to a human C1s. The C1s is preferably a human C1s.

In one embodiment, the antigen-binding region may be an antibody variable region. The antibody variable region may be whole or partial of antibody variable region as long as the antigen-binding region does not destroy the property of the isolated antibody, such as the displacement function and/or the blocking function, and the following binding activity of the antibody;
in a case where a binding activity of the antibody to human and/or cynomolgus C1s is measured by surface plasmon resonance,
i) a dissociation constant (KD) value in neutral pH range can be reliably calculated, and a KD value in acidic pH range cannot be reliably calculated due to no binding activity or considerably low binding activity, or
ii) a ratio of a KD value in acidic pH range to a KD value in neutral pH range, an acidic KD/ neutral KD ratio, is more than 10 provided that both of the KD values in neutral pH range and in acidic pH range can be reliably calculated.

In the embodiment, the antibody variable region is humanized. The antigen-binding region is preferably a humanized antibody variable region. It is expected that side effect is avoided compared with non-humanized antibody when such humanized antibody is used for medication.

In one embodiment, the antigen-binding region of the isolated anti-C1s antibody comprises a combination of HVR-H1, HVR-H2, HVR-H3, HVR-L1, HVR-L2, and HVR-L3 selected from the group consisting of 1) to 6) below:
1) HVR-H1, HVR-H2, HVR-H3, HVR-L1, HVR-L2, and HVR-L3 that comprise the amino acid sequences consisting of SEQ ID NOs: 25, 26, 27, 60, 61, and 62, respectively;
2) HVR-H1, HVR-H2, HVR-H3, HVR-L1, HVR-L2, and HVR-L3 that comprise the amino acid sequences consisting of SEQ ID NOs: 37, 38, 39, 56, 57, and 58, respectively;
3) HVR-H1, HVR-H2, HVR-H3, HVR-L1, HVR-L2, and HVR-L3 that comprise the amino acid sequences consisting of SEQ ID NOs: 25, 26, 27, 56, 57, and 58, respectively;
4) HVR-H1, HVR-H2, HVR-H3, HVR-L1, HVR-L2, and HVR-L3 that comprise the amino acid sequences consisting of SEQ ID NOs: 25, 26, 27, 48, 49, and 50, respectively;
5) HVR-H1, HVR-H2, HVR-H3, HVR-L1, HVR-L2, and HVR-L3 that comprise the amino acid sequences consisting of SEQ ID NOs: 29, 30, 31, 52, 53, and 54, respectively; and
6) HVR-H1, HVR-H2, HVR-H3, HVR-L1, HVR-L2, and HVR-L3 that comprise the amino acid sequences consisting of SEQ ID NOs: 33, 34, 35, 56, 57, and 58, respectively.

In another embodiment of the present invention, an isolated anti-C1s antibody comprises a heavy chain variable region, a light chain variable region and an antibody constant region. In the embodiment, the isolated anti-C1s antibody comprises a heavy chain variable region (VH) and a light chain variable region (VL) selected from the group consisting of 1) to 6) below:
1) a VH and VL that comprise the amino acid sequences consisting of SEQ ID NOs: 24 and 59, respectively;
2) a VH and VL that comprise the amino acid sequences consisting of SEQ ID NOs: 36 and 55, respectively;
3) a VH and VL that comprise the amino acid sequences consisting of SEQ ID NOs: 24 and 55, respectively;
4) a VH and VL that comprise the amino acid sequences consisting of SEQ ID NOs: 24 and 47, respectively;
5) a VH and VL that comprise the amino acid sequences consisting of SEQ ID NOs: 28 and 51, respectively; and
6) a VH and VL that comprise the amino acid sequences consisting of SEQ ID NOs: 32 and 55, respectively.

### (a6) Antibody constant region

In one embodiment, the antibody constant region in the isolated antibody encompasses but not limited to a constant region of human antibody. The constant region of human antibody may include a heavy chain and a light chain. The human antibody encompasses but not limited to human IgG1. The human antibody is preferably human IgG1.

In one embodiment, the antibody constant region comprises at least one amino acid that can increase binding ability of the isolated antibody to FcRn in acidic pH range compared with that of a isolated antibody without said at least one amino acid.

In the embodiment, the constant region comprises
(a) Ala at position 434; Glu, Arg, Ser, or Lys at position 438; and Glu, Asp, or Gln at position 440, according to EU numbering;
(b) Ala at position 434; Arg or Lys at position 438; and Glu or Asp at position 440, according to EU numbering;
(c) Ile or Leu at position 428; Ala at position 434; Ile, Leu, Val, Thr, or Phe at position 436; Glu, Arg, Ser, or Lys at position 438; and Glu, Asp, or Gln at position 440, according to EU numbering;
(d) Ile or Leu at position 428; Ala at position 434; Ile, Leu, Val, Thr, or Phe at position 436; Arg or Lys at position 438; and Glu or Asp at position 440, according to EU numbering;
(e) Leu at position 428; Ala at position 434; Val or Thr at position 436; Glu, Arg, Ser, or Lys at position 438; and Glu, Asp, or Gln at position 440, according to EU numbering; or
(f) Leu at position 428; Ala at position 434; Val or Thr at position 436; Arg or Lys at position 438; and Glu or Asp at position 440, according to EU numbering.

WO2013/046704 specifically reported dual amino acid residue substitutions of Q438R/S440E, Q438R/S440D, Q438K/S440E, and Q438K/S440D according to EU numbering, which result in a significant reduction of the rheumatoid factor binding when combined with an amino acid substitution that can increase the FcRn binding under an acidic condition.

In the embodiment, the constant region preferably comprises a combination of amino acid substitutions selected from the group consisting of:
(I) (a) N434A/Q438R/S440E; (b) N434A/Q438R/S440D; (c) N434A/Q438K/S440E; (d) N434A/Q438K/S440D; (e) N434A/Y436T/Q438R/S440E; (f) N434A/Y436T/Q438R/S440D; (g) N434A/Y436T/Q438K/S440E; (h) N434A/Y436T/Q438K/S440D; (i) N434A/Y436V/Q438R/S440E; (j) N434A/Y436V/Q438R/S440D; (k) N434A/Y436V/Q438K/S440E; (l) N434A/Y436V/Q438K/S440D; (m) N434A/R435H/F436T/Q438R/ S440E; (n) N434A/R435H/F436T/Q438R/S440D; (o) N434A/R435H/F436T/Q438K/S440E; (p) N434A/R435H/F436T/Q438K/S440D; (q) N434A/R435H/F436V/Q438R/S440E; (r) N434A/R435H/F436V/Q438R/S440D; (s) N434A/R435H/F436V/Q438K/S440E; (t) N434A/R435H/F436V/Q438K/S440D; (u) M428L/N434A/Q438R/S440E; (v) M428L/N434A/Q438R/S440D; (w) M428L/N434A/Q438K/S440E; (x) M428L/N434A/Q438K/S440D; (y) M428L/N434A/Y436T/Q438R/S440E; (z) M428L/N434A/Y436T/Q438R/S440D; (aa) M428L/N434A/Y436T/Q438K/S440E; (ab) M428L/N434A/Y436T/Q438K/S440D; (ac) M428L/N434A/Y436V/Q438R/S440E; (ad) M428L/N434A/Y436V/Q438R/S440D; (ae) M428L/N434A/Y436V/Q438K/S440E; (af) M428L/N434A/Y436V/Q438K/S440D; (ag) L23 5R/G236R/S239K/M428L/N434A/Y436T/Q438R/S440E; (ah) L235R/G236R/A327G/A33OS/P331S/M428L/N434A/Y436T/Q438R/S440E, according to EU numbering; or
(II) (a) N434A/Q438R/S440E; (b) N434A/Y436T/Q438R/S440E; (c) N434A/Y436V/Q438R/S440E; (d) M428L/N434A/Q438R/S440E; (e) M428L/N434A/Y436T/Q438R/S440E; (f) M428L/N434A/Y436V/Q438R/S440E; (g) L235R/G236R/S239K/M428L/N434A/Y436T/Q438R/S440E; and (h) L235R/G236R/A327G/A330S/P331S/M428L/N434A/Y436T/Q438R/S440E, according to EU numbering.

In another embodiment, the constant region preferably comprises at least one amino acid selected from a group consisting of leucine at position 428, alanine at position 434 and threonine at position 436 (all numbers are according to EU numbering system). In the embodiment, the constant region more preferably comprises leucine at position 428, alanine at position 434 and threonine at position 436 (all numbers are according to EU numbering system).

### Fc region variants

### (Sweeping technology)

In certain embodiments, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions. In some embodiments, the Fc region is an Fc region of human IgG1.

To enhance the reduction of plasma antigen concentration and/or improve pharmacokinetics of antibodies, amino acid residues at the site for binding to FcRn in the Fc region of IgG can be modified to enhance their uptake into cells. When an antibody with pH dependency is modified in this way, the mutant will be a "sweeping" antibody that can more strongly bind to FcRn and allow the antigen to be efficiently transferred into the endosome (where pH is acidic) and then degraded, but can itself be more efficiently recycled to the cell surface. Such a modified, "sweeping" antibody can strongly bind to FcRn at neutral pH and on the cell surface and enhance the uptake and degradation of the antigen, compared to the original (parent) antibody without the modification. (Semin Immunopathol. 2018; 40(1): 125-140).

In some aspects, the antibody comprises an Fc region that has at least one amino acid modification in the Fc region so as to enhance the reduction of plasma antigen concentration and/or improve pharmacokinetics of the antibody.

A region with decreased Fcγ receptor-binding activity is particularly preferred as the antigen constant region of the present invention. For example, the antibody of the present invention has a mutant constant region comprising at least one amino acid alteration that decreases Fcy receptor-binding activity. Here, an Fcy receptor (herein, also denoted as Fcy receptor, FcyR, or FcgR) refers to a receptor that can bind to the Fc region of IgG1, IgG2, IgG3, or IgG4, and includes all members belonging to the family of proteins substantially encoded by Fcy receptor genes. In humans, this family includes, but is not limited to, FcyRI (CD64) including isoforms FcyRIa, FcyRIb, and FcyRIc; FcyRII (CD32) including isoforms FcyRIIa (including allotypes H131 (type H) and R131 (type R), FcyRIIb (including FcyRIIb-1 and FcyRIIb-2), and FcyRIIc; and FcyRIII (CD16) including isoforms FcyRIIIa (including allotypes V158 and F158) and FcyRIIIb (including allotypes FcyRIIIb-NA1 and FcyRIIIb-NA2); as well as any undiscovered human FcyRs, and FcyR isoforms or allotypes. FcyRs include, but are not limited to, those derived from humans, mice, rats, rabbits, and monkeys, and may be derived from any organism. Mouse FcyRs include, but are not limited to, FcyRI (CD64), FcyRII (CD32), FcyRIII (CD16), and FcyRIII-2 (CD16-2), as well as any undiscovered mouse FcyRs, and FcyR isoforms or allotypes. Suitable examples of such Fcy receptors include human FcyRI (CD64), FcyRIIa (CD32), FcyRIIb (CD32), FcyRIIIa (CD16) and/or FcyRIIIb (CD16).

Activating receptors which carry an immunoreceptor tyrosine-based activation motif (ITAM) and inhibitory receptors which carry an immunoreceptor tyrosine-based inhibitory motif (ITIM) are present among FcyRs. FcyRs are categorized into activating FcyRs: FcyRI, FcyRIIa R, FcyRIIa H, FcyRIIIa, and FcyRIIIb, and inhibitory FcyR: FcyRIIb.

The polynucleotide sequence and amino acid sequence of FcyRI are shown in NM_000566.3 and NP_000557.1, respectively; the polynucleotide sequence and amino acid sequence of FcyRIIa are shown in BC020823.1 and AAH20823.1, respectively; the polynucleotide sequence and amino acid sequence of FcyRIIb are shown in BC146678.1 and AAI46679.1, respectively; the polynucleotide sequence and amino acid sequence of FcyRIIIa are shown in BC033678.1 and AAH33678.1, respectively; and the polynucleotide sequence and amino acid sequence of FcyRIIIb are shown in BC128562.1 and AAI28563.1, respectively (RefSeq accession number). There are two types of gene polymorphisms for FcyRIIa, where the amino acid at position 131 of FcyRIIa is substituted with histidine (type H) or arginine (type R) (J. Exp. Med, 172, 19-25, 1990). Furthermore, there are two types of gene polymorphisms for FcyRIIb, where the amino acid at position 232 of FcyRIIb is substituted with isoleucine (type I) or threonine (type T) (Arthritis. Rheum. 46: 1242-1254 (2002)). In addition, there are two types of gene polymorphisms for FcyRIIIa, where the amino acid at position 158 of FcyRIIIa is substituted with valine (type V) or phenylalanine (type F) (J. Clin. Invest. 100(5): 1059-1070 (1997)). There are also two types of gene polymorphisms for FcyRIIIb, which are type NA1 and type NA2 (J. Clin. Invest. 85: 1287-1295 (1990)).

Whether the binding activity to an Fcy receptor is decreased can be confirmed by well-known methods such as FACS, ELISA format, screening by Amplified Luminescent Proximity Homogeneous Assay (ALPHA), surface plasmon resonance (SPR)-based BIACORE method, and others (Proc. Natl. Acad. Sci. USA (2006) 103(11), 4005-4010).

For example, the binding property of each sample to FcyRs at pH 7.4 is determined at 25 degrees C using BIACORE (registered trademark) T200 instrument (Cytiva). First, protein L (BioVision) is immobilized onto all of flow cells of a CM4 sensor chip using Amine Coupling Kit, type2 (Cytiva). 50 mM phosphate buffer (pH7.4) containing 150 mM NaCl, 0.05% Tween (registered trademark) 20 is used as a running buffer, and antibodies prepared to have a binding response of 500 RU or 2000 RU are captured on the sensor surface. FcyRs diluted with the running buffer (for example, human or monkey FcyRs) are injected thereto and the amount bound with the antibodies is measured. The sensor surface is regenerated every cycle, using 10 mM glycine hydrochloride solution, pH1.5. From obtained measurement results, FcyR binding amount divided by the binding amount of each captured antibody (Binding/capture) is calculated using BIACORE (registered trademark) T200 evaluation software, version 2.0 (Cytiva). That is, since the binding amount of FcγR depends on the amount of the captured antibody, corrected values are calculated by dividing the binding amount of FcγR by the amount of each captured antibody and are compared between antibodies.

In the antibodies of the present invention, the FcyR binding amount divided by the binding amount of each captured antibody (Binding/capture) indicates 1.0, 0.9, 0.8, 0.7, 0.6, 0.5, 0.4, 0.3, 0.2, 0.1, 0.09, 0.08, 0.07, 0.06, 0.05, 0.04, 0.03, 0.02, or 0.01 or less, preferably indicates 0.05, 0.04, 0.03, or 0.02 or less, or particularly preferably indicates 0.01 or less, or cannot be reliably calculated due to no binding activity or considerably low binding activity. Furthermore, the relative Fcγ receptor-binding activity of the antibody of the present invention, which has a mutant constant region comprising at least one amino acid alteration that decreases Fcγ receptor-binding activity, compared to an antibody having a (parent) constant region which does not comprise the amino acid alteration, can be represented by a relative value (relative binding to human FcyRs) that is calculated by dividing the Binding/capture value obtained with the antibody of the present invention which has a mutant constant region comprising at least one amino acid alteration that decreases Fcy receptor-binding activity by the Binding/capture value obtained with an antibody having a (parent) constant region which does not comprise the amino acid alteration (for example, Herceptin). The relative value is 0.06 or less, 0.05 or less, 0.04 or less, 0.03 or less, 0.02 or less, 0.01 or less, or 0.00 or less, or preferably 0.00 or less, or cannot be reliably calculated due to no binding activity or considerably low binding activity.

ALPHA screening is performed with ALPHA technology which uses two beads, a donor and an acceptor bead, based on the following principle. Luminescent signals are detected only when molecules bound to donor beads interact biologically with molecules bound to the acceptor beads, and the two beads are in close proximity to each other. The laser-excited photosensitizer within the donor beads converts ambient oxygen to excited-state singlet oxygen. Singlet oxygen is dispersed around the donor beads; and when it reaches the adjacent acceptor beads, a chemiluminescent reaction is induced within the beads, and light is ultimately emitted. When molecules bound to the donor beads do not interact with molecules bound to the acceptor beads, the chemiluminescent reaction does not take place because singlet oxygen produced by the donor beads does not reach the acceptor beads.

For example, when an antibody contains an antibody Fc region as the FcRn-binding domain, an antibody having a wild-type Fc region and an antibody having a mutant Fc region produced by adding amino acid mutations to change the binding to an Fcy receptor are prepared, a biotinylated antibody is bound to the donor beads, and an Fcy receptor tagged with glutathione S transferase (GST) is bond to the acceptor beads. In the presence of an antibody having a mutant Fc region, the antibody having a wild-type Fc region interacts with the Fcy receptor and produces 520-620 nm signals. When the antibody having a mutant Fc region is untagged, it competes with the antibody having a wild-type Fc region for interaction with the Fcy receptor. The relative binding affinity can be determined by quantifying the decrease in fluorescence observed as a result of the competition. Biotinylation of antibodies using Sulfo-NHS-biotin and such is well known. As a method for tagging an Fcγ receptor with GST, the method of expressing the Fcy receptor and GST in a cell carrying a vector that can express a fusion gene produced by fusing a polynucleotide encoding the Fcy receptor in frame with a GST-encoding polynucleotide, and purifying it using a glutathione column can be appropriately adopted. The obtained signals are suitably analyzed, for example, by fitting them into a one-site competition model that utilizes a non-linear regression analysis with software such as GRAPHPAD PRISM (GraphPad, San Diego).

One of the substances (ligand) observed for interaction is immobilized onto a gold thin film on a sensor chip, and by shining light from the reverse side of the sensor chip so that total reflection takes place at the interface between the gold thin film and glass, a portion with reduced reflection intensity is formed in part of the reflected light (SPR signal). The other substance (analyte) observed for interaction is made to flow over the sensor chip surface; and when the ligand binds to the analyte, the mass of the immobilized ligand molecule increases and the refractive index of the solvent on the sensor chip surface changes. The position of the SPR signal shifts as a result of this change in the refractive index (reversely, the signal position returns if this binding dissociates). The Biacore system shows the amount of shift mentioned above, or more specifically the time variable of mass, by plotting the change in mass on the sensor chip surface on the vertical axis as the measurement data (sensorgram). Kinetic parameters such as association rate constant (ka) and dissociation rate constant (kd) are determined from the curve in the sensorgram, and the affinity (KD) is determined from the ratio of these constants. In the BIACORE method, a method for measuring inhibition is also suitably used. An example of the method for measuring inhibition is described in Proc. Natl. Acad. Sci USA (2006) 103 (11): 4005-4010.

Herein, "decreased Fcγ receptor-binding activity" or "decreases Fcγ receptor-binding activity" means that, for example, based on the above-described analytical method, the Fcy receptor-binding activity of an antibody having a control antibody constant region (for example, parent antibody, that it, the original antibody before alteration of the present invention) is compared to the Fcy receptor-binding activity of the antibody after alteration of the present invention in which one or more amino acid mutations (e.g., additions, insertions deletions, or substitutions) are introduced into the antibody constant region of the original (parent) antibody, and the binding activity of the antibody after alteration of the present invention shows 50% or less, preferably 45% or less, 40% or less, 35% or less, 30% or less, 20% or less, 15% or less, 10% or less, 9% or less, 8% or less, 7% or less, 6% or less, or particularly preferably 5% or less, 4% or less, 3% or less, 2% or less, 1% or less, or 0% as compared to the binding activity of the parent antibody.

For the control antibody (parent antibody), antibodies before alteration having, for example, a domain comprising an Fc region of a monoclonal IgG1, IgG2, IgG3, or IgG4 antibody may be suitably used. Further, when an antibody containing a mutant of an Fc region of a particular antibody isotype is used as the test substance, the effect of a mutation possessed by the mutant on the Fcy receptor-binding activity is tested by using as a control an antibody having an Fc region of an antibody of that particular isotype. In this way, antibodies containing an Fc region mutant whose binding activity toward the Fcy receptor verified to be decreased are suitably produced.

Examples of such mutants include mutants with a 231A-238S deletion (WO 2009/011941), or C226S, C229S, P238S, (C220S) (J. Rheumatol (2007) 34, 11), C226S, C229S (Hum. Antibod. Hybridomas (1990) 1(1), 47-54), C226S, C229S, E233P, L234V, or L235A (Blood (2007) 109, 1185-1192) mutants, where the amino acids are specified by EU numbering.

That is, suitable examples include antibodies having an Fc region in which any of the amino acids at positions 220, 226, 229, 231, 232, 233, 234, 235, 236, 237, 238, 239, 240, 264, 265, 266, 267, 269, 270, 295, 296, 297, 298, 299, 300, 325, 327, 328, 329, 330, 331, and 332 specified according to EU numbering has been substituted in the amino acids constituting the Fc region of an antibody of a specific isotype. Positions 235 and 236 are particularly preferred. The isotype of the antibody from which the Fc region originates is not particularly limited, and the Fc region derived from an IgG1, IgG2, IgG3, or IgG4 monoclonal antibody can be used appropriately, and the Fc region derived from a naturally-occurring human IgG1 antibody is suitably used.

For example, an antibody having an Fc region that comprises any substitution specified below based on EU numbering from among amino acids constituting the IgG1 antibody Fc region (wherein the number indicates the position of the amino acid residue specified according to EU numbering, the one-letter amino acid code positioned before the number indicates the amino acid residue before the substitution, and the one-letter amino acid code positioned after the number indicates the amino acid residue after the substitution):
(a) L234F, L235E, P331S
(b) C226S, C229S, P238S
(c) C226S, C229S
(d) C226S, C229S, E233P, L234V, L235A;
or an Fc region lacking the amino acid sequence of positions 231 to 238 from among amino acids constituting the IgG1 antibody Fc region may be appropriately used.

Furthermore, antibodies having an Fc region that comprises any substitution specified below based on EU numbering from among amino acids constituting the IgG2 antibody Fc region (wherein the number indicates the position of the amino acid residue specified according to EU numbering, the one-letter amino acid code positioned before the number indicates the amino acid residue before the substitution, and the one-letter amino acid code positioned after the number indicates the amino acid residue after the substitution):
(e) H268Q, V309L, A330S, P331S
(f) V234A
(g) G237A
(h) V234A, G237A
(i) A235E, G237A
(j) V234A, A235E, G237A
may be appropriately used.

Furthermore, antibodies having an Fc region that comprises any substitution specified below based on EU numbering from among amino acids constituting the IgG3 antibody Fc region (wherein the number indicates the position of the amino acid residue specified according to EU numbering, the one-letter amino acid code positioned before the number indicates the amino acid residue before the substitution, and the one-letter amino acid code positioned after the number indicates the amino acid residue after the substitution):
(k) F241A
(l) D265A
(m) V264A
may be appropriately used.

Furthermore, antibodies having an Fc region that comprises any substitution specified below based on EU numbering from among amino acids constituting the IgG4 antibody Fc region (wherein the number indicates the position of the amino acid residue specified according to EU numbering, the one-letter amino acid code positioned before the number indicates the amino acid residue before the substitution, and the one-letter amino acid code positioned after the number indicates the amino acid residue after the substitution):
(n) L235A, G237A, E318A
(o) L235E
(p) F234A, L235A
may be appropriately used.

Other preferred examples include antibodies having an Fc region in which any of the amino acids at positions 233, 234, 235, 236, 237, 327, 330, and 331 specified according to EU numbering in the amino acids constituting the Fc region of a naturally-occurring human IgG1 antibody is substituted with amino acids of corresponding EU numbering in the corresponding IgG2 or IgG4.

Other preferred examples suitably include antibodies having an Fc region in which any one or more of the amino acids at positions 235 and 236 specified according to EU numbering in the amino acids constituting the Fc region of a naturally-occurring human IgG1 antibody are substituted by other amino acids. The type of amino acid present after substitution is not particularly limited, and an antibody having an Fc region in which any one or two of the amino acids at positions 235 and 236 are substituted with arginine is particularly preferred.

In certain embodiments, the invention contemplates an antibody variant that possesses some but not all effector functions, which make it a desirable candidate for applications in which the half life of the antibody in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the antibody lacks Fc gamma R binding (hence likely lacking ADCC activity), but retains FcRn binding ability. The primary cells for mediating ADCC, NK cells, express Fc gamma RIII only, whereas monocytes express Fc gamma RI, Fc gamma RII and Fc gamma RIII. FcR expression on hematopoietic cells is summarized in Table 3 on page 464 of Ravetch and Kinet, Annu. Rev. Immunol. 9:457-492 (1991). Non-limiting examples of in vitro assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACT1 (registered trademark) non-radioactive cytotoxicity assay for flow cytometry (CellTechnology, Inc. Mountain View, CA; and CytoTox 96 (registered trademark) non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and in vivo clearance/half life determinations can also be performed using methods known in the art (see, e.g., Petkova, S.B. et al., Int'l. Immunol. 18(12): 1759-1769 (2006)).

In one embodiment, the antibody of the present application is an antibody in which an antibody constant region comprises an H chain constant region comprising the amino acid sequence consisting of SEQ ID NO: 45 and an L chain constant region comprising the amino acid sequence consisting of SEQ ID NO: 23.

In one embodiment, the antibody of the present application is an antibody comprising a heavy chain (H chain) and a light chain (L chain) selected from the group consisting of 1) to 6) below:
1) an H chain and L chain that comprise the amino acid sequences consisting of SEQ ID NOs: 66 and 67, respectively;
2) an H chain and L chain that comprise the amino acid sequences consisting of SEQ ID NOs: 68 and 69, respectively;
3) an H chain and L chain that comprise the amino acid sequences consisting of SEQ ID NOs: 70 and 71, respectively;
4) an H chain and L chain that comprise the amino acid sequences consisting of SEQ ID NOs: 72 and 73, respectively;
5) an H chain and L chain that comprise the amino acid sequences consisting of SEQ ID NOs: 74 and 75, respectively; and
6) an H chain and L chain that comprise the amino acid sequences consisting of SEQ ID NOs: 76 and 77, respectively.

### (a7) Other embodiments

### (Antibody Variants)

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a7-1) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table A under the heading of "preferred substitutions." More substantial changes are provided in Table A under the heading of "exemplary substitutions," and as further described below in reference to amino acid side chain classes. Amino acid substitutions may be introduced into an antibody of interest and the products may be screened for a desired activity, e.g., retained/improved antigen binding, decreased immunogenicity, or improved ADCC or CDC.

**(TABLE A)**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; lie | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, methionine (Met), alanine (Ala), valine (Val), leucine (Leu), isoleucine (Ile);
(2) neutral hydrophilic: cysteine (Cys), serine (Ser), threonine (Thr), asparagine (Asn), Glutamine (Gln);
(3) acidic: aspartic acid (Asp), glutamic acid (Glu);
(4) basic: histidine (His), lysine (Lys), arginine (Arg);
(5) residues that influence chain orientation: glycine (Gly), proline (Pro);
(6) aromatic: tryptophan (Trp), tyrosine (Tyr), phenylalanine (Phe).

Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies are displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex may be analyzed to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion of an enzyme (e.g. for ADEPT) or a polypeptide which increases the plasma half-life of the antibody to the N- or C-terminus of the antibody.

### a7-2) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about +/- 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, FUT8, knockout CHO cells (see, e.g., Yamane-Ohnuki et al. Biotech. Bioeng. 87: 614 (2004); Kanda, Y et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### a7-3) Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### a7-4) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, polypropylene glycol homopolymers, polypropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### B. Recombinant Methods and Compositions

Antibodies may be produced using recombinant methods and compositions, e.g., as described in U.S. Patent No. 4,816,567. In one embodiment, isolated nucleic acid encoding an anti-C1s antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp2/0 cell). In one embodiment, a method of making an anti-C1s antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-C1s antibody, a nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. See, e.g., US Patent Nos. 5,959,177, 6,040,498, 6,420,548, 7,125,978, and 6,417,429 (describing PLANTIBODIES (registered trademark) technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

Antibodies with pH-dependent characteristics may be obtained by using screening methods and/or mutagenesis methods e.g., as described in WO 2009/125825. The screening methods may comprise any process by which an antibody having pH-dependent binding characteristics is identified within a population of antibodies specific for a particular antigen. In certain embodiments, the screening methods may comprise measuring one or more binding parameters (e.g., KD or kd) of individual antibodies within an initial population of antibodies both at acidic pH and neutral pH. The binding parameters of the antibodies may be measured using, e.g., surface plasmon resonance, or any other analytic method that allows for the quantitative or qualitative assessment of the binding characteristics of an antibody to a particular antigen. In certain embodiments, the screening methods may comprise identifying an antibody that binds to an antigen with an acidic KD /neutral KD ratio of 2 or greater. Alternatively, the screening methods may comprise identifying an antibody that binds to an antigen with an acidic kd/neutral kd ratio of 2 or greater.

In another embodiment, the mutagenesis methods may comprise incorporating a deletion, substitution, or addition of an amino acid within the heavy and/or light chain of the antibody to enhance the pH-dependent binding of the antibody to an antigen. In certain embodiments, the mutagenesis may be carried out within one or more variable domains of the antibody, e.g., within one or more HVRs (e.g., CDRs). For example, the mutagenesis may comprise substituting an amino acid within one or more HVRs (e.g., CDRs) of the antibody with another amino acid. In certain embodiments, the mutagenesis may comprise substituting one or more amino acids in at least one HVR (e.g., CDR) of the antibody with histidine. In certain embodiments, "enhanced pH-dependent binding" means that the mutated version of the antibody exhibits a greater acidic KD/neutral KD ratio, or a greater acidic kd/neutral kd ratio, than the original "parent" (i.e., the less pH-dependent) version of the antibody prior to mutagenesis. In certain embodiments, the mutated version of the antibody has an acidic KD/neutral KD ratio of 2 or greater. Alternatively, the mutated version of the antibody has an acidic kd/neutral kd ratio of 2 or greater.

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals (usually non-human mammals) are immunized against the antigen, immunogenic conjugates, or derivatives by combining, e.g., 100 micro g or 5 micro g of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Monoclonal antibodies are obtained from a population of substantially homogeneous antibodies, i.e., the individual antibodies comprising the population are identical except for possible naturally occurring mutations and/or post-translational modifications (e.g., isomerizations, amidations) that may be present in minor amounts. Thus, the modifier "monoclonal" indicates the character of the antibody as not being a mixture of discrete antibodies.

For example, the monoclonal antibodies may be made using the hybridoma method first described by Kohler et al., Nature 256(5517):495-497 (1975). In the hybridoma method, a mouse or other appropriate host animal, such as a hamster, is immunized as hereinabove described to elicit lymphocytes that produce or are capable of producing antibodies that will specifically bind to the protein used for immunization. Alternatively, lymphocytes may be immunized *in vitro.*

The immunizing agent will typically include the antigenic protein or a fusion variant thereof. Generally either peripheral blood lymphocytes (PBLs) are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press (1986), pp. 59-103).

Immortalized cell lines are usually transformed mammalian cells, particularly myeloma cells of rodent, bovine and human origin. Usually, rat or mouse myeloma cell lines are employed. The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which are substances that prevent the growth of HGPRT-deficient cells.

Preferred immortalized myeloma cells are those that fuse efficiently, support stable high-level production of antibody by the selected antibody-producing cells, and are sensitive to a medium such as HAT medium. Among these, preferred are murine myeloma lines, such as those derived from MOPC-21 and MPC-11 mouse tumors available from the Salk Institute Cell Distribution Center, San Diego, California USA, and SP-2 cells (and derivatives thereof, e.g., X63-Ag8-653) available from the American Type Culture Collection, Manassas, Virginia USA. Human myeloma and mouse-human heteromyeloma cell lines also have been described for the production of human monoclonal antibodies (Kozbor et al. J. Immunol. 133(6):3001-3005 (1984); Brodeur et al., Monoclonal Antibody Production Techniques and Applications, Marcel Dekker, Inc., New York, pp. 51-63 (1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunosorbent assay (ELISA). Such techniques and assays are known in the art. For example, binding affinity may be determined by the Scatchard analysis of Munson, Anal. Biochem. 107(1):220-239 (1980).

After hybridoma cells are identified that produce antibodies of the desired specificity, affinity, and/or activity, the clones may be subcloned by limiting dilution procedures and grown by standard methods (Goding, supra). Suitable culture media for this purpose include, for example, D-MEM or RPMI-1640 medium. In addition, the hybridoma cells may be grown *in vivo* as tumors in a mammal.

The monoclonal antibodies secreted by the subclones are suitably separated from the culture medium, ascites fluid, or serum by conventional immunoglobulin purification procedures such as, for example, protein A-Sepharose, hydroxyapatite chromatography, gel electrophoresis, dialysis, or affinity chromatography.

### III. Assays

Anti-C1s antibodies provided herein may be identified, screened for, or characterized for their physical/chemical properties and/or biological activities by various assays known in the art.

### A. Binding assays and other assays

In one aspect, an antibody of the invention is tested for its antigen binding activity, e.g., by known methods such as ELISA, Western blot, etc.

In another aspect, competition assays may be used to identify an antibody that competes for binding to C1s with any anti-C1s antibody described herein, or identify an antibody that binds to the same epitope as any anti-C1s antibody described herein. In certain embodiments, when such a competing antibody is present in excess, it blocks (e.g., reduces) the binding of a reference antibody to C1s by at least 10%, 15%, 20%, 25%, 30%, 35%, 40%, 45%, 50%, 55%, 60%, 65%, 70%, 75% or more. In certain embodiments, such a competing antibody binds to the same epitope (e.g., a linear or a conformational epitope) that is bound by any anti-C1s antibody described herein. Detailed exemplary methods for mapping an epitope to which an antibody binds are provided in Morris (1996) "Epitope Mapping Protocols," in Methods in Molecular Biology vol. 66 (Humana Press, Totowa, NJ). In certain embodiments, such a competition assays can be conducted at neutral pH condition. In some embodiments, the competition assay is tandem competition assay using, for example, Octet (registered trademark) systems.

In an exemplary competition assay, immobilized C1s is incubated in a solution comprising a first labeled antibody that binds to C1s (e.g., one of those described herein) and a second unlabeled antibody that is being tested for its ability to compete with the first antibody for binding to C1s. The second antibody may be present in a hybridoma supernatant. As a control, immobilized C1s is incubated in a solution comprising the first labeled antibody but not the second unlabeled antibody. After incubation under conditions permissive for binding of the first antibody to C1s, excess unbound antibody is removed, and the amount of label associated with immobilized C1s is measured. If the amount of label associated with immobilized C1s is substantially reduced in the test sample relative to the control sample, then that indicates that the second antibody is competing with the first antibody for binding to C1s. See Harlow and Lane (1988) Antibodies: A Laboratory Manual ch.14 (Cold Spring Harbor Laboratory, Cold Spring Harbor, NY).

In another aspect, an antibody that binds to the same epitope as an anti-C1s antibody provided herein or that competes for binding to C1s with an anti-C1s antibody provided herein may be identified using sandwich assays. Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three part complex. See David & Greene, U.S. Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme. An antibody which simultaneously binds to C1s with an anti-C1s antibody provided herein can be determined to be an antibody that binds to a different epitope from the anti-C1s antibody. Therefore, an antibody which does not simultaneously bind to C1s with an anti-C1s antibody provided herein can be determined to be an antibody that binds to the same epitope as the anti-C1s antibody or that competes for binding to C1s with the anti-C1s antibody.

### B. Activity assays

In one aspect, assays are provided for identifying anti-C1s antibodies thereof having biological activity. Biological activity may include blocking the activation of the classical pathway and generation of cleavage products resulting from the activation of the said pathway, C2a, C2b, C3a, C3b, C4a, C4b, C5a and C5b. Antibodies having such biological activity *in vivo* and/or *in vitro* are also provided.

In certain embodiments, an antibody of the invention is tested for such biological activity. In some embodiments, the antibody of the invention can be evaluated for its ability to inhibit complement-mediated hemolysis of sheep red blood cells (RBC) that have been sensitized by antibodies directed against sheep RBC antigens, i.e., using an RBC assay. In some embodiments, the antibody of the invention can be evaluated for its ability to inhibit complement-mediated hemolysis of chicken red blood cells (cRBC) that have been sensitized by antibodies directed against cRBC antigens. Using human serum as a source of complement proteins, the activity of the antibody of the invention can be determined by measuring the amount of haemoglobin released by a spectrophotometric method.

RBC assay can be suitably performed using known methods such as the method disclosed in J. Vis. Exp. 2010; (37): 1923. This article describes how to conduct the 50% Haemolytic Complement (CH50) assay as the RBC lysis assay. Briefly, this assay measures the activation of the classical complement pathway, and detects the reduction, absence, or inactivity of any component of the pathway. It assesses the activity of complement components in the serum to lyse red blood cells. When an antibody is incubated with test serum, the pathway is activated and causes haemolysis. If one or more components of the classical pathway are decreased, the CH50 value is decreased. The CH50 assay is not exactly the same as the assay used in the Examples herein which rather measures % inhibition against cell lysis by complement components; however, the concept and basic set up is substantially the same as the present invention. In an embodiment, the RBC assay is performed as follows. Human serum is pre-incubated with the antibody of interest (e.g., for 3 hours at 37 degrees Celsius (degrees C)). The serum is then added to an equal volume of sensitized sheep red blood cells and incubated (e.g., for one hour at 37 degrees C) to allow for lysis of the red blood cells. The reaction is then stopped. The mixture is centrifuged to pellet unlysed cells, and the supernatant is withdrawn, and absorbance (OD) at 415nm is used to analyze the release of hemoglobin. To calculate the percentage inhibition (%) of red blood cell lysis, 0% inhibition is set as the condition where no antibody (buffer only) is added, and 100% inhibition is set as the condition where EDTA is added at a final concentration of 5mM (see, e.g., Example 7). When the antibody shows a percentage inhibition (%) of red blood cell lysis, this means that the antibody has a neutralizing activity for human serum complement, e.g., an activity to inhibit the interaction between C1q and C1r2s2 complex.

Thus, RBC assay can be used to evaluate a neutralizing activity for human serum complement of an antibody, in order to assess the activity to inhibit the interaction between C1q and C1r2s2 complex. In an embodiment, the present invention provides an isolated antibody that inhibits the interaction between C1q and C1r2s2 complex, where the antibody has a neutralizing activity for human serum complement of at least 70% in RBC assay.

### C. Assessment of immunogenic potential

The immunogenic potential of the antibodies are evaluated by using as an indicator the proportion of IL-2-secreting CD4⁺ T cells before active proliferation is exhibited as described in WO2018/124005 (Kubo C. et al). Specifically, CD8⁻CD25^{low}PBMCs (peripheral blood mononuclear cells) are prepared from human PBMCs, and the cells are cultured for 67 hours in the presence of the antibodies.

### IV Immunoconjugates

The invention also provides immunoconjugates comprising an anti-C1s antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, saponaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include ²¹¹At, ¹³¹I, ¹²⁵I, ⁹⁰Y, ¹⁸⁶Re, ¹⁸⁸Re, ¹⁵³Sm, ²¹²Bi, ³²P, ²¹²Pb and radioactive isotopes of Lu. When the radioconjugate is used for detection, it may comprise a radioactive atom for scintigraphic studies, for example Tc-99m or ¹²³I, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionuclide to the antibody. See WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### V. Methods and Compositions for Diagnostics and Detection

In certain embodiments, any of the anti-C1s antibodies provided herein is useful for detecting the presence of C1s in a biological sample. The term "detecting" as used herein encompasses quantitative or qualitative detection. In certain embodiments, a biological sample comprises a cell or tissue, such as serum, whole blood, plasma, biopsy sample, tissue sample, cell suspension, saliva, sputum, oral fluid, cerebrospinal fluid, amniotic fluid, ascites fluid, milk, colostrum, mammary gland secretion, lymph, urine, sweat, lacrimal fluid, gastric fluid, synovial fluid, peritoneal fluid, ocular lens fluid or mucus.

In one embodiment, an anti-C1s antibody for use in a method of diagnosis or detection is provided. In a further aspect, a method of detecting the presence of C1s in a biological sample is provided. In certain embodiments, the method comprises contacting the biological sample with an anti-C1s antibody as described herein under conditions permissive for binding of the anti-C1s antibody to C1s, and detecting whether a complex is formed between the anti-C1s antibody and C1s. Such method may be an in vitro or in vivo method. In one embodiment, an anti-C1s antibody is used to select subjects eligible for therapy with an anti-C1s antibody, e.g. where C1s is a biomarker for selection of patients.

Exemplary disorders that may be diagnosed using an antibody of the invention include, but are not limited to, age-related macular degeneration, Alzheimer's disease, amyotrophic lateral sclerosis, anaphylaxis, argyrophilic grain dementia, arthritis (e.g., rheumatoid arthritis), asthma, atherosclerosis, atypical hemolytic uremic syndrome, autoimmune diseases, Barraquer-Simons syndrome, Behcet's disease, British type amyloid angiopathy, bullous pemphigoid, Buerger's disease, C1q nephropathy, cancer, catastrophic antiphospholipid syndrome, cerebral amyloid angiopathy, cold agglutinin disease, corticobasal degeneration, Creutzfeldt-Jakob disease, Crohn's disease, cryoglobulinemic vasculitis, dementia pugilistica, dementia with Lewy Bodies (DLB), diffuse neurofibrillary tangles with calcification, Discoid lupus erythematosus, Down's syndrome, focal segmental glomerulosclerosis, formal thought disorder, frontotemporal dementia (FTD), frontotemporal dementia with parkinsonism linked to chromosome 17, frontotemporal lobar degeneration, Gerstmann-Straussler-Scheinker disease, Guillain-Barre syndrome, Hallervorden-Spatz disease, hemolytic -uremic syndrome, hereditary angioedema, hypophosphastasis, idiopathic pneumonia syndrome, immune complex diseases, inclusion body myositis, infectious disease (e.g., disease caused by bacterial (e.g., Neisseria meningitidis or Streptococcus) viral (e.g., human immunodeficiency virus (HIV)), or other infectious agents), inflammatory disease, ischemia / reperfusion injury, mild cognitive impairment, immunothrombocytopenic purpura (ITP), molybdenum cofactor deficiency (MoCD) type A, membranoproliferative glomerulonephritis (MPGN) I, membranoproliferative glomerulonephritis (MPGN) II (dense deposit disease), membranous nephritis, multi-infarct dementia, lupus (e.g., systemic lupus erythematosus (SLE)), glomerulonephritis, Kawasaki disease, multifocal motor neuropathy, multiple sclerosis, multiple system atrophy, myasthenia gravis, myocardial infarction, myotonic dystrophy, neuromyelitis optica, Niemann-Pick disease type C, non-Guamanian motor neuron disease with neurofibrillary tangles, Parkinson's disease, Parkinson's disease with dementia, paroxysmal nocturnal hemoglobinuria, Pemphigus vulgaris, Pick's disease, postencephalitic parkinsonism, polymyositis, prion protein cerebral amyloid angiopathy, progressive subcortical gliosis, progressive supranuclear palsy, psoriasis, sepsis, Shiga-toxin E coli (STEC)-HuS, spinal muscular atrophy, stroke, subacute sclerosing panencephalitis, Tangle only dementia, transplant rejection, vasculitis (e.g., ANCA associated vasculitis), Wegner's granulomatosis, sickle cell disease, cryoglobulinemia, mixed cryoglobulinemia, essential mixed cryoglobulinemia, Type II mixed cryoglobulinemia, Type III mixed cryoglobulinemia, nephritis, drug-induced thrombocytopenia, lupus nephritis, bullous pemphigoid, Epidermolysis bullosa acquisita, delayed hemolytic transfusion reaction, hypocomplementemic urticarial vasculitis syndrome, pseudophakic bullous keratopathy, and platelet refractoriness.

In certain embodiments, labeled anti-C1s antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, beta-galactosidase, glucoamylase, lysozyme, saccharide oxidases, e.g., glucose oxidase, galactose oxidase, and glucose-6-phosphate dehydrogenase, those coupled with an enzyme that employs hydrogen peroxide to oxidize a dye precursor such as HRP, lactoperoxidase, or microperoxidase, heterocyclic oxidases such as uricase and xanthine oxidase, biotin/avidin, spin labels, bacteriophage labels, stable free radicals, and the like.

### VI. Pharmaceutical Formulations

Pharmaceutical formulations of an anti-C1s antibody as described herein are prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX (registered trademark), Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide the formulation which is used for combination therapy, Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for in vivo administration are generally sterile. Sterility may be readily accomplished, e.g., by filtration through sterile filtration membranes.

### VII. Therapeutic Methods and Compositions

Any of the anti-C1s antibodies provided herein may be used in therapeutic methods.

In one aspect, an anti-C1s antibody for use as a medicament is provided. In further aspects, an anti-C1s antibody for use in treating a complement-mediated disease or disorder is provided. In certain embodiments, an anti-C1s antibody for use in a method of treatment is provided. In certain embodiments, the invention provides an anti-C1s antibody for use in a method of treating an individual having a complement-mediated disease or disorder comprising administering to the individual an effective amount of the anti-C1s antibody. In one such embodiment, the method further comprises administering to the individual an effective amount of at least one additional therapeutic agent. An "individual" according to any of the above embodiments is preferably a human.

In further embodiments, the invention provides an anti-C1s antibody for use in treating a complement-mediated disease or disorder. In further embodiments, anti-C1s antibodies may be for use in enhancing the clearance of C1s from plasma. In further embodiments, anti-C1s antibodies may be for use in enhancing the clearance of C1r2s2 from plasma. In further embodiments, anti-C1s antibodies may be for use in enhancing the clearance of C1r2s2 from plasma not but C1q from plasma. In some cases, the antibody inhibits a component of the classical complement pathway; in some cases, the classical complement pathway component is C1s. In certain embodiments, the invention provides an anti-C1s antibody for use in a method of treating a complement-mediated disease or disorder. In certain embodiments, the invention provides an anti-C1s antibody for use in a method of enhancing the clearance of C1s from plasma. In certain embodiments, the invention provides an anti-C1s antibody for use in a method of enhancing the clearance of C1r2s2 from plasma. In certain embodiments, the invention provides an anti-C1s antibody for use in a method of enhancing the clearance of C1r2s2 from plasma not but C1q from plasma. In certain embodiments, the invention provides an anti-C1s antibody for use in a method of inhibiting a component of the classical complement pathway; in some cases, the classical complement pathway component is C1s. An "individual" according to any of the above embodiments is preferably a human.

In one aspect, the present disclosure provides a method of modulating complement activation. In some embodiments the method inhibits complement activation, for example to reduce production of C4b2a. In some embodiments, the present disclosure provides a method of modulating complement activation in an individual having a complement-mediated disease or disorder, the method comprising administering to the individual an anti-C1s antibody of the present disclosure or a pharmaceutical composition of the present disclosure, wherein the pharmaceutical composition comprises an anti-C1s antibody of the present disclosure. In some embodiments such a method inhibits complement activation. In some embodiments, the individual is a mammal. In some embodiments, the individual is a human. Administration can be by any route known to those skilled in the art, including those disclosed herein. In some embodiments, administration is intravenous or subcutaneous. In some embodiments, administration is intrathecal.

A complement-mediated disease or disorder is a disorder characterized by an abnormal amount of complement C1s or an abnormal level of complement C1s proteolytic activity in a cell, a tissue, or a fluid of an individual.

In some cases, a complement-mediated disease or disorder is characterized by the presence in a cell, a tissue, or a fluid of an elevated (higher than normal) amount of C1s or of an elevated level of complement C1s activity. For example, in some cases, a complement-mediated disease or disorder is characterized by the presence in brain tissue and/or cerebrospinal fluid of an elevated amount and/or an elevated activity of C1s. A "higher than normal" amount of C1s in a cell, a tissue, or a fluid indicates that the amount of C1s in the cell, tissue or fluid is higher than a normal, control level, e.g., higher than a normal, control level for an individual or population of individuals of the same age group. A "higher than normal" level of C1s activity in a cell, a tissue, or a fluid indicates that the proteolytic cleavage effected by C1s in the cell, tissue or fluid is higher than a normal, control level, e.g., higher than a normal, control level for an individual or population of individuals of the same age group. In some cases, an individual having a complement-mediated disease or disorder exhibits one or more additional symptoms of such a disease or disorder.

In other cases, a complement-mediated disease or disorder is characterized by the presence in a cell, a tissue, or a fluid of a lower than normal amount of C1s or of a lower level of complement C1s activity. For example, in some cases, a complement-mediated disease or disorder is characterized by the presence in brain tissue and/or cerebrospinal fluid of a lower amount and/or a lower activity of C1s. A "lower than normal" amount of C1s in a cell, a tissue, or a fluid indicates that the amount of C1s in the cell, tissue or fluid is lower than a normal, control level, e.g., lower than a normal, control level for an individual or population of individuals of the same age group. A "lower than normal" level of C1s activity in a cell, a tissue, or a fluid indicates that the proteolytic cleavage effected by C1s in the cell, tissue or fluid is lower than a normal, control level, e.g., lower than a normal, control level for an individual or population of individuals of the same age group. In some cases, an individual having a complement-mediated disease or disorder exhibits one or more additional symptoms of such a disease or disorder.

A complement-mediated disease or disorder is a disease or disorder in which the amount or activity of complement C1s is such that it causes a disease or disorder in an individual. In some embodiments, the complement-mediated disease or disorder is selected from the group consisting of autoimmune disease, cancer, hematological disease, infectious disease, inflammatory disease, ischemia-reperfusion injury, neurodegenerative disease, neurodegenerative disorder, ocular disease, renal disease, transplant rejection, vascular disease, and vasculitis disease. In some embodiments, the complement-mediated disease or disorder is an autoimmune disease. In some embodiments, the complement-mediated disease or disorder is cancer. In some embodiments, the complement-mediated disease or disorder is an infectious disease. In some embodiments, the complement-mediated disease or disorder is an inflammatory disease. In some embodiments, the complement-mediated disease or disorder is a hematological disease. In some embodiments, the complement-mediated disease or disorder is an ischemia-reperfusion injury. In some embodiments, the complement-mediated disease or disorder is an ocular disease. In some embodiments, the complement-mediated disease or disorder is a renal disease. In some embodiments, the complement-mediated disease or disorder is transplant rejection. In some embodiments, the complement-mediated disease or disorder is antibody-mediated transplant rejection. In some embodiments, the complement-mediated disease or disorder is a vascular disease. In some embodiments, the complement-mediated disease or disorder is a vasculitis disorder. In some embodiments, the complement-mediated disease or disorder is a neurodegenerative disease or disorder. In some embodiments, the complement-mediated disease is a neurodegenerative disease. In some embodiments, the complement-mediated disorder is a neurodegenerative disorder. In some embodiments, the complement-mediated disease or disorder is a tauopathy.

Examples of a complement-mediated disease or disorder include, but are not limited to, age-related macular degeneration, Alzheimer's disease, amyotrophic lateral sclerosis, anaphylaxis, argyrophilic grain dementia, arthritis (e.g., rheumatoid arthritis), asthma, atherosclerosis, atypical hemolytic uremic syndrome, autoimmune diseases, Barraquer-Simons syndrome, Behcet's disease, British type amyloid angiopathy, bullous pemphigoid, Buerger's disease, C1q nephropathy, cancer, catastrophic antiphospholipid syndrome, cerebral amyloid angiopathy, cold agglutinin disease, corticobasal degeneration, Creutzfeldt-Jakob disease, Crohn's disease, cryoglobulinemic vasculitis, dementia pugilistica, dementia with Lewy Bodies (DLB), diffuse neurofibrillary tangles with calcification, Discoid lupus erythematosus, Down's syndrome, focal segmental glomerulosclerosis, formal thought disorder, frontotemporal dementia (FTD), frontotemporal dementia with parkinsonism linked to chromosome 17, frontotemporal lobar degeneration, Gerstmann-Straussler-Scheinker disease, Guillain-Barre syndrome, Hallervorden-Spatz disease, hemolytic-uremic syndrome, hereditary angioedema, hypophosphastasis, idiopathic pneumonia syndrome, immune complex diseases, inclusion body myositis, infectious disease (e.g., disease caused by bacterial (e.g., Neisseria meningitidis or Streptococcus) viral (e.g., human immunodeficiency virus (HIV)), or other infectious agents), inflammatory disease, ischemia / reperfusion injury, mild cognitive impairment, immunothrombocytopenic purpura (ITP), molybdenum cofactor deficiency (MoCD) type A, membranoproliferative glomerulonephritis (MPGN) I, membranoproliferative glomerulonephritis (MPGN) II (dense deposit disease), membranous nephritis, multi-infarct dementia, lupus (e.g., systemic lupus erythematosus (SLE)), glomerulonephritis, Kawasaki disease, multifocal motor neuropathy, multiple sclerosis, multiple system atrophy, myasthenia gravis, myocardial infarction, myotonic dystrophy, neuromyelitis optica, Niemann-Pick disease type C, non-Guamanian motor neuron disease with neurofibrillary tangles, Parkinson's disease, Parkinson's disease with dementia, paroxysmal nocturnal hemoglobinuria, Pemphigus vulgaris, Pick's disease, postencephalitic parkinsonism, polymyositis, prion protein cerebral amyloid angiopathy, progressive subcortical gliosis, progressive supranuclear palsy, psoriasis, sepsis, Shiga-toxin E coli (STEC)-HuS, spinal muscular atrophy, stroke, subacute sclerosing panencephalitis, Tangle only dementia, transplant rejection, vasculitis (e.g., ANCA associated vasculitis), Wegner's granulomatosis, sickle cell disease, cryoglobulinemia, mixed cryoglobulinemia, essential mixed cryoglobulinemia, Type II mixed cryoglobulinemia, Type III mixed cryoglobulinemia, nephritis, drug-induced thrombocytopenia, lupus nephritis, bullous pemphigoid, Epidermolysis bullosa acquisita, delayed hemolytic transfusion reaction, hypocomplementemic urticarial vasculitis syndrome, pseudophakic bullous keratopathy, and platelet refractoriness.

Alzheimer's disease and certain forms of Frontotemporal dementia (Pick's disease, sporadic Frontotemporal dementia and Frontotemporal dementia with Parkinsonism linked to chromosome 17) are the most common forms of tauopathy. In accordance with this, the present invention relates to any method as described above, wherein the tauopathy is Alzheimer's, Pick's disease, sporadic Frontotemporal dementia and Frontotemporal dementia with Parkinsonism linked to chromosome 17. Other tauopathies include, but are not limited to, Progressive supranuclear palsy (PSP), Corticobasal degeneration (CBD) and Subacute sclerosing panencephalitis.

A neurodegenerative tauopathy includes Alzheimer's disease, amyotrophic lateral sclerosis/parkinsonism-dementia complex, argyrophilic grain dementia, British type amyloid angiopathy, cerebral amyloid angiopathy, corticobasal degeneration, Creutzfeldt-Jakob disease, dementia pugilistica, diffuse neurofibrillary tangles with calcification, Down's syndrome, frontotemporal dementia, frontotemporal dementia with parkinsonism linked to chromosome 17, frontotemporal lobar degeneration, Gerstmann-Straussler-Scheinker disease, Hallervorden-Spatz disease, inclusion body myositis, multiple system atrophy, myotonic dystrophy, Niemann-Pick disease type C, non-Guamanian motor neuron disease with neurofibrillary tangles, Pick's disease, postencephalitic parkinsonism, prion protein cerebral amyloid angiopathy, progressive subcortical gliosis, progressive supranuclear palsy, subacute sclerosing panencephalitis, Tangle only dementia, multi-infarct dementia, ischemic stroke, chronic traumatic encephalopathy (CTE), traumatic brain injury (TBI), and stroke.

The present disclosure also provides methods of treating a synucleinopathy, e.g., Parkinson's disease (PD); dementia with Lewy Bodies (DLB); multiple system atrophy (MSA); etc. For example, PD with dementia (PDD) can be treated with a method of the present disclosure.

In some embodiments, the complement-mediated disease or disorder comprises Alzheimer's disease. In some embodiments, the complement-mediated disease or disorder comprises Parkinson's disease. In some embodiments, the complement-mediated disease or disorder comprises transplant rejection. In some embodiments, the complement-mediated disease or disorder is antibody-mediated transplant rejection.

In some embodiments, an anti-C1s antibody of the present disclosure prevents or delays the onset of at least one symptom of a complement-mediated disease or disorder in an individual. In some embodiment, an anti-C1s antibody of the present disclosure reduces or eliminates at least one symptom of a complement-mediated disease or disorder in an individual. Examples of symptoms include, but are not limited to, symptoms associated with autoimmune disease, cancer, hematological disease, infectious disease, inflammatory disease, ischemia-reperfusion injury, neurodegenerative disease, neurodegenerative disorder, renal disease, transplant rejection, ocular disease, vascular disease, or a vasculitis disorder. The symptom can be a neurological symptom, for example, impaired cognitive function, memory impairment, loss of motor function, etc. The symptom can also be the activity of Cls protein in a cell, tissue, or fluid of an individual. The symptom can also be the extent of complement activation in a cell, tissue, or fluid of an individual.

In some embodiments, administering an anti-C1s antibody of the present disclosure to an individual modulates complement activation in a cell, tissue, or fluid of an individual. In some embodiments, administration of an anti-C1s antibody of the present disclosure to an individual inhibits complement activation in a cell, tissue, or fluid of an individual. For example, in some embodiments, an anti-C1s antibody of the present disclosure, when administered in one or more doses as monotherapy or in combination therapy to an individual having a complement-mediated disease or disorder, inhibits complement activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to complement activation in the individual before treatment with the anti-C1s antibody.

In some embodiments, an anti-C1s antibody of the present disclosure reduces C3 deposition onto red blood cells; for example, in some embodiments, an anti-C1s antibody of the present disclosure reduces deposition of C3b, iC3b, etc., onto RBCs. In some embodiments, an anti-C1s antibody of the present disclosure inhibits complement-mediated red blood cell lysis.

In some embodiments, an anti-C1s antibody of the present disclosure reduces C3 deposition onto platelets; for example, in some embodiments, an anti-C1s antibody of the present disclosure reduces deposition of C3b, iC3b, etc., onto platelets.

In some embodiments, administering an anti-C1s antibody of the present disclosure results in an outcome selected from the group consisting of: (a) a reduction in complement activation; (b) an improvement in cognitive function; (c) a reduction in neuron loss; (d) a reduction in phospho-Tau levels in neurons; (e) a reduction in glial cell activation; (f) a reduction in lymphocyte infiltration; (g) a reduction in macrophage infiltration; (h) a reduction in antibody deposition, (i) a reduction in glial cell loss; (j) a reduction in oligodendrocyte loss; (k) a reduction in dendritic cell infiltration; (1) a reduction in neutrophil infiltration; (m) a reduction in red blood cell lysis; (n) a reduction in red blood cell phagocytosis; (o) a reduction in platelet phagocytosis; (p) a reduction in platelet lysis; (q) an improvement in transplant graft survival; (r) a reduction in macrophage mediated phagocytosis; (s) an improvement in vision; (t) an improvement in motor control; (u) an improvement in thrombus formation; (v) an improvement in clotting; (w) an improvement in kidney function; (x) a reduction in antibody mediated complement activation; (y) a reduction in autoantibody mediated complement activation; (z) an improvement in anemia; (aa) reduction of demyelination; (ab) reduction of eosinophilia; (ac) a reduction of C3 deposition on red blood cells (e.g., a reduction of deposition of C3b, iC3b, etc., onto RBCs); and (ad) a reduction in C3 deposition on platelets (e.g., a reduction of deposition of C3b, iC3b, etc., onto platelets); and (ae) a reduction of anaphylatoxin toxin production; (af) a reduction in autoantibody mediated blister formation; (ag) a reduction in autoantibody induced pruritis; (ah) a reduction in autoantibody induced erythematosus; (ai) a reduction in autoantibody mediated skin erosion; (aj) a reduction in red blood cell destruction due to transfusion reactions; (ak) a reduction in red blood cell lysis due to alloantibodies; (al) a reduction in hemolysis due to transfusion reactions; (am) a reduction in allo-antibody mediated platelet lysis; (an) a reduction in platelet lysis due to transfusion reactions; (ao) a reduction in mast cell activation; (ap) a reduction in mast cell histamine release; (aq) a reduction in vascular permeability; (ar) a reduction in edema; (as) a reduction in complement deposition on transplant graft endothelium;
(at) a reduction of anaphylatoxin generation in transplant graft endothelium; (au) a reduction in the separation of the dermal-epidermal junction; (av) a reduction in the generation of anaphylatoxins in the dermal-epidermal junction; (aw) a reduction in alloantibody mediated complement activation in transplant graft endothelium; (ax) a reduction in antibody mediated loss of the neuromuscular junction; (ay) a reduction in complement activation at the neuromuscular junction; (az) a reduction in anaphylatoxin generation at the neuromuscular junction; (ba) a reduction in complement deposition at the neuromuscular junction; (bb) a reduction in paralysis; (be) a reduction in numbness; (bd) increased bladder control; (be) increased bowel control; (bf) a reduction in mortality associated with autoantibodies; and (bg) a reduction in morbidity associated with autoantibodies.

In some embodiments, an anti-C1s antibody of the present disclosure, when administered in one or more doses as monotherapy or in combination therapy to an individual having a complement-mediated disease or disorder, is effect to achieve a reduction of at least about 10%, at least about 15%, at least about 20%, at least about 25%, at least about 30%, at least about 40%, at least about 50%, at least about 60%, at least about 70%, at least about 80%, at least about 90%, or more than 90%, of one or more of the following outcomes: (a) complement activation; (b) decline in cognitive function; (c) neuron loss; (d) phospho-Tau levels in neurons; (e) glial cell activation; (f) lymphocyte infiltration; (g) macrophage infiltration; (h) antibody deposition, (i) glial cell loss; (j) oligodendrocyte loss; (k) dendritic cell infiltration; (1) neutrophil infiltration; (m) red blood cell lysis; (n) red blood cell phagocytosis; (o) platelet phagocytosis; (p) platelet lysis; (q) transplant graft rejection; (r) macrophage mediated phagocytosis; (s) vision loss; (t) antibody mediated complement activation; (u) autoantibody mediated complement activation; (v) demyelination; (w) eosinophilia; compared to the level or degree of the outcome in the individual before treatment with the anti-C1s antibody.

In some embodiments, an anti-C1s antibody of the present disclosure, when administered in one or more doses as monotherapy or in combination therapy to an individual having a complement-mediated disease or disorder, is effect to achieve an improvement of at least about 10%, at least about 15%, at least about 20%, at least about 25%, at least about 30%, at least about 40%, at least about 50%, at least about 60%, at least about 70%, at least about 80%, at least about 90%, or more than 90%, of one or more of the following outcomes: a) cognitive function; b) transplant graft survival; c) vision; d) motor control; e) thrombus formation; f) clotting; g) kidney function; and h) hematocrit (red blood cell count), compared to the level or degree of the outcome in the individual before treatment with the anti-C1s antibody.

In some embodiments, administering an anti-C1s antibody of the present disclosure to an individual reduces complement activation in the individual. For example, in some embodiments, an anti-C1s antibody of the present disclosure, when administered in one or more doses as monotherapy or in combination therapy to an individual having a complement-mediated disease or disorder, reduces complement activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to complement activation in the individual before treatment with the anti-C1s antibody.

In some embodiments, administering an anti-C1s antibody of the present disclosure improves cognitive function in the individual. For example, in some embodiments, an antiC1s antibody of the present disclosure, when administered in one or more doses as monotherapy or in combination therapy to an individual having a complement-mediated disease or disorder, improves cognitive function in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the cognitive function in the individual before treatment with the anti-C1s antibody.

In some embodiments, administering an anti-C1s antibody of the present disclosure reduces the rate of decline in cognitive function in the individual. For example, in some embodiments, an anti-C1s antibody of the present disclosure, when administered in one or more doses as monotherapy or in combination therapy to an individual having a complement-mediated disease or disorder, reduces the rate of decline of cognitive function in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the rate of decline in cognitive function in the individual before treatment with the anti-C1s antibody.

In some embodiments, administering an anti-C1s antibody of the present disclosure to an individual reduces neuron loss in the individual. For example, in some embodiments, an anti-C1s antibody of the present disclosure, when administered in one or more doses as monotherapy or in combination therapy to an individual having a complement-mediated disease or disorder, reduces neuron loss in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to neuron loss in the individual before treatment with the anti-C1s antibody.

In some embodiments, administering an anti-C1s antibody of the present disclosure to an individual reduces phospho-Tau levels in the individual. For example, in some embodiments, an anti-C1s antibody of the present disclosure, when administered in one or more doses as monotherapy or in combination therapy to an individual having a complement-mediated disease or disorder, reduces phospho-Tau in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the phospho-Tau level in the individual before treatment with the anti-C1s antibody.

In some embodiments, administering an anti-C1s antibody of the present disclosure to an individual reduces glial cell activation in the individual. For example, in some embodiments, an anti-C1s antibody of the present disclosure, when administered in one or more doses as monotherapy or in combination therapy to an individual having a complement-mediated disease or disorder, reduces glial activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to glial cell activation in the individual before treatment with the anti-C1s antibody. In some embodiments, the glial cells are astrocytes or microglia.

In some embodiments, administering an anti-C1s antibody of the present disclosure to an individual reduces lymphocyte infiltration in the individual. For example, in some embodiments, an anti-C1s antibody of the present disclosure, when administered in one or more doses as monotherapy or in combination therapy to an individual having a complement-mediated disease or disorder, reduces lymphocyte infiltration in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to lymphocyte infiltration in the individual before treatment with the anti-C1s antibody.

In some embodiments, administering an anti-C1s antibody of the present disclosure to an individual reduces macrophage infiltration in the individual. For example, in some embodiments, an anti-C1s antibody of the present disclosure, when administered in one or more doses as monotherapy or in combination therapy to an individual having a complement-mediated disease or disorder, reduces macrophage infiltration in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to macrophage infiltration in the individual before treatment with the anti-C1s antibody.

In some embodiments, administering an anti-C1s antibody of the present disclosure to an individual reduces antibody deposition in the individual. For example, in some embodiments, an anti-C1s antibody of the present disclosure, when administered in one or more doses as monotherapy or in combination therapy to an individual having a complement-mediated disease or disorder, reduces antibody deposition in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to antibody deposition in the individual before treatment with the anti-C1s antibody.

In some embodiments, administering an anti-C1s antibody of the present disclosure to an individual reduces anaphylatoxin (e.g., C3a, C4a, C5a) production in an individual. For example, in some embodiments, an anti-C1s antibody of the present disclosure, when administered in one or more doses as monotherapy or in combination therapy to an individual having a complement-mediated disease or disorder, reduces anaphylatoxin production in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the level of anaphylatoxin production in the individual before treatment with the anti-C1s antibody.

In some embodiments, the present disclosure provides use of an anti-C1s antibody of the present disclosure or a pharmaceutical composition comprising an anti-C1s antibody of the present disclosure and a pharmaceutically acceptable excipient to treat an individual having a complement-mediated disease or disorder. In some embodiments, the present disclosure provides use of an anti-C1s antibody of the present disclosure to treat an individual having a complement-mediated disease or disorder. In some embodiments, the present disclosure provides use of a pharmaceutical composition comprising an anti-C1s antibody of the present disclosure and a pharmaceutically acceptable excipient to treat an individual having a complement-mediated disease or disorder.

In some embodiments, the present disclosure provides use of an anti-C1s antibody of the present disclosure in the manufacture of a medicament for the treatment of an individual having a complement-mediated disease or disorder.

In some embodiments, the present disclosure provides use of an anti-C1s antibody of the present disclosure or a pharmaceutical composition comprising an anti-C1s antibody of the present disclosure and a pharmaceutically acceptable excipient to inhibit complement activation. In some embodiments, the present disclosure provides use of an anti-C1s antibody of the present disclosure or a pharmaceutical composition comprising an anti-C1s antibody of the present disclosure and a pharmaceutically acceptable excipient to inhibit complement activation in an individual having a complement-mediated disease or disorder. In some embodiments, the present disclosure provides use of an anti-C1s antibody of the present disclosure to inhibit complement activation in an individual having a complement-mediated disease or disorder. In some embodiments, the present disclosure provides use of a pharmaceutical composition comprising an anti-C1s antibody of the present disclosure and a pharmaceutically acceptable excipient to inhibit complement activation in an individual having a complement-mediated disease or disorder.

In some embodiments, the present disclosure provides use of an anti-C1s antibody of the present disclosure in the manufacture of a medicament for modulating complement activation. In some embodiments, the medicament inhibits complement activation. In some embodiments, the medicament inhibits complement activation in an individual having a complement-mediated disease or disorder.

In some embodiments, the present disclosure provides an anti-C1s antibody of the present disclosure or a pharmaceutical composition comprising an anti-C1s antibody of the present disclosure and a pharmaceutically acceptable excipient for use in medical therapy. In some embodiments, the present disclosure provides an anti-C1s antibody of the present disclosure for use in medical therapy. In some embodiments, the present disclosure provides a pharmaceutical composition comprising an anti-C1s antibody of the present disclosure and a pharmaceutically acceptable excipient for use in medical therapy.

In some embodiments, the present disclosure provides an anti-C1s antibody of the present disclosure or a pharmaceutical composition comprising an anti-C1s antibody of the present disclosure and a pharmaceutically acceptable excipient for treating an individual having a complement-mediated disease or disorder. In some embodiments, the present disclosure provides an anti-C1s antibody of the present disclosure for treating an individual having a complement-mediated disease or disorder. In some embodiments, the present disclosure provides a pharmaceutical composition comprising an anti-C1s antibody of the present disclosure and a pharmaceutically acceptable excipient for treating an individual having a complement-mediated disease or disorder.

In some embodiments, the present disclosure provides an anti-C1s antibody of the present disclosure or a pharmaceutical composition comprising an anti-C1s antibody of the present disclosure and a pharmaceutically acceptable excipient for modulating complement activation. In some embodiments, the present disclosure provides an anti-C1s antibody of the present disclosure for modulating complement activation. In some embodiments, the present disclosure provides a pharmaceutical composition comprising an anti-C1s antibody of the present disclosure and a pharmaceutically acceptable excipient for modulating complement activation. In some embodiments, the anti-C1s antibody inhibits complement activation.

In a further aspect, the invention provides the use of an anti-C1s antibody in the manufacture or preparation of a medicament. In one embodiment, the medicament is for treatment of a complement-mediated disease or disorder. In a further embodiment, the medicament is for use in a method of treating a complement-mediated disease or disorder comprising administering to an individual having a complement-mediated disease or disorder an effective amount of the medicament. In one such embodiment, the method further comprises administering to the individual an effective amount of at least one additional therapeutic agent, e.g., as described below. In a further embodiment, the medicament is for use in enhancing the clearance of (or removing) C1s from plasma. In a further embodiment, the medicament is for use in enhancing the clearance of (or removing) C1r2s2 from plasma. In a further embodiment, the medicament is for use in enhancing the clearance of (or removing) C1r2s2 from plasma not but C1q from plasma. In a further embodiment, the medicament is for use in inhibiting a component of the classical complement pathway; in some cases, the classical complement pathway component is Cls.

In a further embodiment, the medicament is for use in a method of treating in an individual having a complement-mediated disease or disorder comprising administering to the individual an amount effective of the medicament. An "individual" according to any of the above embodiments may be a human.

In a further aspect, the invention provides a method for treating a complement-mediated disease or disorder. In one embodiment, the method comprises administering to an individual having such a complement-mediated disease or disorder an effective amount of an anti-C1s antibody. In one such embodiment, the method further comprises administering to the individual an effective amount of at least one additional therapeutic agent, as described below. An "individual" according to any of the above embodiments may be a human.

In a further aspect, the invention provides a method for enhancing the clearance of (or removing) C1s from plasma in an individual. In a further aspect, the invention provides a method for enhancing the clearance of (or removing) C1r2s2 from plasma in an individual. In a further aspect, the invention provides a method for enhancing the clearance of (or removing) C1r2s2from plasma not but C1q from plasma in an individual In some cases, the invention provides a method for inhibiting a component of the classical complement pathway in an individual; in some cases, the classical complement pathway component is Cls. In one embodiment, an "individual" is a human.

In a further aspect, the invention provides pharmaceutical formulations comprising any of the anti-C1s antibodies provided herein, e.g., for use in any of the above therapeutic methods. In one embodiment, a pharmaceutical formulation comprises any of the anti-C1s antibodies provided herein and a pharmaceutically acceptable carrier. In another embodiment, a pharmaceutical formulation comprises any of the anti-C1s antibodies provided herein and at least one additional therapeutic agent, e.g., as described below.

Antibodies of the invention can be used either alone or in combination with other agents in a therapy. For instance, an antibody of the invention may be co-administered with at least one additional therapeutic agent.

Such combination therapies noted above encompass combined administration (where two or more therapeutic agents are included in the same or separate formulations), and separate administration, in which case, administration of the antibody of the invention can occur prior to, simultaneously with, and/or following, administration of the additional therapeutic agent or agents. In one embodiment, administration of the anti-C1s antibody and administration of an additional therapeutic agent occur within about one month, or within about one, two or three weeks, or within about one, two, three, four, five, or six days, of each other. Antibodies of the invention can also be used in combination with radiation therapy.

An antibody of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 micro g/kg to 15 mg/kg (e.g. 0.1mg/kg-10mg/kg) of antibody can be an initial candidate dosage for administration to the patient, whether, for example, by one or more separate administrations, or by continuous infusion. One typical daily dosage might range from about 1 micro g/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). An initial higher loading dose, followed by one or more lower doses may be administered. However, other dosage regimens may be useful. The progress of this therapy is easily monitored by conventional techniques and assays.

It is understood that any of the above formulations or therapeutic methods may be carried out using an immunoconjugate of the invention in place of or in addition to an antiC1s antibody.

### VIII. Articles of Manufacture

In another aspect of the invention, an article of manufacture containing materials useful for the treatment, prevention and/or diagnosis of the disorders described above is provided. The article of manufacture comprises a container and a label on or a package insert associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active ingredient in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to an anti-C1s antibody.

### [Examples]

The following are examples of methods and compositions of the invention. It is understood that various other embodiments may be practiced, given the general description provided above.

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

### [Example 1]

### Preparation of recombinant C1r2s2

### 1.1. Expression and purification of human C1r2s2 tetramer (hC1r2s2)

The sequences used for expression and purification are human C1s (NCBI Reference Sequence: NP_958850.1) (SEQ ID NO: 1) and human C1r (NCBI Reference Sequence: NP_001724.3). The human C1r sequence includes mutations R463Q and S654A (SEQ ID NO: 2). For the expression of recombinant human C1r2s2 tetramers, human C1s and human C1r were transiently co-expressed using the HEK293 (Expi293 (registered trademark)) cell line (Thermo Fisher, Carlsbad, CA, USA) or the FreeStyle (registered trademark) 293-F cell line (Thermo Fisher, Carlsbad, CA, USA). Culture supernatant expressing recombinant human C1r2s2 was diluted 3-fold with MilliQ (registered trademark) water, CaCl₂ (Wako) was added thereto to make a final concentration of 2 mM, the pH was adjusted with 1N NaOH to pH8, this was applied to Q Sepharose HP anion exchange chromatography column (GE healthcare) equilibrated with 50 mM Tris-HCl, 2 mM CaCl₂, pH8.0, and eluted with NaCl gradient. Eluted fractions containing recombinant human C1r2s2 tetramers were collected, concentrated, and subsequently subjected to a Superdex (registered trademark) 200 gel filtration column (GE healthcare) equilibrated with 1x TBS (Wako), 2 mM CaCl₂ buffer. Fractions containing recombinant human C1r2s2 tetramers were then pooled, concentrated if needed, and stored at -80°C.

### 1.2. Expression and purification of cynomolgus C1r2s2 tetramer (cyC1r2s2)

The sequences used for expression and purification are cynomolgus C1s (SEQ ID NO: 3) and cynomolgus C1r. The cynomolgus C1r sequence includes mutations R463Q and S654A (SEQ ID NO: 4). For the expression of recombinant cynomolgus C1r2s2 tetramers, cynomolgus C1s and cynomolgus C1r were transiently co-expressed using the HEK293 (Expi293 (registered trademark)) cell line (Thermo Fisher, Carlsbad, CA, USA). Culture supernatant expressing recombinant cynomolgus C1r2s2 was diluted 3-fold with MilliQ (registered trademark) water, CaCl₂ (Wako) was added thereto to make a final concentration of 2 mM, the pH was adjusted with 1N NaOH to pH8, this was applied to Q Sepharose HP anion exchange chromatography column (GE healthcare) equilibrated with 50 mM Tris-HCl, 2 mM CaCl₂, pH8.0, and eluted with NaCl gradient. Eluted fractions containing recombinant human C1r2s2 tetramers were collected, concentrated, and subsequently subjected to a Superdex (registered trademark) 200 gel filtration column (GE healthcare) equilibrated with 1x TBS (Wako), 2 mM CaCl₂ buffer. Fractions containing recombinant cynomolgus C1r2s2 tetramers were then pooled and stored at -80°C.

### [Example 2]

### Preparation of recombinant Fc gamma receptors

### 2.1. Preparation of cynomolgus Fc gamma receptors (cyFcyRs)

The genes of the extracellular domain of cynomolgus Fc gamma receptor were constructed using methods known to those skilled in the art and cloning the cDNA of each Fc gamma receptor from cynomolgus monkeys. The amino acid sequences of the extracellular domain of Fc gamma receptors are shown in the Sequence Listing as follows: (cynomolgus Fc gamma receptor Ia [cyFcyRIa] is shown in SEQ ID NO: 5, cynomolgus Fc gamma receptor IIa1 [cyFcγRIIa1] is shown in SEQ ID NO: 6, cynomolgus Fc gamma receptor IIa2 [cyFcyRIIa2] is shown in SEQ ID NO: 7, cynomolgus Fc gamma receptor IIa3 [cyFcyRIIa3] is shown in SEQ ID NO: 8, cynomolgus Fc gamma receptor IIb [cyFcyRIIb] is shown in SEQ ID NO: 9, cynomolgus Fc gamma receptor IIIa (R) [cyFcγRIIIa(R)] is shown in SEQ ID NO: 10, cynomolgus Fc gamma receptor IIIa (S) [cyFcγRIIIa(S)] is shown in SEQ ID NO: 11). Next, the genetic sequence encoding His tag was attached to the 3' terminal of each gene. Each of the obtained genes was inserted into an expression vector designed for expression in mammalian cells by methods known to those skilled in the art. The expression vector was introduced into human embryonic kidney cell-derived FreeStyle293 cells (Invitrogen) to express target proteins. After culturing, the obtained culture supernatant was, in principle, filtrated and purified by the following 4 steps. As the first step, cation exchange chromatography using SP Sepharose FF was carried out. As the second step, affinity chromatography against His tag (HisTrap HP) was carried out. As the third step, gel filtration column chromatography (Superdex200) was carried out. As the fourth step, sterile filtration was carried out. Absorbance of the purified protein at 280 nm was measured using a spectrophotometer, and the concentration of the purified protein was determined using the absorption coefficient calculated by the PACE method (Protein Science 4:2411-2423 (1995)).

### 2.2. Preparation of human Fc gamma receptors (hFcγRs)

The genetic sequences of the extracellular domain of human Fc gamma receptors were obtained from human Fc gamma receptor Ia (NCBI Reference Sequence: NM_000566.3), human Fc gamma receptor IIa (NCBI Reference Sequence: NM_001136219.1), human Fc gamma receptor IIb (NCBI Reference Sequence: NM_004001.3), human Fc gamma receptor IIIa (NCBI Reference Sequence: NM_001127593.1), and human Fc gamma receptor IIIb (NCBI Reference Sequence: NM_000570.3). The polymorphic sites were designed with reference to the following documents (regarding human Fc gamma receptor IIa: Warmerdam, P. A. M. et al., 1990, J. Exp. Med. 172:19-25; regarding human Fc gamma receptor IIIa: Wu, J. et al., 1997, J. Clin. Invest. 100(5):1059-1070; regarding human Fc gamma receptor IIIb: Ory, P. A. et al., 1989, J. Clin. Invest. 84:1688-1691).

The amino acid sequences of the extracellular domain of Fc gamma receptors used for expression and purification are shown in the Sequence Listing as follows: (human Fc gamma receptor Ia [hFcyRIa] is shown in SEQ ID NO: 12, human Fc gamma receptor IIa_167R [hFcyRIIa_167R] is shown in SEQ ID NO: 13, human Fc gamma receptor IIa_167H [hFcγIIa_167H] is shown in SEQ ID NO: 14, human Fc gamma receptor IIb [hFcyRIIb] is shown in SEQ ID NO: 15, human Fc gamma receptor IIIa_176F [hFcγRIIIa_176F] is shown in SEQ ID NO: 16, human Fc gamma receptor IIIa_176V [hFcγRIIIa_176V] is shown in SEQ ID NO: 17, human Fc gamma receptor IIIb_NA1 [hFcγRIIIb_NA1] is shown in SEQ ID NO: 18, human Fc gamma receptor IIIb_NA2 [hFcγRIIIb_NA2] is shown in SEQ ID NO: 19). Next, His tag was attached to the C terminus and each of the obtained genes was inserted into an expression vector designed for expression in mammalian cells by methods known to those skilled in the art. The expression vector was introduced into human embryonic kidney cell-derived FreeStyle293 cells (Invitrogen) to express target proteins. After culturing, the obtained culture supernatant was, in principle, filtrated and purified by the following 4 steps, or by 3 steps excluding the initial anion exchange chromatography step. As the first step, anion exchange chromatography using Q Sepharose Fast Flow (GE Healthcare) was carried out. As the second step, affinity chromatography against His tag using HisTrap HP (GE Healthcare) was carried out. As the third step, gel filtration column chromatography using HiLoad 26/600 Superdex 200 pg (GE Healthcare) was carried out. As the fourth step, sterile filtration was carried out. Absorbance of the purified protein at 280 nm was measured using a spectrophotometer, and the concentration of the purified protein was determined using the absorption coefficient calculated by the PACE method (Protein Science 4:2411-2423 (1995)).

### [Example 3]

### Preparation of anti-C1s antibodies

### 3.1. Preparation of IPN009VH2VK3-SG1148

Polynucleotides of the heavy and light chain variable regions of an anti-C1s antibody, IPN009VH2 (SEQ ID NO: 20) and IPN009VK3 (SEQ ID NO: 21) (as described in WO2019/098212), were synthesized. These heavy and light chain variable regions were cloned into expression vectors containing the heavy chain constant region SG1148 (SEQ ID NO: 22) and the light chain constant region SK1 (SEQ ID NO: 23), respectively.

Anti-C1s antibody IPN009VH2VK3-SG1148 was expressed transiently using Expi293(registered trademark) F cells (Life technologies), according to the manufacturer's instructions. Recombinant antibodies were purified with protein A (GE Healthcare) and eluted in D-PBS, Tris Buffered Saline (TBS), or His buffer (20 mM Histidine, 150 mM NaCl, pH6.0). Size exclusion chromatography was further conducted to remove high molecular weight and/or low molecular weight components, if necessary

### 3.2. Creation of anti-C1s antibodies with optimized pH dependency, isoelectric point, and binding ability to Fc gamma receptors, and preparation of various anti-C1s antibodies

In order to create anti-C1s sweeping antibodies which enhance clearance of C1s from plasma and to achieve long term neutralization of C1s in blood, 3 antibodies were prepared in which the heavy-chain constant region SG1077R with enhanced binding to FcyR (cynomolgus surrogate Fc corresponding to TT91R for humans) has been linked to each of the 3 Fabs (COS0637pHv2, COS0637pHv3, and COS0637pHv8 (Japanese Patent Application No.: 2019-189148))(see Table 1 for the sequences). These antibodies were used to perform pharmacokinetics (PK) tests in monkeys; however, the PK profiles for all the antibodies were inferior than common therapeutic antibodies, and the complement activity could not be suppressed for a long period (Examples 4 and 10). COS0637pHv2-SG1077R with the most inferior PK, and COS0637pHv3-SG1077R and COS0637pHv8-SG1077R with slightly superior PK were compared. COS0637pHv3-SG1077R and COS0637pHv8-SG1077R had lower theoretical isoelectric points (pI) compared to COS0637pHv2-SG1077R (8.76 (COS0637pHv3-SG1077R), 8.76 (COS0637pHv8-SG1077R), and 9.27 (COS0637pHv2-SG1077R), respectively). Further, the binding ability of Fab was compared. Compared to COS0637pHv2, COS0637pHv3 and COS0637pHv8 had stronger pH dependency (Japanese Patent Application No.: 2019-189148: KD(pH5.8)/KD(pH7.4)=44(COS0637pHv2), 278(COS0637pHv3), 117(COS0637pHv8)). Thus, the inventors hypothesized that the PK of an anti-C1s antibody can be further improved by further enhancement of pH dependency and reduction of pI, and tests were carried out on this hypothesis. Further, in order to examine the influence on PK and FcyR binding, PK studies in monkeys were performed (Example 4) for COS0637pHv2-SG1077R and for COS0637pHv2-FcgSil (Tables 9 and 10), which was prepared by silencing all of the binding to cynomolgus FcyR in COS0637pHv2-SG1077R. As a result, it was revealed that the PK of anti-C1s antibody COS0637pHv2-SG1077R could be improved by silencing binding to FcyR. Based on the above results, improvement in pharmacokinetics was attempted by optimizing both the Fab and Fc. The list of antibodies and their sequence ID numbers used for the monkey PK studies are shown in Table 1. Further, sequences of the antibodies COS0637pHv8-TT91R and COS0637pHv3-TT91R used as controls in the binding tests are also shown in Table 1.

**(Table 1) Name of antibodies used in the monkey PK test and antibodies used as controls in the binding test, and corresponding SEQ ID NOs**

| Antibody Name | SEQ ID NO: | | | |
|---|---|---|---|---|
| | VH | VL | CH | CL |
| COS0637pHv2-SG1077R | 41 | 46 | 42 | 23 |
| COS0637pHv2-FcgSil | 41 | 46 | 43 | 23 |
| COS0637pHv3-SG1077R | 40 | 64 | 42 | 23 |
| COS0637pHv8-SG1077R | 24 | 63 | 42 | 23 |
| COS0637pHv8-TT91R | 24 | 63 | 44 | 23 |
| COS0637pHv3-TT91R | 40 | 64 | 44 | 23 |

In order for the optimization of antibodies aimed at improving pharmacokinetics and medicinal effect, COS0637pHv8-TT91RFab which was confirmed to have high pH dependency (Example 5) was used as a template to search for amino acid alterations on Fab that improves pH dependency, and to search for amino acid alterations on Fc that decreases pI and decreases binding to FcyR.

First, in order to improve pH dependency, amino acids of COS0637pHv8 were modified to produce the antibodies shown in Table 2 having improved pH dependency (improvement in pH dependency is shown in Example 5).

**(Table 2) Name of antibodies with improved pH dependency and corresponding SEQ ID NOs**

| Antibody Name | SEQ ID NO: | | | | | | | | | | | |
|---|---|---|---|---|---|---|---|---|---|---|---|---|
| | H chain | L chain | VH | VL | CH | CL | HVR -H1 | HVR -H2 | HVR -H3 | HVR -L 1 | HVR -L2 | HVR -L3 |
| COS0637pHvl5-TT91R | 78 | 79 | 24 | 59 | 44 | 23 | 25 | 26 | 27 | 60 | 61 | 62 |
| COS0637pHvl6-TT91R | 80 | 81 | 36 | 55 | 44 | 23 | 37 | 38 | 39 | 56 | 57 | 58 |
| COS0637pHvl7-TT91R | 82 | 83 | 24 | 55 | 44 | 23 | 25 | 26 | 27 | 56 | 57 | 58 |
| COS0637pHv21-TT91R | 84 | 85 | 24 | 47 | 44 | 23 | 25 | 26 | 27 | 48 | 49 | 50 |
| COS0637pHv23-TT91R | 86 | 87 | 28 | 51 | 44 | 23 | 29 | 30 | 31 | 52 | 53 | 54 |
| COS0637pHv25-TT91R | 88 | 89 | 32 | 55 | 44 | 23 | 33 | 34 | 35 | 56 | 57 | 58 |

Further, these antibodies were all shown as having "displacement function/activity" or "C1q displacement function/activity", which promotes release of C1q from the C1qrs complex by binding with the complex of C1q and C1r2s2 (C1qrs complex) (Example 6).

Further, a heavy chain constant region whose pI has been decreased and whose binding to FcyR has been suppressed (G1A3FcgSil+LowpI) was prepared (Examples 7 and 8). The alterations G137E, H268Q, K274Q, R355Q, and Q419E (all according to the EU numbering system) introduced to the native IgG1 sequence decreased pI. The alterations L235R and G236R introduced to the native IgG1 sequence suppressed binding to FcyR.

Molecules whose pH dependency has been improved, pI has been decreased, and binding ability to FcyR has been suppressed were produced by combining these Fab sequences and heavy chain constant region sequences (Table 3). Further, these anti-C1s Fab sequences with further improved pH dependency can be combined with, for example, Fcs for sweeping antibodies with enhanced FcyR binding such as TT91R and SG1077R, to exhibit higher sweeping ability and longer complement activity-neutralizing ability compared to antibodies with low pH dependency.

**(Table 3) Name of antibodies produced by combining Fab with improved pH dependency and Fc with decreased pI and suppressed FcyR-binding, and corresponding SEQ ID NOs**

| Antibody Name | SEQ ID NO: | | | | | |
|---|---|---|---|---|---|---|
| | H chain | L chain | VH | VL | CH | CL |
| COS0637pHv15-G1A3FcgSil+LowpI | 66 | 67 | 24 | 59 | 45 | 23 |
| COS0637pHv16-G1A3FcgSil+LowpI | 68 | 69 | 36 | 55 | 45 | 23 |
| COS0637pHv17-G1A3FcgSil+LowpI | 70 | 71 | 24 | 55 | 45 | 23 |
| COS0637pHv21-G1A3FcgSi1+LowpI | 72 | 73 | 24 | 47 | 45 | 23 |
| COS0637pHv23-GlA3FcgSil+LowpI | 74 | 75 | 28 | 51 | 45 | 23 |
| COS0637pHv25-G1A3FcgSil+LowpI | 76 | 77 | 32 | 55 | 45 | 23 |
| COS0637pHv8-G1A3FcgSil+LowpI | - | - | 24 | 63 | 45 | 23 |

For each antibody sequence, genes encoding the heavy chain variable region (VH) were synthesized and combined with modified human IgG1 heavy chain constant region (CH) (SG1, SEQ ID NO: 65), modified human IgG1 CH, e.g., SG1077R (SEQ ID NO: 42), FcgSil (SEQ ID NO: 43), TT91R (SEQ ID NO: 44), GIA3FcgSil+LowpI (SEQ ID NO: 45), and SG1148 (SEQ ID NO: 22). Genes encoding the light chain variable region (VL) were synthesized and combined with human light chain constant region (CL) (SK1, SEQ ID NO: 23). These combined sequences were cloned into expression vectors by methods known to those skilled in the art.

A mixture of expression vectors for the heavy chain and light chain was co-introduced into HEK293 cells for antibody expression, and each antibody was purified from the collected culture supernatant using Protein A or Protein G according to methods known to those skilled in the art. Gel filtration was further carried out as necessary.

### [Example 4]

### In vivo studies for anti-C1s antibodies

### In vivo studies using cynomolgus monkeys

The concentration of antibodies and endogenous C1s in plasma after administration of anti-C1s antibodies were evaluated by *in vivo* studies using 3-5 years old cynomolgus monkeys born in Cambodia (SNBL, Ltd.). Anti-C1s antibodies were administered at a dose of 10 mg/kg using a disposable syringe and indwelling needle to the forearm cephalic vein. Administration was carried out at a rate of 0.5 mL/kg/min or 2 mL/min. Regarding COS0637pHv2-SG1077R, blood was collected before administration, 5 minutes after administration, 2 hours after administration, 8 hours after administration, 1 day after administration, 2 days after administration, 4 days after administration, 7 days after administration, 14 days after administration, 21 days after administration, 28 days after administration, 42 days after administration, and 56 days after administration. For the other antibodies, blood was further collected 10 days after administration. Blood was collected from the femoral vein using heparin sodium prefilled syringe. Blood was immediately ice-cooled, and then centrifuged (4°C, 1700 xg, 5 minutes or 10 minutes) to collect plasma. Plasma was cryopreserved in a deep freezer (acceptable range: -70°C or less). The anti-C1s antibodies administered were the 4 antibodies: COS0637pHv2-SG1077R, COS0637pHv3-SG1077R, COS0637pHv8-SG1077R, and COS0637pHv2-FcgSil.

### Measurement of antibody concentration in plasma by electrochemiluminescence (ECL) method

Concentrations of COS0637pHv2-SG1077R and COS0637pHv2-FcgSil in the plasma of cynomolgus monkeys were measured by the ECL method. Anti-human IgG Fc mouse antibodies (SouthernBiotech, 9040-01) were dispensed into a MULTI-ARRAY 96-well plate (Meso Scale Discovery) and left to stand at room temperature for 1 hour for immobilization. For the standard curve and cynomolgus monkey plasma samples, dilution of 100-fold or more was carried out. The standard curve was prepared at 0.410, 1.02, 2.56, 6.40, 16.0, 40.0, and 100 µg/mL as the concentration in cynomolgus monkey plasma. After washing the plate onto which anti-human IgG Fc antibodies were immobilized, diluted samples were added to the plate, and this was stirred at room temperature for 1 hour. After washing the plate, biotinylated anti-human IgG antibodies (Bethyl Laboratories, A80-319A) were added and stirred at room temperature for 1 hour. After washing the plate, SULFO-TAG Streptavidin (Meso Scale Discovery) was added and stirred at room temperature for 1 hour. After washing the plate, Read Buffer T (x2) (Meso Scale Discovery) was immediately added to the plate and signals were detected using SECTOR Imager 2400 (Meso Scale Discovery). Concentration of each antibody was calculated using the analysis software SOFTmax PRO (Molecular Devices) based on the signals of the standard curve. Changes in antibody concentrations in plasma obtained by the present measurements are shown in Fig. 1-1.

### Measurement of antibody concentration in plasma by high performance liquid chromatography-electrospray ionization-tandem mass spectrometry (LC/ESI-MS/MS)

Concentrations of COS0637pHv3-SG1077R and COS0637pHv8-SG1077R in cynomolgus monkey plasma were measured by LC/ESI-MS/MS. The standard curve was prepared at 0.781, 1.56, 3.13, 6.25, 12.5, 25.0, and 50 µg/mL as the concentration in cynomolgus monkey plasma. Two microliter of standard curve and plasma samples were added to 50 µL of magnetic beads onto which antibodies that specifically bind to COS0637pHv3-SG1077R or COS0637pHv8-SG1077R were immobilized (Magnosphere MS300/ Low Carboxyl, JSR), and this was shaken at 25°C for 1.5 hours. Magnetic beads in the samples were washed 3 times with 0.2 mL of PBS containing 0.05% Tween 20, and then washed with 0.2 mL PBS. Magnetic beads were suspended in 24 µL of 50 mmol/L ammonium bicarbonate containing 7.5 mol/L urea, 8 mmol/L dithiothreitol and 0.1 µg/mL lysozyme (chicken egg white), and then this was shaken at 56°C for 45 minutes. Next, 2 µL of 500 mmol/L iodoacetamide was added, and this was shaken at 37°C for 30 minutes while blocking light. Next, 160 µL of 50 mmol/L ammonium bicarbonate containing 0.5 µg/mL sequencing grade modified trypsin (Promega) was added. Samples were shaken at 37°C for 16 hours, and then the reaction was stopped by adding 5 µL of 10% trifluoroacetic acid. Fifty microliter of enzyme digested samples were used for analysis with LC/ESI-MS/MS. Xevo TQ-S triple quadrupole instrument (Waters) connected to I-class UPLC (Waters) was used for LC/ESI-MS/MS analysis. Anti-C1s antibody-specific peptide NQVSLTC(Carbamidomethyl)LVK was detected by selected reaction monitoring (SRM). SRM transition was as follows: anti-C1s antibody parent ion [M+2H]²⁺ (m/z 581.3), daughter ion y⁷ ion (m/z 820.4). The internal calibration standard curve was prepared by 1/x² weighted linear regression using concentration and peak area. The concentration of antibodies in cynomolgus monkey plasma was calculated using the analysis software Masslynx Ver.4.1 (Waters). Changes in antibody concentrations in plasma obtained by the present measurements are shown in Fig. 1-1.

### Measurement of endogenous C1s concentration in plasma by high performance liquid chromatography-electrospray ionization-tandem mass spectrometry (LC/ESI-MS/MS)

Concentration of C1s in cynomolgus monkey plasma was measured by LC/ESI-MS/MS. The standard curve was prepared at 0.477, 0.954, 1.91, 3.82, 7.63, 15.3, and 30.5 µg/mL as the concentration of cynomolgus C1s in plasma, by diluting cynomolgus C1r2s2 with mouse plasma. Cynomolgus monkey plasma sample was diluted 5-fold using mouse plasma. Two microliter of standard curve and plasma samples were mixed with 26 µL of a mixed solution of 50 mmol/L ammonium bicarbonate (7.5 mol/L urea/ 100 mmol/L dithiothreitol/ 10 µg/mL lysozyme (chicken egg white)/ 100 µg/mL human C1s = 20/ 2/ 2/ 2), and then this was shaken at 56°C for 45 minutes. Human C1s was used as the internal standard. Next, 2 µL of 500 mmol/L iodoacetamide was added, and this was shaken at 37°C for 30 minutes while blocking light. Next, 160 µL of 50 mmol/L ammonium bicarbonate containing 0.5 µg/mL sequencing grade modified trypsin (Promega) was added. Samples were shaken at 37°C for 16 hours, and then the reaction was stopped by adding 5 µL of 10% trifluoroacetic acid. Fifty microliter of enzyme digested samples were used for analysis with LC/ESI-MS/MS. Xevo TQ-S triple quadrupole instrument (Waters) connected to I-class UPLC (Waters) was used for LC/ESI-MS/MS analysis. Cynomolgus C1s-specific peptide LLEVPEAR was detected by selected reaction monitoring (SRM). SRM transition was as follows: anti-C1s antibody parent ion [M+2H]²⁺ (m/z 463.8), daughter ion y⁶ ion (m/z 700.4). The internal calibration standard curve was prepared by 1/x² weighted linear regression using concentration and peak area. The concentration of C1s in cynomolgus monkey plasma was calculated using the analysis software Masslynx Ver.4.1 (Waters). Changes in C1s concentrations in plasma obtained by the present measurements are shown in Fig. 1-2.

### Effect of pH dependency and FcγR silent antibodies on concentration of anti-C1s antibodies and complement inhibiting activity in cynomolgus monkeys

The three anti-C1s antibodies (COS0637pHv2-, COS0637pHv3-, and COS0637pHv8-SG1077R) having Fes with alterations that enhance binding to monkey FcyRIIa and FcγRIIb reduced the concentration of C1s in plasma to 18.9% to 26.4% at maximum by 2 days after administration compared to that before their administration (Fig. 1-2). However, these antibodies disappeared faster compared to ordinary therapeutic IgG antibodies (Fig. 1-1), and along with the decrease in antibody concentration in plasma after 14 days post-administration, total C1s concentration was observed to increase again. However, in COS0637pHv3- and COS0637pHv8-SG1077R with improved pH dependency regarding antigen binding, improvement in antibody PK was observed compared to COS0637pHv2-SG1077R. This showed that the complement inhibiting activity achieved by COS0637pHv3- and COS0637pHv8-SG1077R administration lasts for 14 days and 21 days post-administration, although that achieved by COS0637pHv2-SG1077R administration lasts up to 7 days post-administration. And thus, long term antigen neutralization and complement inhibition by COS0637pHv3- and COS0637pHv8-SG1077R was demonstrated (Fig. 4).

Meanwhile, the anti-C1s antibody having Fes with alterations that reduce binding to monkey FcyRs, COS0637pHv2-FcgSil, showed good PK compared to antibodies with alterations to enhance binding to monkey FcyRIIa and FcyRIIb (Fig. 1-1). The complement inhibiting activity achieved by COS0637pHv2-FcgSil administration lasted up to 14 days post-administration (Fig. 4). Total C1s concentration achieved by COS0637pHv2-FcgSil administration decreased only to a maximum of 75.5% compared to that before administration (Fig. 1-2); however, due to improvement in PK, compared to COS0637pHv2- SG1077R, long term antigen neutralization and complement inhibiting activity were shown.

### [Example 5]

### Binding assays under different pH conditions using Biacore (registered trademark)

The binding property of each sample at pH7.4 and pH6.0 was determined at 37°C using BIACORE (registered trademark) T200 instrument (Cytiva). First, anti-human C1r2s2 antibody IPN009VH2Vk3-SG1148 (IPN009) was immobilized onto all of the flow cells of a CM5 sensor chip using Amine Coupling Kit, type2 (Cytiva). 20 mM ACES, 150 mM NaCl, 1.2 mM CaCl₂, 1 mg/mL BSA (not containing IgG), 1 mg/mL CMD, 0.05% Tween (registered trademark) 20, 0.005 w/v% NaN₃, pH7.4 or pH6.0 buffer was used as the running buffer. Next, recombinant human C1r2s2 tetramers (hC1r2s2) and recombinant cynomolgus C1r2s2 tetramers (cyC1r2s2), prepared to have a binding response of 100 Resonance unit (RU), were captured on the sensor surface by IPN009. The antibody solution was added thereto at 30 µL/min for 120 seconds, the running buffer was then added at 30 µL/min for 180 seconds, and the dissociation constant (K_{D}) of each antibody for hC1r2s2 and cyC1r2s2 was calculated. In order to calculate the K_{D} value at pH7.4, antibody solutions diluted with a running buffer of pH7.4 to make 0, 0.8, 1.6, 3.1, 6.3, 13 nM were injected, and in order to calculate the K_{D} value at pH6.0, antibody solutions prepared with a running buffer of pH6.0 to make 0, 6.3, 12.5, 25, 50, 100 nM were injected. The sensor surface was regenerated every cycle, using 3 M MgCl₂ (prepared in-house). The K_{D} value was calculated using BIACORE (registered trademark) T200 evaluation software version 2.0 (Cytiva). The binding strengths under each pH condition were compared by dividing the K_{D} value at pH6.0 with the K_{D} value at pH7.4. (Table 4)

**(Table 4) Binding activity to human C1r2s2 antibodies under acidic conditions and neutral conditions**

| hC1r2s2 | | | |
|---|---|---|---|
| Antibody Name | KD (mol/L) | | *K*_{D} (pH6.0) / *K*_{D} (pH7.4) |
| | pH6.0 | pH7.4 | |
| COS0637pHv8-TT91R | 1.25E-08 | 2.49E-10 | 50.2 |
| COS0637pHv3-TT91R | 1.03E-08 | 3.88E-10 | 26.5 |
| COS0637pHv15-G1A3FcgSil+LowpI | 3.18E-07 | 3.99E-10 | 797.0 |
| COS0637pHv16-G1A3FcgSil+LowpI | 2.92E-08 | 1.81E-10 | 161.3 |
| COS0637pHv17-G1A3FcgSil+LowpI | 3.29E-08 | 2.30E-10 | 143.0 |
| COS0637pHv21-G1A3FcgSil+LowpI | 3.13E-08 | 2.60E-10 | 120.4 |
| COS0637pHv23-G1A3FcgSil+LowpI | 2.74E-08 | 2.49E-10 | 110.0 |
| COS0637pHv25-G1A3FcgSil+LowpI | 2.13E-08 | 1.98E-10 | 107.6 |
| COS0637pHv8-G1A3FcgSil+LowpI | 1.93E-08 | 2.72E-10 | 71.0 |

The KD values to human C 1r2s2 at pH6.0 and pH7.4 calculated using Biacore, and the ratio of the KD value at pH 6.0 to the KD value at pH7.4 are shown.

**(Table 5) Binding activity to cynomolgus C1r2s2 antibodies under acidic conditions and neutral conditions**

| cyC1r2s2 | | | |
|---|---|---|---|
| Antibody Name | KD (mol/L) | | *K*_{D} (pH6.0) / *K*_{D} (pH7.4) |
| | pH6.0 | pH7.4 | |
| COS0637pHv8-TT91R | 2.33E-08 | 3.45E-10 | 67.5 |
| COS0637pHv3-TT91R | 2.20E-08 | 5.02E-10 | 43.8 |
| COS0637pHv15-G1A3FcgSil+LowpI | 1.11E-07 | 4.92E-10 | 225.6 |
| COS0637pHv16-G1A3FcgSil+LowpI | 9.12E-08 | 1.96E-10 | 465.3 |
| COS0637pHv17-G1A3FcgSil+LowpI | 1.06E-07 | 2.52E-10 | 420.6 |
| COS0637pHv21-G1A3FcgSil+LowpI | 6.29E-08 | 3.46E-10 | 181.8 |
| COS0637pHv23-G1A3FcgSil+LowpI | 4.63E-08 | 3.34E-10 | 138.6 |
| COS0637pHv25-G1A3FcgSil+LowpI | 6.71E-08 | 2.43E-10 | 276.1 |
| COS0637pHv8-G1A3FcgSil+LowpI | 3.03E-08 | 3.65E-10 | 83.0 |

The KD values to monkey C1r2s2 at pH6.0 and pH7.4 calculated using Biacore, and the ratio of the KD value at pH 6.0 to the KD value at pH7.4 are shown.

### [Example 6]

### Evaluation of anti-C1s antibodies for the C1q displacement function

The antibody's C1q displacement function was demonstrated at 37°C by the C1r2s2 capture method using BIACORE (registered trademark) T200 instrument (Cytiva). The antihuman C1r2s2 antibody IPN009VH2Vk3-SG1148 was immobilized onto a CM5 sensor chip using Amine Coupling Kit, type2 (Cytiva). The antibodies, recombinant human C1r2s2 tetramers (hC1r2s2), and native human C1q (CompTech, hC1q) were prepared with a pH7.4 running buffer (20 mM ACES, 150 mM NaCl, 1.2 mM CaCl₂, 1 mg/mL BSA (not containing IgG), 1 mg/mL CMD, 0.05% Tween (registered trademark) 20, 0.005 w/v% NaN₃, pH7.4). First, hC1r2s2 was captured on the sensor surface by IPN009VH2Vk3-SG1148. As the amount of capture, 200 resonance unit (RU) was aimed. Next, hC1q diluted with the running buffer to make 100 nM was injected, and immediately thereafter, the antibody solution diluted with the running buffer to 500 nM was injected at 10 µL/min for 1500 seconds. The sensor surface was regenerated every cycle, using 3 M MgCl₂ (prepared in-house). The results are shown in Figs. 2-1 to 2-14. The sensorgrams were obtained using BIACORE (registered trademark) T200 evaluation software, version 2.0 (Cytiva). The solid lines indicate the sensorgrams obtained when hC1q was injected to hC 1r2s2 and then buffer was injected (C1r2s2+C1q), and they reflect C1qrs complex formation on the sensor chip surface (herein below referred to as sensorgram 1). The dashed-dotted lines indicate the sensorgrams obtained when hC1q was injected to hC1r2s2 and then antibody was injected (C1r2s2+C1q+Ab), and they reflect the effect of the antibodies on C1qrs complexes on the sensor chip surface (herein below referred to as sensorgram 2). The broken lines indicate the sensorgrams obtained when hC1q was not injected to hC1r2s2 and only the antibodies were injected (C1r2s2+Ab), and they illustrate the binding of the antibodies to hC1r2s2 only on the sensor chip surface (herein below referred to as sensorgram 3). Figs. 2-1 to 2-14 show sensorgrams 1 to 3. For comparison of these sensorgrams, binding response of C1r2s2 was normalized as 100 RU.

Anti-C1s antibodies bind to C1qrs, and thus, from the time point after C1q addition, sensorgram response increases as the antibodies are added. Regarding C1s antibodies that do not have the C1q displacement function, from the time point after C1q addition, sensorgram response increases as the antibodies are added, and the response unit hardly changes at the time of stopping antibody addition (Citation: WO 2019198807, Fig. 2A, COS0583).

Regarding antibodies that have the C1q displacement function, from the time point after C1q addition, sensorgram response increases as antibodies are added; however, the response unit at the time of stopping antibody addition is lower than the response unit when only Buffer is added after C1q addition, or the degree of decrease of the response unit at the time of stopping antibody addition to the time immediately after start of antibody addition is greater than the degree of decrease when only buffer is added.

In view of sensorgram 3 in Figs. 2-1 to 2-14, each Ab was thought to stably bind C1r2s2 in the absence of C1q. In sensorgram 1, C1q stably bound with C1r2s2 in the absence of Ab. Moreover, in most antibodies, the response unit at the time of stopping antibody addition in sensorgram 2 was lower than that in sensorgram 1, and in some antibodies, the degree of decrease in the response unit at the time of stopping addition compared to immediately after antibody addition in sensorgram 2 was greater than the degree of decrease in sensorgram 1. The above results indicate that all of the modified antibodies could dissociate C1q from C1qrs complex.

### [Example 7]

### Evaluation of anti-C1s antibodies for isoelectric point (pI)

### 4.1. Measurement of anti-C1s antibody isoelectric point (pI) using capillary isoelectric focusing (clEF)

Maurice (Protein simple) was used with a capillary cartridge to perform clEF. The anolyte and catholyte used were 0.08 M phosphate containing 0.1 w/v% methylcellulose (MC) and 0.1 M sodium hydroxide containing 0.1 w/v% MC, respectively. All of the analyzed samples contained a working 0.2 mg/mL antibody, 0.35 w/v% MC, 6.0 mM IDA (iminodiacetic acid), 10 mM arginine, 0.5 w/v% pI marker 5.85 and 0.5 w/v% pI marker 9.99, and 2 vol% pharmalyte 8-10.5 and 2 vol% pharmalyte 5-8. All samples were vortexed and centrifuged briefly prior to loading into the autosampler compartment. Samples were focused at conditions of 1.5 kV for 1 minute followed by 3.0 kV for 7 minutes each. The autosampler compartment was kept at 10°C. Measurements were repeated 2 times for each sample, and the pI value was obtained by calculating the average of n = 2 measurements. The calculated pI values are shown in Table 6.

**(Table 6) Measured pI of each anti-C1s antibody having Fes with reduced pI and suppressed binding to FcyRs**

| Antibody Name | pI (Average of n2 measurements) |
|---|---|
| COS0637pHv8-TT91R | 9.0 |
| COS0637pHv8-G1A3FcgSil+LowpI | 7.7 |
| COS0637pHv15-G1A3FcgSil+LowpI | 7.5 |
| COS0637pHv16-G1A3FcgSil+LowpI | 7.8 |
| COS0637pHv17-G1A3FcgSil+LowpI | 7.8 |
| COS0637pHv21-G1A3FcgSil+LowpI | 7.6 |
| COS0637pHv23-G1A3FcgSil+LowpI | 7.6 |
| COS0637pHv25-G1A3FcgSil+LowpI | 7.8 |

The pI of each antibody measured using Maurice (Protein simple) is shown. The pI value is the average of the values obtained with two measurements.

### [Example 8]

### Confirmation of binding to FcγR

The binding property of each sample to FcγRs at pH7.4 was determined at 25°C using BIACORE (registered trademark) T200 instrument (Cytiva). First, protein L (BioVision) was immobilized onto all of the flow cells of a CM4 sensor chip using Amine Coupling Kit, type2 (Cytiva). 50 mM phosphate buffer (pH7.4) containing 150 mM NaCl, 0.05% Tween (registered trademark) 20 was used as the running buffer, and antibodies with the binding response of which were prepared to 500 RU or 2000 RU were captured on the sensor surface. Human and monkey FcyRs diluted with the running buffer were injected thereto and the amount bound with the antibodies was measured. hFcγRIa and cyFcyRIa were diluted to 8 nM, and the rest were diluted to 1000 nM. The sensor surface was regenerated every cycle, using 10 mM glycine hydrochloride solution, pH1.5 (prepared in-house). From the obtained measurement results, FcyR binding amount divided by the binding amount of each captured antibody (Binding/capture) was calculated using BIACORE (registered trademark) T200 evaluation software, version 2.0 (Cytiva) (Table 7 and Table 9). Further, from these results, the Binding/capture values for other specimens are shown in the tables (Table 8 and Table 10), in which the Binding/capture value obtained with Herceptin (Chugai Pharmaceutical Co., Ltd.) (human IgG1/kappa) is set as 1.

**(Table 7) Response value per 1 RU of captured antibody to human FcyRs**

| | | hFcγR Ia | hFcγR IIa_167 H | hFcγR IIa_167 R | hFcγR IIb | hFcγR IIIa_17 6F | hFcγR IIIa_17 6V | hFcγR IIIb_N A1 | hFcγR IIIb_N A2 |
|---|---|---|---|---|---|---|---|---|---|
| hFcγRs (binding/ capture) | Herceptin | 0.20 | 0.06 | 0.05 | 0.01 | 0.04 | 0.10 | 0.01 | 0.02 |
| | COS0637pHv8-G1A3FcgSil+LowpI | 0.00 | 0.00 | 0.00 | 0.00 | 0.00 | 0.00 | 0.00 | 0.00 |

Binding/capture values for human FcyRs calculated using Biacore are shown.

**(Table 8) Relative binding to human FcyRs**

| | | hFcγR Ia | hFcγR IIa_16 7H | hFcγR IIa_16 7R | hFcγR IIb | hFcγR IIIa_17 6F | hFcγR IIIa_17 6V | hFcγR IIIb_N A1 | hFcγR IIIb_N A2 |
|---|---|---|---|---|---|---|---|---|---|
| hFcγRs (Relative value of binding/ capture) | Herceptin | 1.00 | 1.00 | 1.00 | 1.00 | 1.00 | 1.00 | 1.00 | 1.00 |
| | COS0637pHv8-G1A3FcgSil+LowpI | 0.00 | -0.01 | -0.01 | -0.08 | -0.05 | -0.02 | -0.12 | -0.07 |

From the Binding/capture values for human FcyRs calculated using Biacore, the relative binding of the antibodies under development to Herceptin was calculated.

**(Table 9) Response value per 1 RU of captured antibody to cynomolgus FcyRs**

| | | cyFcγR Ia | cyFcγR IIa1 | cyFcγR IIa2 | cyFcγR IIa3 | cyFcγR IIb | cyFcγR IIIa(R) | cyFcγR IIIa(S) |
|---|---|---|---|---|---|---|---|---|
| cyFcγRs (binding/ capture) | Herceptin | 0.22 | 0.02 | 0.02 | 0.01 | 0.03 | 0.12 | 0.13 |
| | COS0637pHv8-G1A3FcgSil+LowpI | 0.00 | 0.00 | 0.00 | 0.00 | 0.00 | 0.00 | 0.00 |
| | COS0637pHv2-SG1077R | 0.02 | 0.05 | 0.05 | 0.03 | 0.08 | 0.01 | 0.01 |
| | COS0637pHv2-FcgSil | 0.00 | 0.00 | 0.00 | 0.00 | 0.00 | 0.00 | 0.00 |

Binding/capture values for monkey FcyRs calculated using Biacore are shown.

**(Table 10) Relative binding to cynomolgus FcyRs**

| | | cyFcγR Ia | cyFcγR IIa1 | cyFcγR IIa2 | cyFcγR IIa3 | cyFcγR IIb | cyFcγR Illa(R) | cyFcγR IIIa(S) |
|---|---|---|---|---|---|---|---|---|
| cyFcγRs (Relative value of binding/ capture) | Herceptin | 1.00 | 1.00 | 1.00 | 1.00 | 1.00 | 1.00 | 1.00 |
| | COS0637pHv8-G1A3FcgSil+LowpI | 0.00 | -0.01 | -0.04 | -0.09 | -0.01 | -0.01 | -0.01 |
| | COS0637pHv2-SG1077R | 0.07 | 2.16 | 2.55 | 4.47 | 2.74 | 0.07 | 0.08 |
| | COS0637pHv2-FcgSil | 0.00 | 0.00 | -0.02 | -0.06 | 0.00 | -0.01 | -0.01 |

From the Binding/capture values for monkey FcyRs calculated using Biacore, the relative binding of the antibodies under development to Herceptin was calculated.

### [Example 9]

### In vivo studies using cynomolgus monkeys

Studies were carried out by methods similar to those of Example 4. The administered anti-C1s antibodies were the 2 antibodies, COS0637pHv16-G1A3FcgSil+LowpI and COS0637pHv21-G1A3FcgSil+LowpI.

### Measurement of antibody concentration in plasma by high performance liquid chromatography-electrospray ionization-tandem mass spectrometry (LC/ESI-MS/MS)

Concentrations of COS0637pHv16-G1A3FcgSil+LowpI and COS0637pHv21-G1A3FcgSil+LowpI in cynomolgus monkey plasma were measured by LC/ESI-MS/MS. The standard curve was prepared at 0.781, 1.56, 3.13, 6.25, 12.5, 25.0, and 50 µg/mL as the concentration in cynomolgus monkey plasma. Two microliter of standard curve and plasma samples were added to 50 µL of magnetic beads onto which antibodies that specifically bind to COS0637pHvl6-G1A3FcgSil+LowpI or COS0637pHv21-G1A3FcgSil+LowpI were immobilized (Magnosphere MS300/ Low Carboxyl, JSR), and after centrifugation this was shaken at 25°C for 1.5 hours. Magnetic beads in the samples were washed 3 times with 0.2 mL of PBS containing 0.05% Tween 20, and then washed with 0.2 mL PBS. Magnetic beads were suspended in 24 µL of 50 mmol/L ammonium bicarbonate containing 7.5 mol/L urea, 8.3 mmol/L dithiothreitol and 0.083 µg/mL lysozyme (chicken egg white), and this was shaken at 56°C for 45 minutes. Next, 2 µL of 500 mmol/L iodoacetamide was added, and this was shaken at 37°C for 30 minutes while blocking light. Next, 160 µL of 50 mmol/L ammonium bicarbonate containing 0.5 µg/mL sequencing grade modified trypsin (Promega) was added. Samples were shaken at 37°C over night, and then the reaction was stopped by adding 5 µL of 10% trifluoroacetic acid. Fifty microliter of enzyme digested samples were used for analysis with LC/ESI-MS/MS. Xevo TQ-S triple quadrupole instrument (Waters) connected to I-class UPLC (Waters) was used for LC/ESI-MS/MS analysis. Anti-C1s antibody-specific peptide GPSVFPLAPSSR was detected by selected reaction monitoring (SRM). SRM transition was as follows: anti-C1s antibody parent ion [M+2H]²⁺ (m/z 607.8), daughter ion y⁷ ion (m/z 727.4). The internal calibration standard curve was prepared by 1/x² weighted linear regression using concentration and peak area. The concentration of antibodies in cynomolgus monkey plasma was calculated using the analysis software Masslynx Ver.4.2 (Waters). Changes in antibody concentrations in plasma obtained by the present measurements are shown in Fig. 3-1.

### Measurement of endogenous C1s concentration in plasma by high performance liquid chromatography-electrospray ionization-tandem mass spectrometry (LC/ESI-MS/MS)

Concentration of C1s in cynomolgus monkey plasma was measured by LC/ESI-MS/MS. The standard curve was prepared at 0.477, 0.954, 1.91, 3.82, 7.63, 15.3, and 30.5 µg/mL as the concentration of C1s in cynomolgus monkey plasma, by diluting cynomolgus C1r2s2 with mouse plasma. Cynomolgus monkey plasma samples were diluted 5-fold using mouse plasma. Two microliter of standard curve and plasma samples were mixed with 26 µL of a mixed solution of 50 mmol/L ammonium bicarbonate (7.5 mol/L urea/ 100 mmol/L dithiothreitol/ 10 µg/mL lysozyme (chicken egg white)/ 300 µg/mL human C1s2r2 = 20/ 2/ 2/ 2), and then this was shaken at 56°C for 45 minutes. Human C1s was used as the internal standard. Next, 2 µL of 500 mmol/L iodoacetamide was added, and this was shaken at 37°C for 30 minutes while blocking light. Next, 160 µL of 50 mmol/L ammonium bicarbonate containing 0.5 µg/mL sequencing grade modified trypsin (Promega) was added. Samples were shaken at 37°C for 16 hours, and then the reaction was stopped by adding 5 µL of 10% trifluoroacetic acid. Fifty microliter of enzyme digested samples were used for analysis with LC/ESI-MS/MS. Xevo TQ-S triple quadrupole instrument (Waters) connected to I-class UPLC (Waters) was used for LC/ESI-MS/MS analysis. Cynomolgus C1s-specific peptide LLEVPEAR was detected by selected reaction monitoring (SRM). SRM transition was as follows: anti-C1s antibody parent ion [M+2H]²⁺ (m/z 463.8), daughter ion y⁶ ion (m/z 700.4). The internal calibration standard curve was prepared by 1/x² weighted linear regression using concentration and peak area. The concentration of C1s in cynomolgus monkey plasma was calculated using the analysis software Masslynx Ver.4.2 (Waters). Changes in C1s concentrations in plasma obtained by the present measurements are shown in Fig. 3-2.

### Effect of pH dependency and pI on concentration of anti-C1s antibodies and complement inhibiting activity in cynomolgus monkeys

Regarding anti-C1s antibodies having Fes with alterations that decrease binding to monkey FcyRs, further alterations were made for improving pH dependency in antigen binding and decreasing antibody pI to produce COS0637pHv16-G1A3FcgSil+LowpI and COS0637pHv21-G1A3FcgSil+LowpI, and the changes in antibody and antigen concentrations were determined. Regarding the changes in C1s concentration after antibody administration, no great difference was observed as compared to the case where COS0637pHv2-FcgSil was administered. Meanwhile, the antibody concentration at 56 days after administration was 20 times or higher than in the COS0637pHv2-FcgSil case, and great improvement in PK was observed. The complement inhibiting activity achieved by administration of each antibody lasted at least up to 56 days after administration (Fig. 4). COS0637pHv16-G1A3FcgSil+LowpI and COS0637pHv21-G1A3FcgSil+LowpI showed great improvement in PK and resulted in long term antigen neutralization and complement inhibiting activity.

### [Example 10]

### Evaluation of complement neutralizing function in monkeys (RBC lysis inhibiting effect)

The antibody's complement inhibiting activity was evaluated as follows using sensitized chicken red blood cells. Plasma collected from a monkey was diluted to 10% with the assay buffer (HBSS Ca²⁺ Mg²⁺ containing 0.05% BSA). Chicken red blood cells (Japan Bio Serum) were sensitized using anti-chicken red blood cell antibodies (Rockland), counted after rinsing, and prepared at 1×10⁸ cells/mL in the assay buffer. Next, monkey-derived plasma was added to an equal amount of sensitized chicken red blood cells, and this was incubated at 37°C for 7 minutes to lyse the red blood cells. The final concentration of monkey plasma in this reaction mixture was 5%. The reaction was stopped by cool assay buffer containing EDTA. This mixture was centrifuged to form pellets of unlysed cells, the supernatant was collected, and release of hemoglobin was analyzed using absorbance (OD) at 415 nm. In order to calculate the percentage of lysed red blood cells (%), lysis of red blood cells with plasma before antibody administration was set as 100%, percentage of lysis under conditions where plasma was not added was set as 0%, and the percentage of lysed red blood cells at each time point was calculated. Plasma from 3 monkeys was used, and 1 well each was evaluated per 1 time point. Data shown in Fig. 4 indicates the mean ± SD of data judged as being anti-antibody negative.

### [Industrial Applicability]

The antibodies of the present invention with improved PK show blood C1s neutralizing activity and complement inhibiting activity over a long term. Such antibodies of the present invention may be used for treating or preventing complement-mediated diseases or disorders.

## Claims

1. An isolated antibody that comprises an antigen-binding region and an antibody constant region,
wherein the antibody promotes dissociation of C1q from C1qrs complex and/or inhibits binding of C1q to C1r2s2, and
wherein the antigen-binding region comprises a combination of HVR-H1, HVR-H2, HVR-H3, HVR-L1, HVR-L2, and HVR-L3 selected from the group consisting of 1) to 6) below:
1) HVR-H1, HVR-H2, HVR-H3, HVR-L1, HVR-L2, and HVR-L3 that comprise the amino acid sequences consisting of SEQ ID NOs: 25, 26, 27, 60, 61, and 62, respectively;
2) HVR-H1, HVR-H2, HVR-H3, HVR-L1, HVR-L2, and HVR-L3 that comprise the amino acid sequences consisting of SEQ ID NOs: 37, 38, 39, 56, 57, and 58, respectively;
3) HVR-H1, HVR-H2, HVR-H3, HVR-L1, HVR-L2, and HVR-L3 that comprise the amino acid sequences consisting of SEQ ID NOs: 25, 26, 27, 56, 57, and 58, respectively;
4) HVR-H1, HVR-H2, HVR-H3, HVR-L1, HVR-L2, and HVR-L3 that comprise the amino acid sequences consisting of SEQ ID NOs: 25, 26, 27, 48, 49, and 50, respectively;
5) HVR-H1, HVR-H2, HVR-H3, HVR-L1, HVR-L2, and HVR-L3 that comprise the amino acid sequences consisting of SEQ ID NOs: 29, 30, 31, 52, 53, and 54, respectively; and
6) HVR-H1, HVR-H2, HVR-H3, HVR-L1, HVR-L2, and HVR-L3 that comprise the amino acid sequences consisting of SEQ ID NOs: 33, 34, 35, 56, 57, and 58, respectively.

2. The antibody of claim 1, wherein the antibody comprises a heavy chain variable region (VH) and a light chain variable region (VL) selected from the group consisting of 1) to 6) below:
1) a VH and VL that comprise the amino acid sequences consisting of SEQ ID NOs: 24 and 59, respectively;
2) a VH and VL that comprise the amino acid sequences consisting of SEQ ID NOs: 36 and 55, respectively;
3) a VH and VL that comprise the amino acid sequences consisting of SEQ ID NOs: 24 and 55, respectively;
4) a VH and VL that comprise the amino acid sequences consisting of SEQ ID NOs: 24 and 47, respectively;
5) a VH and VL that comprise the amino acid sequences consisting of SEQ ID NOs: 28 and 51, respectively; and
6) a VH and VL that comprise the amino acid sequences consisting of SEQ ID NOs: 32 and 55, respectively.

3. The antibody of claim 1 or 2, wherein a ratio of a KD value in acidic pH range to a KD value in neutral pH range, an acidic KD/ neutral KD ratio, is 107 or more.

4. The antibody of any one of claims 1 to 3, wherein the antigen-binding region can specifically bind to a CUB1-EGF-CUB2 domain of human C1s.

5. The antibody of any one of claims 1 to 4, wherein the antibody has a mutant constant region comprising at least one amino acid alteration that decreases Fcy receptor-binding activity.

6. The antibody of claim 5, wherein the mutant constant region comprises an amino acid alteration at at least one of positions 235 and 236 according to EU numbering.

7. The antibody of any one of claims 1 to 6, wherein the antibody has a mutant constant region comprising at least one amino acid alteration, and wherein the amino acid alteration decreases isoelectric point (pI) of the mutant constant region as compared to that of a parent constant region.

8. The antibody of claim 7, wherein the mutant constant region comprises an amino acid alteration at at least one of positions 137, 268, 274, 355, and 419 according to EU numbering.

9. The antibody of any one of claims 1 to 8, wherein pI is 7.8 or less.

10. The antibody of any one of claims 1 to 8, wherein the constant region comprises a heavy chain constant region comprising the amino acid sequence consisting of SEQ ID NO: 45 and a light chain constant region comprising the amino acid sequence consisting of SEQ ID NO: 23.

11. An antibody having binding activity to C1s, wherein the antibody comprises a combination of HVR-H1, HVR-H2, HVR-H3, HVR-L1, HVR-L2, and HVR-L3 selected from the group consisting of 1) to 6) below:
1) HVR-H1, HVR-H2, HVR-H3, HVR-L1, HVR-L2, and HVR-L3 that comprise the amino acid sequences consisting of SEQ ID NOs: 25, 26, 27, 60, 61, and 62, respectively;
2) HVR-H1, HVR-H2, HVR-H3, HVR-L1, HVR-L2, and HVR-L3 that comprise the amino acid sequences consisting of SEQ ID NOs: 37, 38, 39, 56, 57, and 58, respectively;
3) HVR-H1, HVR-H2, HVR-H3, HVR-L1, HVR-L2, and HVR-L3 that comprise the amino acid sequences consisting of SEQ ID NOs: 25, 26, 27, 56, 57, and 58, respectively;
4) HVR-H1, HVR-H2, HVR-H3, HVR-L1, HVR-L2, and HVR-L3 that comprise the amino acid sequences consisting of SEQ ID NOs: 25, 26, 27, 48, 49, and 50, respectively;
5) HVR-H1, HVR-H2, HVR-H3, HVR-L1, HVR-L2, and HVR-L3 that comprise the amino acid sequences consisting of SEQ ID NOs: 29, 30, 31, 52, 53, and 54, respectively; and
6) HVR-H1, HVR-H2, HVR-H3, HVR-L1, HVR-L2, and HVR-L3 that comprise the amino acid sequences consisting of SEQ ID NOs: 33, 34, 35, 56, 57, and 58, respectively.

12. The antibody of claim 11, wherein the antibody comprises a heavy chain variable region (VH) and a light chain variable region (VL) selected from the group consisting of 1) to 6) below:
1) a VH and VL that comprise the amino acid sequences consisting of SEQ ID NOs: 24 and 59, respectively;
2) a VH and VL that comprise the amino acid sequences consisting of SEQ ID NOs: 36 and 55, respectively;
3) a VH and VL that comprise the amino acid sequences consisting of SEQ ID NOs: 24 and 55, respectively;
4) a VH and VL that comprise the amino acid sequences consisting of SEQ ID NOs: 24 and 47, respectively;
5) a VH and VL that comprise the amino acid sequences consisting of SEQ ID NOs: 28 and 51, respectively; and
6) a VH and VL that comprise the amino acid sequences consisting of SEQ ID NOs: 32 and 55, respectively.

13. The antibody of claim 11 or 12, wherein the antibody constant region comprises an H chain constant region comprising the amino acid sequence consisting of SEQ ID NO: 45 and an L chain constant region comprising the amino acid sequence consisting of SEQ ID NO: 23.

14. An antibody comprising a heavy chain (H chain) and a light chain (L chain) selected from the group consisting of 1) to 6) below:
1) an H chain and L chain that comprise the amino acid sequences consisting of SEQ ID NOs: 66 and 67, respectively;
2) an H chain and L chain that comprise the amino acid sequences consisting of SEQ ID NOs: 68 and 69, respectively;
3) an H chain and L chain that comprise the amino acid sequences consisting of SEQ ID NOs: 70 and 71, respectively;
4) an H chain and L chain that comprise the amino acid sequences consisting of SEQ ID NOs: 72 and 73, respectively;
5) an H chain and L chain that comprise the amino acid sequences consisting of SEQ ID NOs: 74 and 75, respectively; and
6) an H chain and L chain that comprise the amino acid sequences consisting of SEQ ID NOs: 76 and 77, respectively.
